# FASCICULE DE BREVET EUROPEEN

(11) **EP 1 385 963 B1**
(45) Date de publication et mention de la délivrance du brevet: **11.06.2008**
(21) Numéro de dépôt: 02720067.4
(22) Date de dépôt: 19.03.2002
(51) Int. Cl.: C12N 15/54, C12N 15/10, C12N 9/12, C12P 21/00

(54) **POLYPEPTIDES DERIVES DES ARN POLYMERASES, ET LEURS UTILISATIONS**
VON RNA POLYMERASEN ABSTAMMENDE POLYPEPTIDE UND IHRE VERWENDUNGEN
POLYPEPTIDES DERIVED FROM RNA POLYMERASES AND USE THEREOF

(30) Priorité: 19.03.2001 FR 0103677
(43) Date de publication de la demande: 04.02.2004
(73) Titulaire: CENTRE NATIONAL DE LA RECHERCHE SCIENTIFIQUE, 75794 Paris Cedex 16 (FR)
(72) Inventeur: GUILLEREZ, Jean, F-91190 Gif sur Yvette (FR); LOPEZ, Pascal, F-75010 Paris (FR); DREYFUS, Marc, F-75013 Paris (FR)
(74) Mandataire: Grosset-Fournier, Chantal Catherine
(86) Numéro de dépôt international: PCT/FR2002/000961
(87) Numéro de publication internationale: WO 2002/074964

(56) Documents cités:
- US-A- 5 385 834
- US-A- 6 107 037
- MAKAROVA OLGA V ET AL: "Transcribing of Escherichia coli genes with mutant T7 RNA polymerases: Stability of lacZ mRNA inversely correlates with polymerase speed." PROCEEDINGS OF THE NATIONAL ACADEMY OF SCIENCES OF THE UNITED STATES, vol. 92, no. 26, 1995, pages 12250-12254, XP002189160 1995 ISSN: 0027-8424 cité dans la demande
- BONNER GARY ET AL: "Characterization of a set of T7 RNA polymerase active site mutants." JOURNAL OF BIOLOGICAL CHEMISTRY, vol. 269, no. 40, 1994, pages 25120-25128, XP002189161 ISSN: 0021-9258 cité dans la demande
- FRUGIER M ET AL: "SYNTHETIC POLYAMINES STIMULATE IN VITRO TRANSCRIPTION BY T7 RNA POLYMERASE" NUCLEIC ACIDS RESEARCH, OXFORD UNIVERSITY PRESS, SURREY, GB, vol. 22, no. 14, 1994, pages 2784-2790, XP002926452 ISSN: 0305-1048 cité dans la demande
- JIN DING JUN: "A mutant RNA polymerase reveals a kinetic mechanism for the switch between nonproductive stuttering synthesis and productive initiation during promoter clearance." JOURNAL OF BIOLOGICAL CHEMISTRY, vol. 271, no. 20, 1996, pages 11659-11667, XP002189159 ISSN: 0021-9258
- LOPEZ PASCAL J ET AL: "The low processitivity of T7 RNA polymerase over the initially transcribed sequence can limit productive initiation in vivo." JOURNAL OF MOLECULAR BIOLOGY, vol. 269, no. 1, 1997, pages 41-51, XP002189162 ISSN: 0022-2836 cité dans la demande

## Description

La présente invention a pour objet des polypeptides dérivés des ARN polymérases, encore désignés ARN polymérases mutées, ainsi que leurs utilisations, notamment dans le domaine de la préparation *in vivo* ou *in vitro* d'ARN d'intérêt.

Lorsqu'il s'agit d'obtenir des quantités importantes de molécules d'ADN de séquence parfaitement définie, de haute pureté et de taille modeste (10 à 100 nt) la synthèse chimique est à ce jour la méthode la plus économique. Il existe maintenant sur le marché de très nombreuses entreprises qui réalisent ces synthèses à la demande. Ces mêmes sociétés proposent aussi la synthèse chimique d'ARN mais la technologie est telle que le coût, au moins dix fois plus élevé que pour l'ADN, est actuellement prohibitif.

On privilégie alors la synthèse enzymatique en utilisant une ARN polymérase couplée à une matrice ADN, synthétisée chimiquement ou d'origine plasmidique, qui comporte la séquence à transcrire en aval de la séquence promoteur de la polymérase utilisée. Les éléments nécessaires (polymérases, rNTP, vecteurs de clonage) sont disponibles sur le marché sous forme de kits optimisés.

L'ARN polymérase du bactériophage T7 est très largement utilisée pour effectuer des réactions de transcription *in vitro* dans le but de synthétiser des quantités importantes (jusqu'à un milligramme) d'ARN à partir de matrices d'ADN recombinant. Ces ARN synthétisés in vitro sont nécessaires pour de nombreuses applications de la Biologie, des Biotechnologies et de la Pharmacie; on peut citer entre autres:
- la traduction *in vitro.*
- l'étude de la maturation des ARN.
- l'effet des ARN anti-sens sur l'expression des gènes.
- les interactions ARN-protéines.
- la synthèse d'homologues de petits ARN cellulaires (ARNt, ARNr).
- la production de ribozymes.

Plus généralement ces ARN sont aussi utilisés comme sondes moléculaires et comme substrats d'études structurales.

Quelques mutants de la polymérase T7 ont déjà été décrits. Il s'agit des ARN polymérases mutées dérivées de l'ARN polymérase sauvage T7, et comprenant l'une des mutations suivantes :
remplacement de l'acide glutamique en position 222 par une lysine (K); cette ARN polymérase ainsi mutée a la propriété de reconnaître un promoteur altéré par mutation, et est décrite dans le brevet US 5 385 834,
remplacement de la tyrosine (Y) en position 639 par une phénylalanine (F); cette ARN polymérase ainsi mutée a la propriété d'incorporer des désoxyribonucléotides au lieu de ribonucléotides, permettant ainsi la synthèse d'ADN au lieu d'ARN, et est décrite dans le brevet US 6 107 037,
remplacement de l'isoleucine (I) en position 810 par une sérine (S) ; cette ARN polymérase ainsi mutée a la propriété d'être plus lente que l'ARN polymérase sauvage, et est décrite dans Bonner et al., The Journal of Biochemical Chemistry, 269, pp. 25120-25128 (1994).

Le problème que se propose de résoudre la présente invention, est lié au fait que l'activité sur une matrice d'ADN donnée de l'ARN polymérase de T7 (et de toutes les autres ARN polymérases connues d'ailleurs) est fortement dépendante de la nature des 6-12 premiers nucléotides transcrits (Séquence Initialement Transcrite, ou ITS) de la séquence nucléotidique codant pour un ARN donné (Milligan et al., 1987). Si l'ITS diffère trop de la séquence consensus 5'GGGAGA... alors la polymérase avorte fréquemment et une quantité importante des ribonucléosides triphosphates est consommée pour synthétiser de petits ARN abortifs au détriment des grands ARN souhaités.

De ce fait, l'expérimentateur est généralement contraint, pour obtenir une transcription efficace, de modifier la séquence qu'il souhaite transcrire pour lui ajouter une ITS favorable (encore désignée ITS consensus ou consensuelle). Dans de nombreux cas, cette contrainte est très gênante, voire inacceptable.

Une méthode potentielle pour obtenir un ARN d'extrémité 5' quelconque non consensuelle utilise un post traitement d'un ARN comportant la séquence désirée en aval d'une ITS consensus; ce dernier ARN (cible) peut donc être produit en abondance. Il faut de plus synthétiser chimiquement un oligonucléotide chimérique ADN-ARN dont la séquence ADN est complémentaire de l'extrémité 3' de l'ARN à éliminer puis digérer par la RNase H l'hybride que peut former l'oligo avec le transcrit cible. Cette méthode complexe et onéreuse se justifie si l'ARN souhaité est de très grande taille et si son extrémité 5' doit être rigoureusement définie.(Li et al. 1999, Lapham et al. 1997).

L'addition, dans le milieu réactionnel, de polyamines de synthèses peut accroître la production d'ARN en diminuant semble-t-il le nombre de cycles abortifs; cependant la détermination des conditions expérimentales optimales (choix de la polyamine, concentration d'utilisation) nécessite une recherche au cas par cas. Notons de plus que l'effet de ces polyamines est faible voire nul lorsque la matrice est d'origine plasmidique (double brin) (Frugier et al. 1994).

Ces deux méthodes utilisent des réactifs qui ne sont pas d'usage courant (polyamines ou oligonucléotides spécifiques) et sont peu adaptées à une utilisation de routine. Il est évident que la possibilité de disposer au départ d'une polymérase qui par nature avorte moins serait une manière beaucoup plus simple de résoudre la difficulté.

L'invention découle de la mise en évidence par les Inventeurs du fait que la modification de la séquence peptidique des ARN polymérases était susceptible de diminuer la sensibilité des ARN polymérases ainsi modifiées à la nature de l'ITS des séquences nucléotidiques à transcrire.

L'invention a pour but de fournir des polypeptides dérivés des ARN polymérases sauvages habituellement utilisées dans le cadre de la production d'ARN d'intérêt, qui soient nettement moins sensibles à la nature de l'ITS non consensuelle que lesdites ARN polymérases sauvages.

L'invention a également pour but de fournir de nouveaux procédés de production d'ARN d'intérêt avec des rendements supérieurs aux procédés utilisant des ARN polymérases sauvages lorsque ces dernières sont sensibles à l'ITS de la séquence nucléotidique codant pour ledit ARN d'intérêt.

L'invention a pour objet l'utilisation d'ARN polymérases mutées moins sensibles que les ARN polymérases sauvages à la Séquence Initialement Transcrite ou ITS contenue dans la séquence d'ADN codant pour un ARN d'intérêt, lesdites ARN polymérases mutées étant choisies parmi :
* celles dérivées de l'ARN polymérase sauvage T7, et comprenant au moins l'une des mutations suivantes :
   - remplacement de l'isoleucine (I) en position 117 par une valine (V),
   - remplacement de l'isoleucine (I) en position 119 par une valine (V),
   - remplacement de la valine (V) en position 134 par une alanine (A),
   - remplacement de l'acide aspartique (D) en position 147 par l'asparagine (N),
   - remplacement de l'histidine (H) en position 230 par une arginine (R),
   - remplacement de la proline (P) en position 266 par une leucine (L),
   - remplacement de l'arginine (R) en position 291 par une cystéine (C),
* celles dérivées de l'ARN polymérase sauvage T3, et comprenant au moins l'une des mutations suivantes :
   - remplacement de l'acide aspartique (D) en position 148 par l'asparagine (N),
   - remplacement de la proline (P) en position 267 par une leucine (L),
   - remplacement de l'arginine (R) en position 292 par une cystéine (C),
* celles dérivées de l'ARN polymérase sauvage K11, et comprenant au moins l'une des mutations suivantes :
   - remplacement de l'acide aspartique (D) en position 167 par l'asparagine (N),
   - remplacement de la proline (P) en position 289 par une leucine (L),
   - remplacement de l'arginine (R) en position 314 par une cystéine (C),
* celles dérivées de l'ARN polymérase sauvage SP6, et comprenant au moins l'une des mutations suivantes :
   - remplacement de l'acide aspartique (D) en position 117 par l'asparagine (N),
   - remplacement de la proline (P) en position 239 par une leucine (L),
   pour la mise en oeuvre d'un procédé de production dudit ARN d'intérêt, ou de protéines codées par cet ARN d'intérêt, à partir de séquences nucléotidiques déterminées comprenant une séquence d'ADN codant pour ledit ARN d'intérêt et dont la transcription est placée sous le contrôle d'un promoteur reconnu par les ARN polymérases sauvages et les ARN polymérases mutées susmentionnées, ledit procédé ayant un rendement de production dudit ARN supérieur au rendement obtenu dans le cas de l'utilisation des ARN polymérases sauvages en présence de la même ITS non consensuelle que celle contenue dans la séquence d'ADN codant pour ledit ARN d'intérêt.

L'activité desdites ARN polymérases ainsi mutées dans le cadre de la transcription de la séquence d'ADN codant pour ledit ARN d'intérêt est nettement moins affectée par la nature des nucléotides constituant l'ITS non consensuelle de cette séquence d'ADN, que ne l'est celle des ARN polymérases sauvages dont elles dérivent, ce qui permet à ces ARN polymérases mutées d'être jusqu'à environ 40 fois plus actives que les polymérases sauvages, et donc au procédé susmentionné de la présente invention d'être caractérisé par un rendement de production dudit ARN supérieur au rendement obtenu dans le cas de l'utilisation des ARN polymérases sauvages en présence de cette même ITS non consensuelle.

Selon un autre aspect particulièrement avantageux de l'invention, les ARN polymérases mutées de l'invention permettent d'obtenir des ARN d'intérêt avec un rendement quasiment identique quelque soit l'ITS présente dans la séquence codant pour ledit ARN d'intérêt.

Avantageusement, l'utilisation des ARN polymérases mutées susmentionnées, permet la mise en oeuvre d'un procédé de production d'ARN d'intérêt dont le rendement est jusqu'à environ dix fois supérieur au rendement obtenu dans le cas de l'utilisation des ARN polymérases sauvages en présence d'une ITS non consensuelle.

Les ARN d'intérêt susceptibles d'être produits en plus grande quantité dans le cadre de la mise en oeuvre d'un procédé selon l'invention utilisant les ARN polymérases mutées susmentionnées, sont des ARN naturels ou chimériques, lesdits ARN comprenant le cas échéant un ou plusieurs nucléoside monophosphate non canonique (à savoir par exemple un désoxy-ribose à la place d'un ribose, ledit sucre pouvant lui-même éventuellement porter un analogue d'une des bases nucléiques naturelles si cet analogue est reconnu comme tel par la polymérase). Dans ce dernier cas, la présente invention a également pour objet l'application du procédé susmentionné utilisant lesdites ARN polymérases susmentionnées, à la mise en oeuvre d'une méthode de détermination de la séquence d'une molécule d'acide nucléique.

L'invention a plus particulièrement pour objet l'utilisation susmentionnée des ARN polymérases mutées suivantes :
- l'ARN polymérase T7 mutée représentée par SEQ ID N0 : 2, comprenant une leucine en position 266 en substitution de la proline,
- l'ARN polymérase T7 mutée représentée par SEQ ID N0 : 4, comprenant une valine en position 117 en substitution de l'isoleucine, et une alanine en position 134 en substitution de la valine,
- l'ARN polymérase T7 mutée représentée par SEQ ID N0 : 6, comprenant une valine en position 119 en substitution de l'isoleucine, et une asparagine en position 147 en substitution de l'acide aspartique,
- l'ARN polymérase T7 mutée représentée par SEQ ID N0 : 8, comprenant une arginine en position 230 en substitution de l'histidine, et une cystéine en position 291 en substitution de l'arginine,
- l'ARN polymérase T7 mutée représentée par SEQ ID N0: 10, comprenant une leucine en position 266 en substitution de la proline, et une phénylalanine en position 639 en substitution de la tyrosine,
- l'ARN polymérase T7 mutée représentée par SEQ ID N0: 12, comprenant une asparagine en position 810 en substitution de l'isoleucine,
- l'ARN polymérase T7 mutée représentée par SEQ ID N0 : 14, comprenant une leucine en position 266 en substitution de la proline, et une asparagine en position 810 en substitution de l'isoleucine,
- l'ARN polymérase T7 mutée représentée par SEQ ID N0 : 16, comprenant une valine en position 119 en substitution de l'isoleucine, une asparagine en position 147 en substitution de l'acide aspartique, et une asparagine en position 810 en substitution de l'isoleucine.

L'invention a également pour objet l'utilisation décrite ci-dessus d'ARN polymérases mutées susmentionnées comprenant en plus de la (ou des) modification(s) d'acide(s) aminé(s) définies ci-dessus, la modification, par substitution, ou délétion, ou addition, d'au moins un autre acide aminé impliqué dans un autre mécanisme que celui de la sensibilité à l'ITS dans le cadre de la transcription de séquences d'ADN, tel qu'une modification d'un acide aminé permettant à l'ARN polymérase ainsi modifiée d'incorporer des nucléosides triphosphates non canoniques (par exemple des dNTP) dans une chaîne d'ARN, et donc permettant de synthétiser de l'ADN, et/ou une modification d'un acide aminé permettant d'obtenir une ARN polymérase plus lente, et donc, en association avec une mutation de l'invention, permettant d'améliore les rendements de synthèse de protéines d'intérêt.

A ce titre l'invention a plus particulièrement pour objet l'utilisation susmentionnée d'ARN polymérases mutées telles que définies ci-dessus, comprenant en plus de la (ou des) mutation(s) définie(s) ci-dessus, une mutation correspondant à la substitution de la tyrosine en position 639 chez T7, ou en position analogue chez les autres polymérases définies ci-dessus, par une phénylalanine, et/ou une mutation correspondant à la substitution de l'isoleucine en position 810 chez T7, ou en position analogue chez les autres polymérases définies ci-dessus, par une sérine, ou par une asparagine.

A ce titre encore, l'invention concerne plus particulièrement l'utilisation de l'ARN polymérase T7 mutée représentée par SEQ ID N0 : 10 susmentionnée.

L'invention a également pour objet l'utilisation :
* les ARN polymérases mutées dérivées de l'ARN polymérase sauvage T7 comprenant au moins une des mutations susmentionnées en position 117, 119, 134, 147, 230, 266, ou 291, et éventuellement au moins une des mutations supplémentaires suivantes:
   - remplacement de la tyrosine (Y) en position 639 par une phénylalanine (F),
   - remplacement de l'isoleucine (I) en position 810 par une asparagine (N),
* les ARN polymérases mutées dérivées de l'ARN polymérase sauvage T3 comprenant au moins une des mutations susmentionnées en position 148, 267, ou 292, et éventuellement au moins une des mutations supplémentaires suivantes:
   - remplacement de la tyrosine (Y) en position 640 par une phénylalanine (F),
   - remplacement de l'isoleucine (I) en position 811 par une asparagine (N),
* les ARN polymérases mutées dérivées de l'ARN polymérase sauvage K11 comprenant au moins une des mutations susmentionnées en position 167, 289, ou 314, et éventuellement au moins une des mutations supplémentaires suivantes:
   - remplacement de la tyrosine (Y) en position 662 par une phénylalanine (F),
   - remplacement de l'isoleucine (I) en position 833 par une asparagine (N),
* les ARN polymérases mutées dérivées de l'ARN polymérase sauvage SP6 comprenant au moins une des mutations susmentionnées en position 117, ou 239, et éventuellement au moins une des mutations supplémentaires suivantes:
   - remplacement de la tyrosine (Y) en position 631 par une phénylalanine (F),
   - remplacement de l'isoleucine (I) en position 804 par une asparagine (N).

L'invention concerne également tout procédé de préparation d'ARN polymérases mutées d'origine phagique telles que définies ci-dessus, à savoir d'ARN polymérases dont l'activité de transcription d'une séquence nucléotidique déterminée comprenant une séquence d'ADN codant pour un ARN d'intérêt et dont la transcription est placée sous le contrôle d'un promoteur reconnu par les ARN polymérases mutées et par les ARN polymérases sauvages d'origine phagique dont elles sont issues, est supérieure à l'activité de transcription de cette séquence déterminée par les ARN polymérases sauvages, en présence de la même ITS non consensuelle que celle contenue dans la séquence d'ADN codant pour ledit ARN d'intérêt, lesdites ARN polymérases mutées étant jusqu'à environ 40 fois plus actives que lesdites ARN polymérases sauvages dans le cadre de la mise en oeuvre de procédés de production d'ARN d'intérêt à partir de ladite séquence nucléotidique déterminée, ledit procédé comprenant :
- une étape de modification de la chaîne peptidique des ARN polymérases sauvages d'origine phagique par substitution, ou délétion ou addition d'au moins un codon du gène codant pour lesdites ARN polymérases sauvages, et transformation de cellules appropriées, telle que *E.coli,* avec des vecteurs contenant le gène ainsi modifié, ainsi qu'une séquence nucléotidique codant pour un marqueur, tel qu'un marqueur de résistance à un antibiotique, ou un marqueur chromogène, codé par une séquence nucléotidique insérée en aval du promoteur reconnu par les ARN polymérases susmentionnées, ce promoteur et la séquence codant pour le marqueur étant séparés par une ITS,
- une étape de détection des cellules susmentionnées produisant des ARN polymérases mutées pour lesquelles le rendement de la production du marqueur particulier, est supérieur au rendement de la production de ce même marqueur obtenu par utilisation des ARN polymérases sauvages dans les mêmes conditions, et
- une étape de purification des ARN polymérases mutées susmentionnées à partir des cellules détectées à l'étape précédente.

L'invention a plus particulièrement pour objet l'utilisation susmentionnée, d'ARN polymérases mutées d'origine phagique telles qu'obtenues par mise en oeuvre du procédé de préparation défini ci-dessus.

L'invention concerne également les ARN polymérases mutées d'origine phagique telles qu'obtenues par mise en oeuvre d'un procédé de préparation défini ci-dessus.

L'invention concerne plus particulièrement les ARN polymérases mutées dérivées des ARN polymérases sauvages telles que T7, T3, K11 ou SP6, et choisies parmi les suivantes :
* celles dérivées de l'ARN polymérase sauvage T7, et comprenant au moins l'une des mutations suivantes :
   - remplacement de l'isoleucine (I) en position 117 par une valine (V),
   - remplacement de l'isoleucine (I) en position 119 par une valine (V),
   - remplacement de la valine (V) en position 134 par une alanine (A),
   - remplacement de l'acide aspartique (D) en position 147 par l'asparagine (N),
   - remplacement de l'histidine (H) en position 230 par une arginine (R),
   - remplacement de la proline (P) en position 266 par une leucine (L),
   - remplacement de l'arginine (R) en position 291 par une cystéine (C),
   lesdites ARN polymérases mutées comprenant éventuellement au moins une des mutations supplémentaires suivantes:
   - remplacement de la tyrosine (Y) en position 639 par une phénylalanine (F),
   - remplacement de l'isoleucine (I) en position 810 par une asparagine (N),
* celles dérivées de l'ARN polymérase sauvage T3, et comprenant au moins l'une des mutations suivantes :
   - remplacement de l'acide aspartique (D) en position 148 par l'asparagine (N),
   - remplacement de la proline (P) en position 267 par une leucine (L),
   - remplacement de l'arginine (R) en position 292 par une cystéine (C),
   lesdites ARN polymérases mutées comprenant éventuellement au moins une des mutations supplémentaires suivantes:
   - remplacement de la tyrosine (Y) en position 640 par une phénylalanine (F),
   - remplacement de l'isoleucine (I) en position 811 par une asparagine (N),
* celles dérivées de l'ARN polymérase sauvage K11, et comprenant au moins l'une des mutations suivantes :
   - remplacement de l'acide aspartique (D) en position 167 par l'asparagine (N),
   - remplacement de la proline (P) en position 289 par une leucine (L),
   - remplacement de l'arginine (R) en position 314 par une cystéine (C),
   lesdites ARN polymérases mutées comprenant éventuellement au moins une des mutations supplémentaires suivantes:
   - remplacement de la tyrosine (Y) en position 662 par une phénylalanine (F),
   - remplacement de l'isoleucine (I) en position 833 par une asparagine (N),
* celles dérivées de l'ARN polymérase sauvage SP6, et comprenant au moins l'une des mutations suivantes :
   - remplacement de l'acide aspartique (D) en position 117 par l'asparagine (N),
   - remplacement de la proline (P) en position 239 par une leucine (L),
   lesdites ARN polymérases mutées comprenant éventuellement au moins une des mutations supplémentaires suivantes:
   - remplacement de la tyrosine (Y) en position 631 par une phénylalanine (F),
   - remplacement de l'isoleucine (I) en position 804 par une asparagine (N).

L'invention a plus particulièrement pour objet les ARN polymérases mutées suivantes :
- l'ARN polymérase T7 mutée représentée par SEQ ID NO : 2, comprenant une leucine en position 266 en substitution de la proline,
- l'ARN polymérase T7 mutée représentée par SEQ ID NO : 4, comprenant une valine en position 117 en substitution de l'isoleucine, et une alanine en position 134 en substitution de la valine,
- l'ARN polymérase T7 mutée représentée par SEQ ID NO : 6, comprenant une valine en position 119 en substitution de l'isoleucine, et une asparagine en position 147 en substitution de l'acide aspartique,
- l'ARN polymérase T7 mutée représentée par SEQ ID NO : 8, comprenant une arginine en position 230 en substitution de l'histidine, et une cystéine en position 291 en substitution de l'arginine,
- l'ARN polymérase T7 mutée représentée par SEQ ID NO : 10, comprenant une leucine en position 266 en substitution de la proline, et une phénylalanine en position 639 en substitution de la tyrosine,
- l'ARN polymérase T7 mutée représentée par SEQ ID NO : 12, comprenant une asparagine en position 810 en substitution de l'isoleucine,
- l'ARN polymérase T7 mutée représentée par SEQ ID NO : 14, comprenant une leucine en position 266 en substitution de la proline, et une asparagine en position 810 en substitution de l'isoleucine,
- l'ARN polymérase T7 mutée représentée par SEQ ID NO : 16, comprenant une valine en position 119 en substitution de l'isoleucine, une asparagine en position 147 en substitution de l'acide aspartique, et une asparagine en position 810 en substitution de l'isoleucine.

L'invention concerne également les séquences nucléotidiques codant pour une ARN polymérase mutée d'origine phagique telle que définie ci-dessus.

A ce titre l'invention a plus particulièrement pour objet les séquences nucléotidiques SEQ ID NO : 1, 3, 5, 7, 9, 11, 13, 15, codant respectivement pour les protéines SEQ ID NO : 2, 4, 6, 8, 10, 12, 14, 16, susmentionnées, ou toute séquence nucléotidique dérivée des séquences nucléotidiques susmentionnées par dégénérescence du code génétique et conservant la propriété de coder pour les protéines susmentionnées.

L'invention a également pour objet tout vecteur, notamment tout plasmide, contenant une séquence nucléotidique telle que définie ci-dessus.

L'invention a également pour objet toute cellule transformée par un vecteur susmentionné, ladite cellule étant choisie notamment parmi celles des bactéries (*E. coli* par exemple), des levures, ou des eucaryotes supérieurs.

L'invention concerne également tout procédé de préparation d'ARN polymérases mutées d'origine phagique susmentionnées, comprenant la mise en culture de cellules transformées définies ci-dessus dans un milieu de culture approprié, et la purification des ARN polymérases produites par lesdites cellules.

L'invention a également pour objet tout procédé de préparation *in vitro* d'ARN d'intérêt, comprenant la mise en présence dans un milieu approprié d'au moins une ARN polymérase mutée telle d'origine phagique que définie ci-dessus, avec des séquences nucléotidiques déterminées comprenant une séquence d'ADN codant pour ledit ARN d'intérêt et dont la transcription est placée sous le contrôle d'un promoteur reconnu par les ARN polymérases sauvages et les ARN polymérases mutées susmentionnées.

L'invention concerne également tout procédé de préparation *in vivo* d'ARN d'intérêt, comprenant la mise en culture de cellules telles que définies ci-dessus, lesdites cellules produisant au moins une ARN polymérase mutée d'origine phagique selon l'invention, et dont le génome a été modifié de manière à contenir des séquences nucléotidiques déterminées comprenant une séquence d'ADN codant pour ledit ARN d'intérêt et dont la transcription est placée sous le contrôle d'un promoteur reconnu par les ARN polymérases sauvages et les ARN polymérases mutées susmentionnées.

L'invention a également pour objet tout procédé de préparation *in vivo* de protéines d'intérêt comprenant la mise en culture de cellules telles que définies ci-dessus, lesdites cellules produisant au moins une ARN polymérase mutée d'origine phagique selon l'invention, et dont le génome a été modifié de manière à contenir des séquences nucléotidiques déterminées comprenant une séquence d'ADN codant pour lesdites protéines d'intérêt et dont la transcription est placée sous le contrôle d'un promoteur reconnu par les ARN polymérases sauvages et les ARN polymérases mutées susmentionnées.

Avantageusement, dans le cas de la mise en oeuvre de procédés de préparation de protéines d'intérêt tels que décrits ci-dessus, les ARN polymérases mutées produites par les cellules, sont choisies parmi SEQ ID NO : 12, 14 et 16.

L'invention concerne également tout procédé de préparation *in vitro* ou *in vivo* d'ARN ou de protéines d'intérêt, tel que décrit ci-dessus, et comprenant en plus une étape d'addition de polyamines de synthèse telles que décrites par Frugier et al. Parmi les polyamines susceptibles d'être utilisées, on peut citer les suivantes :

NH₂(CH₂)₃NH(CH₂)₁₀NH(CH₂)₃NH₂

NH₂(CH₂)₃NH(CH₂)₃NH(CH₂)₁₀NH(CH₂)₃NH(CH₂)₃NH₂

L'invention sera davantage illustrée à l'aide de la description détaillée qui suit de l'obtention d'ARN polymérases mutées d'origine phagique selon l'invention, et de leur utilisation pour augmenter la production d'ARN déterminés par rapport à la production de ces mêmes ARN à l'aide d'ARN polymérases sauvages.

Alors que l'ARN polymérase du bactériophage T7 est normalement très processive en phase d'élongation, il n'en est pas de même durant la transcription de l'ITS: là, il existe une haute probabilité pour que le transcrit naissant soit prématurément relargué (transcription abortive) (Martin et al., 1988). C'est le nombre de cycles abortifs que l'enzyme doit réaliser, en moyenne, avant de transcrire l'ITS avec succès et d'entrer en phase d'élongation, qui détermine la fréquence avec laquelle les transcrits complets sont synthétisés (Ikeda, 1992). Ce nombre est minimal lorsque l'ITS correspond à la séquence 5' des gènes de classe III du bactériophage T7 (ITS 'consensuelle': 5' GGGAGA...). Toutefois, même dans ces conditions, les transcrits abortifs constituent une forte proportion (40% à 60%) des transcrits totaux. Cette proportion augmente très rapidement lorsque l'ITS s'éloigne du consensus, ou lorsque l'ARN polymérase porte des mutations qui diminuent sa vitesse d'élongation, augmentant le temps nécessaire à la traversée de l'ITS (Bonnet et al., 1994; Bonner et al., 1992). Éventuellement, la combinaison d'une ITS non consensuelle et d'une polymérase lente conduit à une situation où l'enzyme boucle indéfiniment en phase abortive: aucun transcrit de grande taille n'est alors synthétisé. Cette propriété peut être observée non seulement in vitro, mais aussi in vivo, dans des cellules d'Escherichia coli exprimant l'ARN polymérase de T7 (Lopez et al., 1997; Makarova et al., 1995). C'est cette dernière situation que nous avons exploitée pour sélectionner des mutants de l'ARN polymérase de T7 dont l'activité est moins sensible à la nature de l'ITS que celle de l'enzyme sauvage.

Nous avons placé un gène cible dont l'expression est facilement détectable - le gène lacZ, dont le produit est la β-galactosidase - précédé d'une ITS non consensuelle, sous le contrôle du promoteur T7. Ce gène est alors introduit dans une cellule bactérienne contenant en outre un plasmide codant pour une polymérase de T7 mutée dans son site catalytique (une polymérase 'lente', donc). Comme indiqué ci-dessus, le système, bloqué en phase abortive, ne produit pas de grands transcrits, et donc pas de β-galactosidase. Toutefois, après mutagenèse au hasard du plasmide, il est possible de sélectionner des clones bactériens qui synthétisent de nouveaux mutants de l'enzyme capables d'exprimer le gène cible. Outre des révertants de la mutation initiale du site catalytique, nous avons ainsi isolé des mutations dans la partie N-terminale de l'enzyme, qui n'est pas impliquée dans la catalyse. Lorsque ces nouvelles mutations ont été introduites dans l'enzyme sauvage, c'est à dire dépourvue de mutations au site catalytique, nous avons observé qu'elles diminuaient la susceptibilité de l'enzyme à la nature exacte de l'ITS. C'est le mutant ponctuel P266L qui est plus particulièrement décrit ici et fait l'objet de la présente invention. En fait, il permet de transcrire à des niveaux utilisables des matrices sur lesquelles l'enzyme sauvage est pratiquement inopérant.

### Descriptif détaillé

### a) Préparation, purification et stockage

Le mutant SEQ ID NO : 2 ponctuel obtenu correspond au changement dans la séquence de la polymérase de T7 de la Proline 266 en Leucine (P266L). Le gène de cette polymérase de T7 mutée est inclus dans le plasmide pAR1219 sous le contrôle d'un promoteur inductible (pLac UV5). La protéine (polymérase) est surexprimée dans la souche BL 21 (ompF-) d'E.coli. Les protocoles d'extraction, purification et stockage de la protéine purifiée sont identiques à ceux de la polymérase sauvage. Pour faciliter la purification la polymérase a été étiquetée par six histidines en N-terminal par transfert de la région mutée en lieu et place de la séquence codante équivalente du plasmide pBH 161. (He et al. 1977). Cette étiquette ne modifie en rien les propriétés catalytiques de la polymérase. Il suffit alors d'un passage du surnageant bactérien sur une colonne d'affinité au Nickel pour obtenir la protéine pure à >90%. La conservation de longue durée (2 ans) à -20°C n'altère pas les propriétés de l'enzyme.

### b) Activité catalytique.

Dans les conditions standard utilisées pour la polymérase sauvage, le mutant est aussi capable de convertir tous les rNTP qui lui sont fournis en ARN. Cependant la vitesse à laquelle cette incorporation est réalisée est environ trois fois plus petite pour le mutant c.a.d. qu'une transcription complète nécessitera un temps plus long (p.ex. 6 h au lieu de 2h).

Comme évoqué plus bas la principale distinction entre la polymérase sauvage et le mutant se situe au niveau de la distribution en fonction de la taille (et donc de la masse) des ARN obtenus. Deux types d'ARN sont observables à l'issue d'une transcription in vitro : les ARN abortifs et les ARN de grande taille.

Nous présentons ci dessous les résultats de transcriptions in vitro obtenus avec la polymérase sauvage et le mutant sur sept ITS très différentes. Les matrices sont des ADN plasmidiques (ca 6 kbp) linéarisés à des sites de restriction situés de 31 à 78 nt en aval du promoteur. Les réactions sont arrêtées à 10, 20 ou 30 mn suivant les cas et le marquage est α³²PGTP, α³²PUTP ou γ³²PGTP. Les conditions expérimentales utilisées sont décrites dans J. Mol. Biol. (1997) 269:41-51. Les transcrits sont séparés sur un gel de polyacrylamide dénaturant de haute résolution.

G10 (5' GGGAGACCA.. linéarisé à 33 nt) porte l'ITS consensuelle. De fait dans ce cas les 30 premiers nucléotides correspondent à la séquence du gène 10 du phage T7 l'un des mieux transcrits par la polymérase de T7.

GGLac (5' GGGGAAUU. linéarisé à 69 nt) porte l'ITS que l'on trouve dans le plasmide commercial pET15b (Novagen) qui comporte le site de fixation du répresseur Lac pour éventuellement réprimer la transcription T7.

Lac (5' GGAAUUG. linéarisé à 30 nt) est un plasmide équivalent au précédent dans lequel le site opérateur est rapproché de 2 nucléotides du promoteur.

TyrG (5' GUCUCGG. linéarisé à 78 nt), Gly (5' ACUCUUU. linéarisé à 71 nt), Tyr (5' CUCUCGG. linéarisé à 78 nt) et Val (5' GGUUUCG. linéarisé à 31 nt) sont des plasmides correspondant à des matrices destinées à synthétiser in vitro des ARNt ou des fragments d'ARNt et réputées difficiles à transcrire (Frugier et al., 1994)

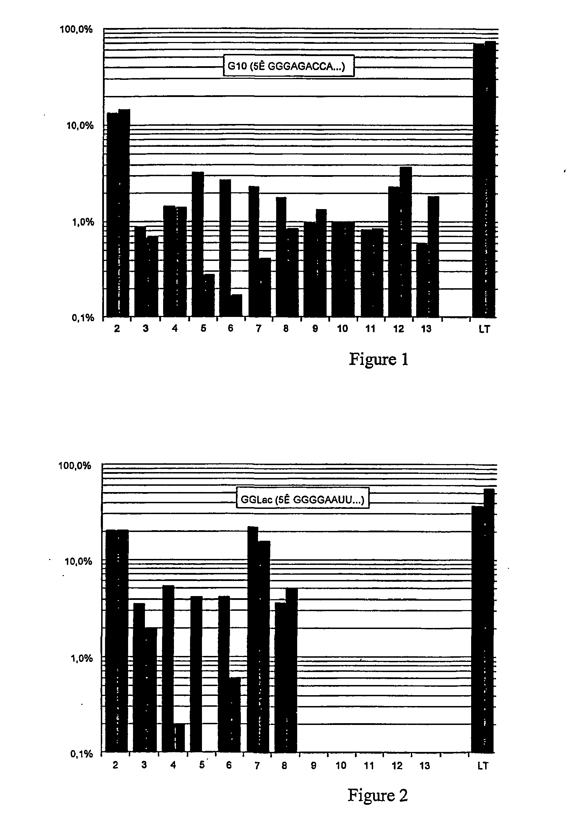

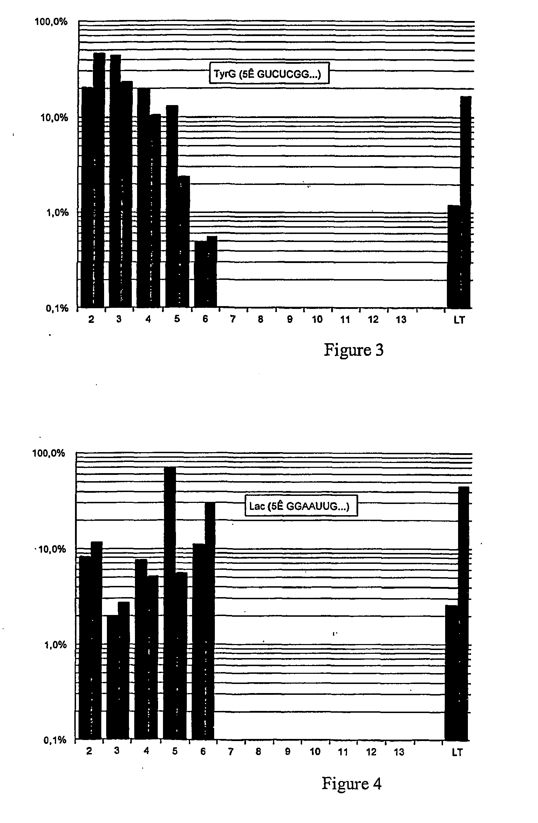

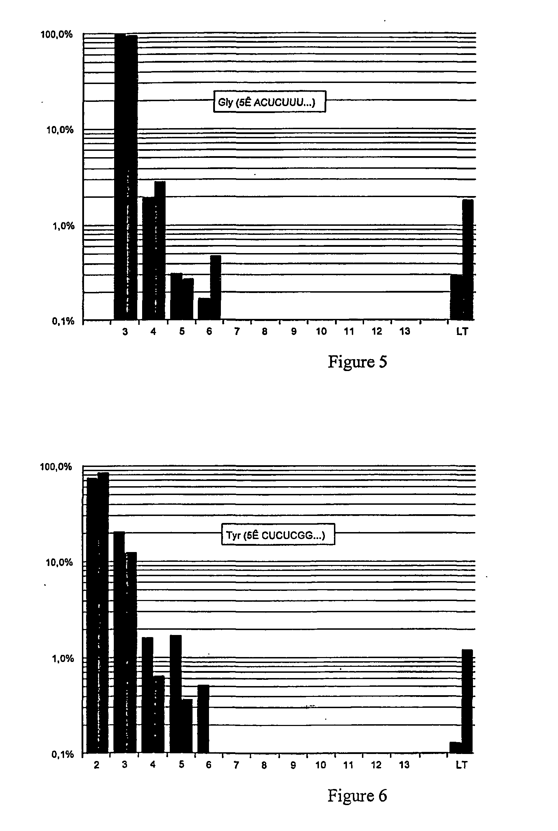

Sont présentés sur les figures 1 à 7, les résultats détaillés indiquant les ARN abortifs observés, leur pourcentage par rapport au nombre total de transcrits, ainsi que les chiffrages équivalents pour les longs transcrits qui on échappé à la phase abortive, dans le cas de l'utilisation de la polymérase T7 sauvage (encore désignée wt, et représentée à l'aide de colonnes noires) et de l'utilisation de l'ARN polymérase mutée SEQ ID NO: 2 (encore désignée P266L, et représentée à l'aide de colonnes grises) en présence de l'ITS G10 (figure 1), GGLac (figure 2), TyrG (figure 3), Lac (figure 4), Gly (figure 5), Tyr (figure 6), Val (figure 7). Les transcrits abortifs sont répartis entre le 2mer et le 13mer (numérotation 2 à 13 en abscisse). Les transcrits issus de polymérases qui ont échappé à la phase abortive sont identifiés longs transcrits (LT en abscisse). Une échelle logarithmique est utilisée en ordonnée pour permettre de quantifier les transcrits peu abondants.

Ces résultats montrent clairement que dans tous les cas le mutant a moins de difficulté que la polymérase sauvage pour incorporer les cinquième, sixième et septième nucléotides. Si à ces positions la polymérase a et/ou doit incorporer une pyrimidine (U ou C) le taux d'avortement est élevé pour la polymérase sauvage et la levée du handicap par le mutant d'autant plus importante.

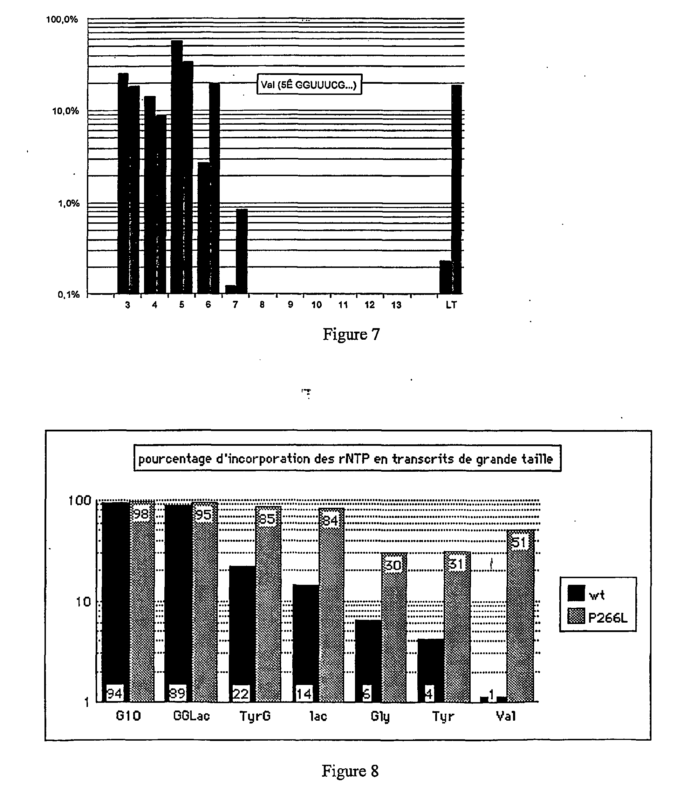

La conséquence immédiate est résumée sur la figure 8 qui reprend les résultats évoqués précédemment en chiffrant les productivités comparées des deux polymérases pour des ITS de plus en plus défavorables. Les valeurs indiquées correspondent au pourcentage d'incorporation des rNTP en transcrits de grande taille dans le cas de l'utilisation de la polymérase T7 sauvage (wt, colonnes noires) et de l'utilisation de l'ARN polymérase mutée SEQ ID NO : 2 (P266L, colonnes grises).

Mis à part G10 et GGLac où les deux polymérases donnent entière satisfaction, dans tous les autres cas la polymérase sauvage a un rendement faible, voire très faible, compris entre 22% et 1 %, alors que celui du mutant demeure raisonnable puisque compris entre 30% et 85%.

### BIBLIOGRAPHIE

Bonner, G., Lafer, E. M. & Sousa, R. (1994). Characterisation of a set of T7 RNA polymerase active site mutants. J. Biol. Chem. 269, 25120-25128.
Bonner, G., Patra, D., Lafer, E. M. & Sousa, R. (1992). Mutations in T7 RNA polymerase which support the proposal for a common polymerase active site structure. EMBO J. 11, 3767-3775.
Davanloo, P., Rosenberg, A. H., Dunn, J. J. & Studier, F. W. (1984). Cloning and expression of the gene for bacteriophage T7 RNA polymerase. Proc. Natl. Acad. Sci. USA 81, 2035-2039.
Frugier, M., C., F., M.W., H., J-M., L. & R., G. (1994). Synthetic polyamines stimulate in vitro transcription by T7 RNA polymerase. Nucl. Acid Res. 22, 2784-2790.
He, B., Rong, M., Lyakhov,D., Gartenstein, H.,Diaz, G.,Castagna,R.,McAllister,W.T. & Durbin, R. K. (1997). Rapid mutagenesis and purification of phage RNA polymerase. Protein Express Purif. 9,142-151.
Ikeda, R. A. (1992). The efficiency of promoter clearance distinguishes T7 class II and class III promoters. J. Biol. Chem. 267, 11322-11328.
Lapham J, Yu YT, Shu MD, Steitz JA, Crothers DM.(1997) The position of site-directed cleavage of RNA using RNase H and 2'-O-methyl oligonucléotides is dependent on the enzyme source. RNA 3,950-951
Li Z, Pandit S, Deutscher MP. (1999) . Maturation of 23S ribosomal RNA requires the exorbonuclease RNase T. RNA S,139-146
Lopez, P. J., Guillerez, J., Sousa, R. & Dreyfus, M. (1997). The low processivity of T7 RNA polymerase over the initially transcribed sequence can limit productive initiation in vivo. J. Mol. Biol. 269, 41-51.
Makarova, O. V., Makarov, E. M., Sousa, R. & Dreyfus, M. (1995). Transcribing Escherichia coli genes with mutant T7 RNA polymerases: stability of lacZ mRNA inversely correlates with polymerase speed. Proc. Natl. Acad. Sci. USA 92, 12250-12254.
Martin, C. T., Muller, D. K. & Coleman, J. E. (1988). Processivity in early stages of transcription by T7 RNA polymerase. Biochemistry 27, 3966-3974.
Milligan, J. F., Groebe, D. R., Witherell, G. W. & Uhlenbeck, O. C. (1987). Oligonucleotide synthesis using T7 RNA polymerase and synthetic DNA templates. Nucleic Acids Res. 15(21), 8783-8798.

## Revendications

1. Utilisation d'ARN polymérases mutées d'origine phagique moins sensibles que les ARN polymérases sauvages à la Séquence Initialement Transcrite ou ITS contenue dans la séquence d'ADN codant pour un ARN d'intérêt, ledites ARN polymérases mutées étant choisies parmi:
* celles dérivées de l'ARN polymérase sauvage T7, et comprenant au moins l'une des mutations suivantes :
- remplacement de l'isoleucine (I) en position 117 par une valine (V),
- remplacement de l'isoleucine (I) en position 119 par une valine (V),
- remplacement de la valine (V) en position 134 par une alanine (A),
- remplacement de l'acide aspartique (D) en position 147 par l'asparagine (N),
- remplacement de l'histidine (H) en position 230 par une arginine (R),
- remplacement de la proline (P) en position 266 par une leucine (L),
- remplacement de l'arginine (R) en position 291 par une cystéine (C),
* celles dérivées de l'ARN polymérase sauvage T3, et comprenant au moins l'une des mutations suivantes :
- remplacement de l'acide aspartique (D) en position 148 par l'asparagine (N),
- remplacement de la proline (P) en position 267 par une leucine (L),
- remplacement de l'arginine (R) en position 292 par une cystéine (C),
* celles dérivées de l'ARN polymérase sauvage K11, et comprenant au moins l'une des mutations suivantes :
- remplacement de l'acide aspartique (D) en position 167 par l'asparagine (N),
- remplacement de la proline (P) en position 289 par une leucine (L),
- remplacement de l'arginine (R) en position 314 par une cystéine (C),
* celles dérivées de l'ARN polymérase sauvage SP6, et comprenant au moins l'une des mutations suivantes :
- remplacement de l'acide aspartique (D) en position 117 par l'asparagine (N),
- remplacement de la proline (P) en position 239 par une leucine (L),
pour la mise en oeuvre d'un procédé de production d'ARN d'intérêt, ou de protéines codées par cet ARN d'intérêt, à partir de séquences nucléotidiques déterminées comprenant une séquence d'ADN codant pour ledit ARN d'intérêt et dont la transcription est placée sous le contrôle d'un promoteur reconnu par les ARN polymérases sauvages et les ARN polymérases mutées susmentionnées, ledit procédé de production ayant un rendement de production dudit ARN supérieur au rendement obtenu dans le cas de l'utilisation des ARN polymérases sauvages en présence de la même ITS non consensuelle que celle contenue dans la séquence d'ADN codant pour ledit ARN d'intérêt.

2. Utilisation selon la revendication 1, pour la mise en oeuvre d'un procédé de production d'ARN d'intérêt dont le rendement est jusqu'à environ 10 fois supérieur au rendement obtenu dans le cas de l'utilisation des ARN polymérases sauvages.

3. Utilisation selon l'une des revendications 1 à 2 des ARN polymérases mutées dérivées de l'ARN polymérase sauvage T7 telles que définies dans la revendication 1, comprenant au moins une des mutations supplémentaires suivantes :
- remplacement de la tyrosine (Y) en position 639 par une phénylalanine (F),
- remplacement de l'isoleucine (I) en position 810 par une asparagine (N).

4. Utilisation selon l'une des revendications 1 à 2 des ARN polymérases mutées dérivées de l'ARN polymérase sauvage T3 telles que définies dans la revendication 1, comprenant au moins une des mutations supplémentaires suivantes :
- remplacement de la tyrosine (Y) en position 640 par une phénylalanine (F),
- remplacement de l'isoleucine (I) en position 811 par une asparagine (N).

5. Utilisation selon l'une des revendications 1 à 2 des ARN polymérases mutées dérivées de l'ARN polymérase sauvage K11 telles que définies dans la revendication 1, comprenant au moins une des mutations supplémentaires suivantes :
- remplacement de la tyrosine (Y) en position 662 par une phénylalanine (F),
- remplacement de l'isoleucine (I) en position 833 par une asparagine (N).

6. Utilisation selon l'une des revendications 1 à .2 des ARN polymérases mutées dérivées de l'ARN polymérase sauvage SP6 telles que définies dans la revendication 1, comprenant au moins une des mutations supplémentaires suivantes :
- remplacement de la tyrosine (Y) en position 631 par une phénylalanine (F),
- remplacement de l'isoleucine (I) en position 804 par une asparagine (N).

7. Utilisation selon l'une des revendications 1 à 6 des ARN polymérases mutées suivantes :
- l'ARN polymérase T7 mutée représentée par SEQ ID NO : 2, comprenant une leucine en position 266 en substitution de la proline,
- l'ARN polymérase T7 mutée représentée par SEQ ID NO : 4, comprenant une valine en position 117 en substitution de l'isoleucine, et une alanine en position 134 en substitution de la valine,
- l'ARN polymérase T7 mutée représentée par SEQ ID NO : 6, comprenant une valine en position 119 en substitution de l'isoleucine, et une asparagine en position 147 en substitution de l'acide aspartique,
- l'ARN polymérase T7 mutée représentée par SEQ ID NO : 8, comprenant une arginine en position 230 en substitution de l'histidine, et une cystéine en position 291 en substitution de l'arginine,
- l'ARN polymérase T7 mutée représentée par SEQ ID NO : 10, comprenant une leucine en position 266 en substitution de la proline, et une phénylalanine en position 639 en substitution de la tyrosine,
- l'ARN polymérase T7 mutée représentée par SEQ ID NO : 12, comprenant une asparagine en position 810 en substitution de l'isoleucine,
- l'ARN polymérase T7 mutée représentée par SEQ ID NO : 14, comprenant une leucine en position 266 en substitution de la proline, et une asparagine en position 810 en substitution de l'isoleucine,
- l'ARN polymérase T7 mutée représentée par SEQ ID NO : 16, comprenant une valine en position 119 en substitution de l'isoleucine, une asparagine en position 147 en substitution de l'acide aspartique, et une asparagine en position 810 en substitution de l'isoleucine.

8. ARN polymérases mutées choisies parmi les suivantes :
* celles dérivées de l'ARN polymérase sauvage T7, et comprenant au moins l'une des mutations suivantes :
- remplacement de l'isoleucine (I) en position 117 par une valine (V),
- remplacement de l'isoleucine (I) en position 119 par une valine (V),
- remplacement de la valine (V) en position 134 par une alanine (A),
- remplacement de l'acide aspartique (D) en position 147 par l'asparagine (N),
- remplacement de l'histidine (H) en position 230 par une arginine (R),
- remplacement de la proline (P) en position 266 par une leucine (L),
- remplacement de l'arginine (R) en position 291 par une cystéine (C),
* celles dérivées de l'ARN polymérase sauvage T3, et comprenant au moins l'une des mutations suivantes :
- remplacement de l'acide aspartique (D) en position 148 par l'asparagine (N),
- remplacement de la proline (P) en position 267 par une leucine (L),
- remplacement de l'arginine (R) en position 292 par une cystéine (C),
* celles dérivées de l'ARN polymérase sauvage K11, et comprenant au moins l'une des mutations suivantes :
- remplacement de l'acide aspartique (D) en position 167 par l'asparagine (N),
- remplacement de la proline (P) en position 289 par une leucine (L),
- remplacement de l'arginine (R) en position 314 par une cystéine (C),
* celles dérivées de l'ARN polymérase sauvage SP6, et comprenant au moins l'une des mutations suivantes :
- remplacement de l'acide aspartique (D) en position 117 par l'asparagine (N),
- remplacement de la proline (P) en position 239 par une leucine (L).

9. ARN polymérases mutées selon la revendication 8, dérivées de l'ARN polymérase sauvage T7, et comprenant au moins une des mutations supplémentaires suivantes :
- remplacement de la tyrosine (Y) en position 639 par une phénylalanine (F),
- remplacement de l'isoleucine (I) en position 810 par une asparagine (N).

10. ARN polymérases mutées selon la revendication 8, dérivées de l'ARN polymérase sauvage T3, et comprenant au moins une des mutations supplémentaires suivantes :
- remplacement de la tyrosine (Y) en position 640 par une phénylalanine (F),
- remplacement de l'isoleucine (I) en position 811 par une asparagine (N).

11. ARN polymérases mutées selon la revendication 8, dérivées de l'ARN polymérase sauvage K11, et comprenant au moins une des mutations supplémentaires suivantes :
- remplacement de la tyrosine (Y) en position 662 par une phénylalanine (F),
- remplacement de l'isoleucine (I) en position 833 par une asparagine (N).

12. ARN polymérases mutées selon la revendication 8, dérivées de l'ARN polymérase sauvage SP6, et comprenant au moins une des mutations supplémentaires suivantes :
- remplacement de la tyrosine (Y) en position 631 par une phénylalanine (F),
- remplacement de l'isoleucine (I) en position 804 par une asparagine (N).

13. ARN polymérases mutées selon l'une des revendications 8 à 12, choisies parmi :
- l'ARN polymérase T7 mutée représentée par SEQ ID NO : 2, comprenant une leucine en position 266 en substitution de la proline,
- l'ARN polymérase T7 mutée représentée par SEQ ID NO : 4, comprenant une valine en position 117 en substitution de l'isoleucine, et une alanine en position 134 en substitution de la valine,
- l'ARN polymérase T7 mutée représentée par SEQ ID NO : 6, comprenant une valine en position 119 en substitution de l'isoleucine, et une asparagine en position 147 en substitution de l'acide aspartique,
- l'ARN polymérase T7 mutée représentée par SEQ ID NO : 8, comprenant une arginine en position 230 en substitution de l'histidine, et une cystéine en position 291 en substitution de l'arginine,
- l'ARN polymérase T7 mutée représentée par SEQ ID NO : 10, comprenant une leucine en position 266 en substitution de la proline, et une phénylalanine en position 639 en substitution de la tyrosine,
- l'ARN polymérase T7 mutée représentée par SEQ ID NO : 12, comprenant une asparagine en position 810 en substitution de l'isoleucine,
- l'ARN polymérase T7 mutée représentée par SEQ ID NO : 14, comprenant une leucine en position 266 en substitution de la proline, et une asparagine en position 810 en substitution de l'isoleucine,
- l'ARN polymérase T7 mutée représentée par SEQ ID NO : 16, comprenant une valine en position 119 en substitution de l'isoleucine, une asparagine en position 147 en substitution de l'acide aspartique, et une asparagine en position 810 en substitution de l'isoleucine.

14. Séquence nucléotidique codant pour une ARN polymérase selon l'une des revendications 8 à 13, choisies parmi les séquences SEQ ID NO : 1, 3, 5, 7, 9, 11, 13, 15, codant respectivement pour les protéines SEQ ID NO : 2, 4, 6, 8, 10, 12, 14, 16, ou toute séquence nucléotidique dérivée des séquences nucléotidiques susmentionnées par dégénérescence du code génétique et conservant la propriété de coder pour les protéines susmentionnées.

15. Vecteur contenant une séquence nucléotidique selon la revendication 14.

16. Cellule transformée par un vecteur selon la revendication 15.

17. Procédé de préparation d'ARN polymérases mutées d'origine phagique selon l'une des revendications 8 à 13, dont l'activité de transcription d'une séquence nucléotidique déterminée comprenant une séquence d'ADN codant pour un ARN d'intérêt et dont la transcription est placée sous le contrôle d'un promoteur reconnu par les ARN polymérases mutées et par les ARN polymérases sauvages, dont elles sont issues, est supérieure à l'activité de transcription de cette séquence déterminée par les ARN polymérases sauvages, en présence de la même ITS non consensuelle que celle contenue dans la séquence d'ADN codant pour ledit ARN d'intérêt, lesdites ARN polymérases mutées étant jusqu'à environ 40 fois plus actives que lesdites ARN polymérases sauvages dans le cadre de la mise en oeuvre de procédés de production d'ARN d'intérêt à partir de ladite séquence nucléotidique déterminée, ledit procédé comprenant :
- une étape de modification de la chaîne peptidique des ARN polymérases sauvages d'origine phagique par substitution, ou délétion ou addition d'au moins un codon du gène codant pour lesdites ARN polymérases sauvages, et transformation de cellules appropriées comme *E.coli,* avec des vecteurs contenant le gène ainsi modifié ainsi qu'une séquence nucléotidique codant pour un marqueur, tel qu'un marqueur de résistance à un antibiotique, ou un marqueur chromogène, insérée en aval du promoteur reconnu par les ARN polymérases susmentionnées, ce promoteur et la séquence codant pour le marqueur étant séparés par une ITS,
- une étape de détection des cellules susmentionnées produisant des ARN polymérases mutées pour lesquelles le rendement de la production du marqueur particulier, est supérieur au rendement de la production de ce même marqueur obtenu par utilisation des ARN polymérases sauvages dans les mêmes conditions, et
- une étape de purification des ARN polymérases mutées susmentionnées à partir des cellules détectées à l'étape précédente.

18. Procédé de préparation *in vitro* d'ARN d'intérêt, comprenant la mise en présence dans un milieu approprié d'au moins une ARN polymérase mutée selon l'une des revendications 8 à 13, avec des séquences nucléotidiques déterminées comprenant une séquence d'ADN codant pour ledit ARN d'intérêt et dont la transcription est placée sous le contrôle d'un promoteur reconnu par les ARN polymérases sauvages et les ARN polymérases mutées susmentionnées.

19. Procédé de préparation *in vivo* d'ARN d'intérêt, comprenant la mise en culture de cellules selon la revendication 16, lesdites cellules produisant au moins une ARN polymérase mutée, selon l'une des revendications 8 à 13, et dont le génome a été modifié de manière à contenir des séquences nucléotidiques déterminées comprenant une séquence d'ADN codant pour ledit ARN d'intérêt et dont la transcription est placée sous le contrôle d'un promoteur reconnu par les ARN polymérases sauvages et les ARN polymérases mutées susmentionnées.

20. Procédé de préparation *in vivo* de protéines d'intérêt comprenant la mise en culture de cellules selon la revendication 16, lesdites cellules produisant au moins une ARN polymérase mutée telle qu'obtenue selon le procédé tel que défini dans la revendication 17 ou une ARN polymérase mutée selon l'une des revendications 8 à 13, et dont le génome a été modifié de manière à contenir des séquences nucléotidiques déterminées comprenant une séquence d'ADN codant pour lesdites protéines d'intérêt et dont la transcription est placée sous le contrôle d'un promoteur reconnu par les ARN polymérases sauvages et les ARN polymérases mutées susmentionnées.

21. Procédé de préparation *in vitro* ou *in vivo* d'ARN ou de protéines d'intérêt, selon l'une des revendications 18 à 20, comprenant également une étape d'addition de polyamines de synthèse.

## Claims

1. Use of mutated RNA polymerases of phage origin less sensitive than the wild-type RNA polymerases, to the Initially Transcribed Sequences or ITS contained in the DNA sequence coding for an RNA of interest, said mutated RNA polymerases being chosen from:
* those derived from the wild-type T7 RNA polymerase, and comprising at least one of the following mutations:
- replacement of the isoleucine (I) in position 117 by a valine (V),
- replacement of the isoleucine (I) in position 119 by a valine (V),
- replacement of the valine (V) in position 134 by an alanine (A),
- replacement of the aspartic acid (D) in position 147 by asparagine (N),
- replacement of the histidine (H) in position 230 by an arginine (R),
- replacement of the proline (P) in position 266 by a leucine (L),
- replacement of the arginine (R) in position 291 by a cysteine (C),
* those derived from the wild-type T3 RNA polymerase, and comprising at least one of the following mutations:
- replacement of the aspartic acid (D) in position 148 by asparagine (N),
- replacement of the proline (P) in position 267 by a leucine (L),
- replacement of the arginine (R) in position 292 by a cysteine (C),
* those derived from the wild-type K11 RNA polymerase, and comprising at least one of the following mutations:
- replacement of the aspartic acid (D) in position 167 by asparagine (N),
- replacement of the proline (P) in position 289 by a leucine (L),
- replacement of the arginine (R) in position 314 by a cysteine (C)
* those derived from the wild-type SP6 RNA polymerase, and comprising at least one of the following mutations:
- replacement of the aspartic acid (D) in position 117 by asparagine (N),
- replacement of the proline (P) in position 239 by a leucine (L),
for the implementation of a process for producing said RNA of interest, or proteins coded by this RNA of interest, starting from determined nucleotide sequences comprising a DNA sequence coding for said RNA of interest and the transcription of which is placed under the control of a promoter recognized by the abovementioned
wild-type RNA polymerases and mutated RNA polymerases, said process having a production yield of said RNA greater than the yield obtained in the case of use of the wild-type RNA polymerases in the presence of the same non-consensual ITS as that contained in the DNA sequence coding for said RNA of interest.

2. Use according to claim 1, for the implementation of a process for producing RNA of interest, the yield of which is up to approximately 10 times greater than the yield obtained in the case of use of the wild-type RNA polymerases.

3. Use according to claim 1 or 2, of mutated RNA polymerases originating from wild-type T7 polymerases according to claim 1, comprising at least one of the following additional mutations:
- replacement of the tyrosine (Y) in position 639 by a phenylalanine (F),
- replacement of the isoleucine (I) in position 810 by an asparagine (N).

4. Use according to claim 1 or 2, of mutated RNA polymerases originating from wild-type T3 polymerases according to claim 1, comprising at least one of the following additional mutations:
- replacement of the tyrosine (Y) in position 640 by a phenylalanine (F),
- replacement of the isoleucine (I) in position 811 by an asparagine (N).

5. Use according to claim 1 or 2, of mutated RNA polymerases originating from wild-type K11 polymerases according to claim 1, comprising at least one of the following additional mutations:
- replacement of the tyrosine (Y) in position 662 by a phenylalanine (F),
- replacement of the isoleucine (I) in position 833 by an asparagine (N).

6. Use according to claim 1 or 2, of mutated RNA polymerases originating from wild-type SP6 polymerases according to claim 1, comprising at least one of the following additional mutations:
- replacement of the tyrosine (Y) in position 631 by a phenylalanine (F),
- replacement of the isoleucine (I) in position 804 by an asparagine (N).

7. Use according to one of claims 1 to 6 of the following mutated RNA polymerases:
- the mutated T7 RNA polymerase represented by SEQ ID NO: 2, comprising a leucine in position 266 substituted for the proline,
- the mutated T7 RNA polymerase represented by SEQ ID NO: 4, comprising a valine in position 117 substituted for the isoleucine, and an alanine in position 134 substituted for the valine,
- the mutated T7 RNA polymerase represented by SEQ ID NO: 6, comprising a valine in position 119 substituted for the isoleucine, and an asparagine in position 147 substituted for the aspartic acid,
- the mutated T7 RNA polymerase represented by SEQ ID NO: 8, comprising an arginine in position 230 substituted for the histidine, and a cysteine in position 291 substituted for the arginine,
- the mutated T7 RNA polymerase represented by SEQ ID NO: 10, comprising a leucine in position 266 substituted for the proline, and a phenylalanine in position 639 substituted for the tyrosine,
- the mutated T7 RNA polymerase represented by SEQ ID NO: 12, comprising an asparagine in position 810 substituted for the isoleucine,
- the mutated T7 RNA polymerase represented by SEQ ID NO: 14, comprising a leucine in position 266 substituted for the proline, and an asparagine in position 810 substituted for the isoleucine,
- the mutated T7 RNA polymerase represented by SEQ ID NO: 16, comprising a valine in position 119 substituted for the isoleucine, an asparagine in position 147 substituted for the aspartic acid, and an asparagine in position 810 substituted for the isoleucine.

8. Mutated RNA polymerases chosen from the following :
* those derived from the wild-type T7 RNA polymerase, and comprising at least one of the following mutations:
- replacement of the isoleucine (I) in position 117 by a valine (V),
- replacement of the isoleucine (I) in position 119 by a valine (V),
- replacement of the valine (V) in position 134 by an alanine (A),
- replacement of the aspartic acid (D) in position 147 by asparagine (N),
- replacement of the histidine (H) in position 230 by an arginine (R),
- replacement of the proline (P) in position 266 by a leucine (L),
- replacement of the arginine (R) in position 291 by a cysteine (C),
* those derived from the wild-type T3 RNA polymerase, and comprising at least one of the following mutations:
- replacement of the aspartic acid (D) in position 148 by asparagine (N),
- replacement of the proline (P) in position 267 by a leucine (L),
- replacement of the arginine (R) in position 292 by a cysteine (C),
* those derived from the wild-type K11 RNA polymerase, and comprising at least one of the following mutations:
- replacement of the aspartic acid (D) in position 167 by asparagine (N),
- replacement of the proline (P) in position 289 by a leucine (L),
- replacement of the arginine (R) in position 314 by a cysteine (C),
* those derived from the wild-type SP6 RNA polymerase, and comprising at least one of the following mutations:
- replacement of the aspartic acid (D) in position 117 by asparagine (N),
- replacement of the proline (P) in position 239 by a leucine (L),

9. Mutated RNA polymerases according to claim 8, originating from wild-type T7 polymerases, comprising at least one of the following additional mutations:
- replacement of the tyrosine (Y) in position 639 by a phenylalanine (F),
- replacement of the isoleucine (I) in position 810 by an asparagine (N).

10. Mutated RNA polymerases according to claim 8, originating from wild-type T3 polymerases, comprising at least one of the following additional mutations:
- replacement of the tyrosine (Y) in position 640 by a phenylalanine (F),
- replacement of the isoleucine (I) in position 811 by an asparagine (N).

11. Mutated RNA polymerases according to claim 8, originating from wild-type K11 polymerases, comprising at least one of the following additional mutations:
- replacement of the tyrosine (Y) in position 662 by a phenylalanine (F),
- replacement of the isoleucine (I) in position 833 by an asparagine (N).

12. Mutated RNA polymerases according to claim 8, originating from wild-type SP6 polymerases, comprising at least one of the following additional mutations:
- replacement of the tyrosine (Y) in position 631 by a phenylalanine (F),
- replacement of the isoleucine (I) in position 804 by an asparagine (N).

13. Mutated RNA polymerases, according to one of the claims 8 to 12, chosen from :
- the mutated T7 RNA polymerase represented by SEQ ID NO: 2, comprising a leucine in position 266 substituted for the proline,
- the mutated T7 RNA polymerase represented by SEQ ID NO: 4, comprising a valine in position 117 substituted for the isoleucine, and an alanine in position 134 substituted for the valine,
- the mutated T7 RNA polymerase represented by SEQ ID NO: 6, comprising a valine in position 119 substituted for the isoleucine, and an asparagine in position 147 substituted for the aspartic acid,
- the mutated T7 RNA polymerase represented by SEQ ID NO: 8, comprising an arginine in position 230 substituted for the histidine, and a cysteine in position 291 substituted for the arginine,
- the mutated T7 RNA polymerase represented by SEQ ID NO: 10, comprising a leucine in position 266 substituted for the proline, and a phenylalanine in position 639 substituted for the tyrosine,
- the mutated T7 RNA polymerase represented by SEQ ID NO: 12, comprising an asparagine in position 810 substituted for the isoleucine,
- the mutated T7 RNA polymerase represented by SEQ ID NO: 14, comprising a leucine in position 266 substituted for the proline, and an asparagine in position 810 substituted for the isoleucine,
- the mutated T7 RNA polymerase represented by SEQ ID NO: 16, comprising a valine in position 119 substituted for the isoleucine, an asparagine in position 147 substituted for the aspartic acid, and an asparagine in position 810 substituted for the isoleucine.

14. Nucleotide sequences coding respectively for the RNA polymerase of one of the claims 8 to 13, chosen from the sequences SEQ ID NO: 1, 3, 5, 7, 9, 11, 13, 15, coding respectively for the proteins SEQ ID NO: 2, 4, 6, 8, 10, 12, 14, 16, or any nucleotide sequence derived from the abovementioned nucleotide sequences by degeneration of the genetic code and retaining the property of coding for the abovementioned proteins.

15. Vector containing a nucleotide sequence according to claim 14.

16. Cell transformed by a vector according to claim 15.

17. Process for preparing mutated RNA polymerases of phage origin according to one of the claims 8 to 13, of which the determined nucleotide sequence transcription activity, comprising a DNA sequence coding for an RNA of interest and the transcription of which is placed under the control of a promoter recognized by the mutated RNA polymerases and by the wild-type RNA polymerases from which they originate, is greater than the transcription activity of this determined sequence by the wild-type RNA polymerases, in particular for preparing mutated RNA polymerases being up to approximately 40 times more active than said wild-type RNA polymerases within the scope of the implementation of processes for producing RNA of interest from said determined nucleotide sequence, said process comprising:
- a step of modification of the peptide chain of the wild-type RNA polymerases of phage origin by substitution, or deletion or addition of at least one codon of the gene coding for said wild-type RNA polymerases, and transformation of appropriate cells, such as *E.coli,* with vectors containing the gene thus modified, and a nucleotide sequence coding for a marker, such as a marker of resistance to an antibiotic, or a chromogenic marker, inserted downstream of the promoter recognized by the abovementioned RNA polymerases, this promoter and the sequence coding for the marker being separated by an ITS,
- a step of detection of the abovementioned cells producing mutated RNA polymerases for which the production yield of a particular marker, is greater than the production yield of this same marker obtained by use of the wild-type RNA polymerases under the same conditions,
- a step of purification of the abovementioned mutated RNA polymerases from the cells detected in the preceding step.

18. Process for preparing *in vitro* RNA of interest, comprising the bringing together, in an appropriate medium, of at least one mutated RNA polymerase according to one of claims 8 to 13, with the determined nucleotide sequences comprising a DNA sequence coding for said RNA of interest and the transcription of which is placed under the control of a promoter recognized by the abovementioned wild-type RNA polymerases and mutated RNA polymerases.

19. Process for preparing *in vivo* RNA of interest, comprising the culture of cells according to claim 16, said cells producing at least one mutated RNA polymerase according to one of claims 8 to 13, and the genome of which has been modified in order to contain determined nucleotide sequences comprising a DNA sequence coding for said RNA of interest and the transcription of which is placed under the control of a promoter recognized by the abovementioned wild-type RNA polymerases and mutated RNA polymerases.

20. Process for preparing *in vivo* proteins of interest comprising the culture of cells according to claim 16, said cells producing at least one mutated RNA polymerase according to the process of claim 17 or one mutated RNA polymerase according to one of claims 8 to 13, and the genome of which has been modified in order to contain determined nucleotide sequences comprising a DNA sequence coding for said proteins of interest and the transcription of which is placed under the control of a promoter recognized by the abovementioned wild-type RNA polymerases and mutated RNA polymerases.

21. Process for preparing *in vitro* or *in vivo* RNA or proteins of interest, according to one of claims 18 to 20, also comprising a stage of addition of synthetic polyamines.

## Patentansprüche

1. Verwendung mutierter RNA-Polymerasen phagischen Ursprungs, die gegenüber der initial transkribierten Sequenz oder ITS, die in der DNA-Sequenz enthalten ist, die für eine interessierende RNA kodiert, weniger empfindlich sind als wilde RNA-Polymerasen, wobei die mutierten RNA-Polymerasen ausgewählt sind aus:
* jenen, die von der wilden RNA-Polymerase T7 abgeleitet sind, und mindestens eine der folgenden Mutationen umfassen:
- Ersatz des Isoleucins (I) an Position 117 durch ein Valin (V),
- Ersatz des Isoleucins (I) an Position 119 durch ein Valin (V),
- Ersatz des Valins (V) an Position 134 durch ein Alanin (A),
- Ersatz der Asparaginsäure (D) an Position 147 durch das Asparagin (N),
- Ersatz des Histidins (H) an Position 230 durch ein Arginin (R),
- Ersatz des Prolins (P) an Position 266 durch ein Leucin (L),
- Ersatz des Arginins (R) an Position 291 durch ein Cystein (C),
* jenen, die von der wilden RNA-Polymerase T3 abgeleitet sind, und mindestens eine der folgenden Mutationen umfassen:
- Ersatz der Asparaginsäure (D) an Position 148 durch das Asparagin (N),
- Ersatz des Prolins (P) an Position 267 durch ein Leucin (L),
- Ersatz des Arginins (R) an Position 292 durch ein Cystein (C),
* jenen, die von der wilden RNA-Polymerase K11 abgeleitet sind, und mindestens eine der folgenden Mutationen umfassen:
- Ersatz der Asparaginsäure (D) an Position 167 durch das Asparagin (N),
- Ersatz des Prolins (P) an Position 289 durch ein Leucin (L),
- Ersatz des Arginins (R) an Position 314 durch ein Cystein (C),
* jenen, die von der wilden RNA-Polymerase SP6 abgeleitet sind, und mindestens eine der folgenden Mutationen umfassen:
- Ersatz der Asparaginsäure (D) an Position 117 durch das Asparagin (N),
- Ersatz des Prolins (P) an Position 239 durch ein Leucin (L),
zur Durchführung eines Verfahrens zur Produktion interessierender RNA oder Proteinen die bei der interessierender RNA kodiert sind, aus bestimmten Nukleotidsequenzen, die eine DNA-Sequenz umfassen, die für die interessierende RNA kodiert und deren Transkription unter die Kontrolle eines Promoters gesetzt wird, der durch die wilden RNA-Polymerasen und die oben genannten mutierten RNA-Polymerasen erkannt wird, wobei das Verfahren zur Produktion eine Produktionsausbeute an RNA aufweist, die höher ist als die Ausbeute, die im Fall der Verwendung der wilden RNA-Polymerasen erhalten wird, in Gegenwart der gleichen nicht konsensuellen ITS wie jene, die in der DNA-Sequenz enthalten ist, die für die interessierend RNA kodiert.

2. Verwendung nach Anspruch 1 zur Durchführung eines Verfahrens zur Produktion interessierender RNA, deren Ausbeute bis zu etwa 10 Mal höher ist als die Ausbeute, die im Fall der Verwendung wilder RNA-Polymerasen erhalten wird.

3. Verwendung nach Anspruch 1 bis 2 der mutierten RNA-Polymerasen, die von der wilden RNA-Polymerase T7 abgeleitet sind, wie in Anspruch 1 definiert, die mindestens eine der folgenden zusätzlichen Mutationen umfassen:
- Ersatz des Tyrosins (Y) an Position 639 durch ein Phenylalanin (F),
- Ersatz des Isoleucins (I) an Position 810 durch ein Asparagin (N),

4. Verwendung nach einem der Ansprüche 1 bis 2 der mutierten RNA-Polymerasen, die von der wilden RNA-Polymerase T3 abgeleitet sind, wie in Anspruch 1 definiert, die mindestens eine der folgenden zusätzlichen Mutationen umfassen:
- Ersatz des Tyrosins (Y) an Position 640 durch ein Phenylalanin (F),
- Ersatz des Isoleucins (I) an Position 811 durch ein Asparagin (N),

5. Verwendung nach einem der Ansprüche 1 bis 2 der mutierten RNA-Polymerasen, die von der wilden RNA-Polymerase K11 abgeleitet sind, wie in Anspruch 1 definiert, die mindestens eine der folgenden zusätzlichen Mutationen umfassen:
- Ersatz des Tyrosins (Y) an Position 662 durch ein Phenylalanin (F),
- Ersatz des Isoleucins (I) an Position 833 durch ein Asparagin (N),

6. Verwendung nach einem der Ansprüche 1 bis 2 der mutierten RNA-Polymerasen, die von der wilden RNA-Polymerase SP6 abgeleitet sind, wie in Anspruch 1 definiert, die mindestens eine der folgenden zusätzlichen Mutationen umfassen:
- Ersatz des Tyrosins (Y) an Position 631 durch ein Phenylalanin (F),
- Ersatz des Isoleucins (I) an Position 804 durch ein Asparagin (N),

7. Verwendung nach einem der Ansprüche 1 bis 6 der folgenden mutierten RNA-Polymerasen:
- mutierte RNA-Polymerase T7, die durch SEQ ID NO: 2 dargestellt wird, die ein Leucin an Position 266 umfasst, das das Prolin substituiert,
- mutierte RNA-Polymerase T7, die durch SEQ ID NO: 4 dargestellt wird, die ein Valin an Position 117, das das Isoleucin substituiert, und ein Alanin an Position 134 umfasst, das das Valin substituiert,
- mutierte RNA-Polymerase T7, die durch SEQ ID NO: 6 dargestellt wird, die ein Valin an Position 119, das das Isoleucin substituiert, und ein Asparagin an Position 147 umfasst, das die Asparaginsäure substituiert,
- mutierte RNA-Polymerase T7, die durch SEQ ID NO: 8 dargestellt wird, die ein Arginin an Position 230, das das Histidin substituiert, und ein Cystein an Position 291 umfasst, das das Arginin substituiert,
- mutierte RNA-Polymerase T7, die durch SEQ ID NO: 10 dargestellt wird, die ein Leucin an Position 266, das das Prolin substituiert, und ein Phenylalanin an Position 639 umfasst, das das Tyrosin substituiert,
- mutierte RNA-Polymerase T7, die durch SEQ ID NO: 12 dargestellt wird, die ein Asparagin an Position 810 umfasst, das das Isoleucin substituiert,
- mutierte RNA-Polymerase T7, die durch SEQ ID NO: 14 dargestellt wird, die ein Leucin an Position 266, das das Prolin substituiert, und ein Asparagin an Position 810 umfasst, das das Isoleucin substituiert,
- mutierte RNA-Polymerase T7, die durch SEQ ID NO: 16 dargestellt wird, die ein Valin an Position 119, das das Isoleucin substituiert, und ein Asparagin an Position 147, das die Asparaginsäure substituiert, und ein Asparagin an Position 810 umfasst, das das Isoleucin substituiert.

8. Mutierte RNA-Polymerasen, die aus den Folgenden ausgewählt sind:
* jenen, die von der wilden RNA-Polymerase T7 abgeleitet sind, und mindestens eine der folgenden Mutationen umfassen:
- Ersatz des Isoleucins (I) an Position 117 durch ein Valin (V),
- Ersatz des Isoleucins (I) an Position 119 durch ein Valin (V),
- Ersatz des Valins (V) an Position 134 durch ein Alanin (A),
- Ersatz der Asparaginsäure (D) an Position 147 durch das Asparagin (N),
- Ersatz des Histidins (H) an Position 230 durch ein Arginin (R),
- Ersatz des Prolins (P) an Position 266 durch ein Leucin (L),
- Ersatz des Arginins (R) an Position 291 durch ein Cystein (C),
* jenen, die von der wilden RNA-Polymerase T3 abgeleitet sind, und mindestens eine der folgenden Mutationen umfassen:
- Ersatz der Asparaginsäure (D) an Position 148 durch das Asparagin (N),
- Ersatz des Prolins (P) an Position 267 durch ein Leucin (L),
- Ersatz des Arginins (R) an Position 292 durch ein Cystein (C),
* jenen, die von der wilden RNA-Polymerase K11 abgeleitet sind, und mindestens eine der folgenden Mutationen umfassen:
- rsatz der Asparaginsäure (D) an Position 167 durch das Asparagin (N),
- Ersatz des Prolins (P) an Position 289 durch ein Leucin (L),
- Ersatz des Arginins (R) an Position 314 durch ein Cystein (C),
* jenen, die von der wilden RNA-Polymerase SP6 abgeleitet sind, und mindestens eine der folgenden Mutationen umfassen:
- Ersatz der Asparaginsäure (D) an Position 117 durch das Asparagin (N),
- Ersatz des Prolins (P) an Position 239 durch ein Leucin (L).

9. Mutierte RNA-Polymerasen nach Anspruch 8, die von der wilden RNA-Polymerase T7 abgeleitet sind, und die mindestens eine der folgenden zusätzlichen Mutationen umfassen:
- Ersatz des Tyrosins (Y) an Position 639 durch ein Phenylalanin (F),
- Ersatz des Isoleucins (I) an Position 810 durch ein Asparagin (N),

10. Mutierte RNA-Polymerasen nach Anspruch 8, die von der wilden RNA-Polymerase T3 abgeleitet sind, und die mindestens eine der folgenden zusätzlichen Mutationen umfassen:
- Ersatz des Tyrosins (Y) an Position 640 durch ein Phenylalanin (F),
- Ersatz des Isoleucins (I) an Position 811 durch ein Asparagin (N),

11. Mutierte RNA-Polymerasen nach Anspruch 8, die von der wilden RNA-Polymerase K11 abgeleitet sind, und die mindestens eine der folgenden zusätzlichen Mutationen umfassen:
- Ersatz des Tyrosins (Y) an Position 662 durch ein Phenylalanin (F),
- Ersatz des Isoleucins (I) an Position 833 durch ein Asparagin (N),

12. Mutierte RNA-Polymerasen nach Anspruch 8, die von der wilden RNA-Polymerase SP6 abgeleitet sind, und die mindestens eine der folgenden zusätzlichen Mutationen umfassen:
- Ersatz des Tyrosins (Y) an Position 631 durch ein Phenylalanin (F),
- Ersatz des Isoleucins (I) an Position 804 durch ein Asparagin (N),

13. Mutierte RNA-Polymerasen nach einem der Ansprüche 8 bis 12, ausgewählt aus:
- der mutierten RNA-Polymerase T7, die durch SEQ ID NO: 2 dargestellt wird, die ein Leucin an Position 266 umfasst, das das Prolin substituiert,
- der mutierten RNA-Polymerase T7, die durch SEQ ID NO: 4 dargestellt wird, die ein Valin an Position 117, das das Isoleucin substituiert, und ein Alanin an Position 134 umfasst, das das Valin substituiert,
- der mutierten RNA-Polymerase T7, die durch SEQ ID NO: 6 dargestellt wird, die ein Valin an Position 119, das das Isoleucin substituiert, und ein Asparagin an Position 147 umfasst, das die Asparaginsäure substituiert,
- der mutierten RNA-Polymerase T7, die durch SEQ ID NO: 8 dargestellt wird, die ein Arginin an Position 230, das das Histidin substituiert, und ein Cystein an Position 291 umfasst, das das Arginin substituiert,
- der mutierten RNA-Polymerase T7, die durch SEQ ID NO: 10 dargestellt wird, die ein Leucin an Position 266, das das Prolin substituiert, und ein Phenylalanin an Position 639 umfasst, das das Tyrosin substituiert,
- der mutierten RNA-Polymerase T7, die durch SEQ ID NO: 12 dargestellt wird, die ein Asparagin an Position 810 umfasst, das das Isoleucin substituiert,
- der mutierten RNA-Polymerase T7, die durch SEQ ID NO: 14 dargestellt wird, die ein Leucin an Position 266, das das Prolin substituiert, und ein Asparagin an Position 810 umfasst, das das Isoleucin substituiert,
- der mutierten RNA-Polymerase T7, die durch SEQ ID NO: 16 dargestellt wird, die ein Valin an Position 119, das das Isoleucin substituiert, und ein Asparagin an Position 147, das die Asparaginsäure substituiert, und ein Asparagin an Position 810 umfasst, das das Isoleucin substituiert.

14. Nukleotidsequenz, die für eine RNA-Polymerase nach einem der Ansprüche 8 bis 13 kodiert, ausgewählt aus den Sequenzen SEQ ID NO: 1, 3, 5, 7, 9, 11, 13, 15, die jeweils für die Proteine SEQ ID NO: 2, 4, 6, 8, 10, 12, 14, 16 kodieren, oder jede Nukleotidsequenz, die von den oben genannten Nukleotidsequenzen durch Degeneration des genetischen Codes abgeleitet ist; und die Eigenschaft konserviert, für die oben genannten Proteine zu kodieren.

15. Vektor, der eine Nukleotidsequenz nach Anspruch 14 enthält.

16. Zelle, die durch einen Vektor nach Anspruch 15 transformiert ist.

17. Verfahren zur Herstellung von mutierten RNA-Polymerasen phagischen Ursprungs nach einem der Ansprüche 8 bis 13, deren Transkriptionsaktivität einer bestimmten Nukleotidsequenz, die eine DNA-Sequenz umfasst, die für eine interessieren RNA kodiert, und deren Transkription unter die Kontrolle eines Promoters gesetzt wird, der von den mutierten RNA-Polymerasen und von wilden RNA-Polymerasen erkannt wird, aus denen er stammt, größer ist als die Transkriptionsaktivität dieser bestimmten Sequenz, durch die wilden RNA-Polymerase, in Gegenwart der gleichen nicht konsensuellen ITS wie jene, die in der DNA-Sequenz enthalten ist, die für die interessierende RNA kodiert, wobei die mutierten RNA-Polymerasen bis zu etwa 40 Mal aktiver sind als die wilden RNA-Polymerasen, im Rahmen der Durchführung von Verfahren zur Produktion von interessierender RNA aus der bestimmten Nukleotidsequenz, wobei das Verfahren umfasst:
- einen Schritt zur Modifikation der Peptidkette der wilden RNA-Polymerasen phagischen Ursprungs durch Substitution oder Deletion oder Addition mindestens eines Codons des Gens, das für die wilden RNA-Polymerasen kodiert, und Transformation geeigneter Zellen, wie etwa *E. coli,* mit Vektoren, die das so modifizierte Gen enthalten, sowie eine Nukleotidsequenz, die für einen Marker kodiert, wie etwa einen Antibiotikaresistenzmarker, oder einen chromogenen Marker, der stromabwärts des Promoters inseriert wird, der von den oben genannten RNA-Polymerasen erkannt wird, wobei dieser Promoter und die Sequenz, die für den Marker kodiert, durch eine ITS getrennt sind,
- einen Schritt der Detektion der oben genannten Zellen, die mutierte RNA-Polymerasen produzieren, bei denen die Produktionsausbeute des besonderen Markers größer ist als die Produktionsausbeute dieses gleichen Markers, der unter Verwendung von wilden RNA-Polymerasen unter den gleichen Bedingungen erhalten wird, und
- einen Schritt der Aufreinigung der oben genannten mutierten RNA-Polymerasen aus den im vorhergehenden Schritt detektierten Zellen.

18. Verfahren zur In-vitro-Herstellung von interessierender RNA, das das Zusammenbringen in einem geeigneten Medium mindestens einer mutierten RNA-Polymerase nach einem der Ansprüche 8 bis 13 mit bestimmten Nukleotidsequenzen umfasst, die eine DNA-Sequenz umfassen, die für die interessierende RNA kodiert und deren Transkription unter die Kontrolle eines Promoters gesetzt wird, der von den wilden RNA-Polymerasen und den oben genannten mutierten RNA-Polymerasen erkannt wird.

19. Verfahren zur In-vivo-Herstellung von interessierender RNA, das das Kultivieren von Zellen nach Anspruch 16 umfasst, wobei die Zellen mindestens eine mutierte RNA-Polymerase nach einem der Ansprüche 8 bis 13 produzieren, und deren Genom modifiziert wurde, um bestimmte Nukleotidsequenzen zu enthalten, die eine DNA-Sequenz umfassen, die für die interessierende RNA kodiert und deren Transkription unter die Kontrolle eines Promoters gesetzt wird, der von den wilden RNA-Polymerasen und den oben genannten mutierten RNA-Polymerasen erkannt wird.

20. Verfahren zur In-vivo-Herstellung von interessierenden Proteinen, das das Kultivieren von Zellen nach Anspruch 16 umfasst, wobei die Zellen mindestens eine mutierte RNA-Polymerase nach dem Verfahren, wie in Anspruch 17 definiert, oder eine mutierte RNA-Polymerase nach einem der Ansprüche 8 bis 13 produzieren, und deren Genom modifiziert wurde, um bestimmte Nukleotidsequenzen zu enthalten, die eine DNA-Sequenz umfassen, die für die interessierenden Proteine kodiert und deren Transkription unter die Kontrolle eines Promoters gesetzt wird, der von den wilden RNA-Polymerasen und den oben genannten mutierten RNA-Polymerasen erkannt wird.

21. Verfahren zur In-vitro- oder In-vivo-Herstellung von interessierender RNA oder interessierenden Proteinen nach einem der Ansprüche 18 bis 20, das ebenfalls einen Schritt der Zugabe von synthetischen Polyaminen umfasst.


### LISTE DE SEQUENCES

<110> CNRS
<120> POLYPEPTIDES DERIVES DES ARN POLYMERASES, ET LEURS UTILISATIONS
<130> IFB 01 CNR MUT7 .
<140>
   <141>
<160> 16
<170> PatentIn Ver. 2.1
<210> 1
   <211> 2652
   <212> ADN
   <213> Séquence artificielle
<220>
   <223> Description de la séquence artificielle: séquences mutées de l'ARN polymérase du bactériophage T7
<220>
   <221> CDS
   <222> (1)..(2652)
<400> 1
   atg aac acg att aac atc gct aag aac gac ttc tct gac atc gaa ctg    48
   Met Asn Thr Ile Asn Ile Ala Lys Asn Asp Phe Ser Asp Ile Glu Leu
    1               5                  10                  15
   gct gct atc ccg ttc aac act ctg gct gac cat tac ggt gag cgt tta    96
   Ala Ala Ile Pro Phe Asn Thr Leu Ala Asp His Tyr Gly Glu Arg Leu
               20                  25                  30
   gct cgc gaa cag ttg gcc ctt gag cat gag tct tac gag atg ggt gaa   144
   Ala Arg Glu Gln Leu Ala Leu Glu His Glu Ser Tyr Glu Met Gly Glu
           35                  40                  45
   gca cgc ttc cgc aag atg ttt gag cgt caa ctt aaa gct ggt gag gtt   192
   Ala Arg Phe Arg Lys Met Phe Glu Arg Gln Leu Lys Ala Gly Glu Val
       50                  55                  60
   gcg gat aac gct gcc gcc aag cct ctc atc act acc cta ctc cct aag   240
   Ala Asp Asn Ala Ala Ala Lys Pro Leu Ile Thr Thr Leu Leu Pro Lys
   65                  70                  75                  80
   atg att gca cgc atc aac gac tgg ttt gag gaa gtg aaa gct aag cgc   288
   Met Ile Ala Arg Ile Asn Asp Trp Phe Glu Glu Val Lys Ala Lys Arg
                   85                  90                  95
   ggc aag cgc ccg aca gcc ttc cag ttc ctg caa gaa atc aag ccg gaa   336
   Gly Lys Arg Pro Thr Ala Phe Gln Phe Leu Gln Glu Ile Lys Pro Glu
               100                 105                 110
   gcc gta gcg tac atc acc att aag acc act ctg gct tgc cta acc agt   384
   Ala Val Ala Tyr Ile Thr Ile Lys Thr Thr Leu Ala Cys Leu Thr Ser
           115                 120                 125
   gct gac aat aca acc gtt cag gct gta gca agc gca atc ggt cgg gcc      432
   Ala Asp Asn Thr Thr Val Gln Ala Val Ala Ser Ala Ile Gly Arg Ala
       130                 135                 140
   att gag gac gag gct cgc ttc ggt cgt atc cgt gac ctt gaa gct aag      480
   Ile Glu Asp Glu Ala Arg Phe Gly Arg Ile Arg Asp Leu Glu Ala Lys
   145                 150                 155                 160
   cac ttc aag aaa aac gtt gag gaa caa ctc aac aag cgc gta ggg cac      528
   His Phe Lys Lys Asn Val Glu Glu Gln Leu Asn Lys Arg Val Gly His
                   165                 170                 175
   gtc tac aag aaa gca ttt atg caa gtt gtc gag gct gac atg ctc tct      576
   Val Tyr Lys Lys Ala Phe Met Gln Val Val Glu Ala Asp Met Leu Ser
               180                 185                 190
   aag ggt cta ctc ggt ggc gag gcg tgg tct tcg tgg cat aag gaa gac      624
   Lys Gly Leu Leu Gly Gly Glu Ala Trp Ser Ser Trp His Lys Glu Asp
           195                 200                 205
   tct att cat gta gga gta cgc tgc atc gag atg ctc att gag tca acc      672
   Ser Ile His Val Gly Val Arg Cys Ile Glu Met Leu Ile Glu Ser Thr
       210                 215                 220
   gga atg gtt agc tta cac cgc caa aat gct ggc gta gta ggt caa gac      720
   Gly Met Val Ser Leu His Arg Gln Asn Ala Gly Val Val Gly Gln Asp
   225                 230                 235                 240
   tct gag act atc gaa ctc gca cct gaa tac gct gag gct atc gca acc      768
   Ser Glu Thr Ile Glu Leu Ala Pro Glu Tyr Ala Glu Ala Ile Ala Thr
                   245                 250                 255
   cgt gca ggt gcg ctg gct ggc atc tct ctg atg ttc caa cct tgc gta      816
   Arg Ala Gly Ala Leu Ala Gly Ile Ser Leu Met Phe Gln Pro Cys Val
               260                 265                 270
   gtt cct cct aag ccg tgg act ggc att act ggt ggt ggc tat tgg gct      864
   Val Pro Pro Lys Pro Trp Thr Gly Ile Thr Gly Gly Gly Tyr Trp Ala
           275                 280                 285
   aac ggt cgt cgt cct ctg gcg ctg gtg cgt act cac agt aag aaa gca      912
   Asn Gly Arg Arg Pro Leu Ala Leu Val Arg Thr His Ser Lys Lys Ala
       290                 295                 300
   ctg atg cgc tac gaa gac gtt tac atg cct gag gtg tac aaa gcg att      960
   Leu Met Arg Tyr Glu Asp Val Tyr Met Pro Glu Val Tyr Lys Ala Ile
   305                 310                 315                 320
   aac att gcg caa aac acc gca tgg aaa atc aac aag aaa gtc cta gcg     1008
   Asn Ile Ala Gln Asn Thr Ala Trp Lys Ile Asn Lys Lys Val Leu Ala
                   325                 330                 335
   gtc gcc aac gta atc acc aag tgg aag cat tgt ccg gtc gag gac atc     1056
   Val Ala Asn Val Ile Thr Lys Trp Lys His Cys Pro Val Glu Asp Ile
               340                 345                 350
   cct gcg att gag cgt gaa gaa ctc ccg atg aaa ccg gaa gac atc gac     1104
   Pro Ala Ile Glu Arg Glu Glu Leu Pro Met Lys Pro Glu Asp Ile Asp
           355                 360                 365
   atg aat cct gag gct ctc acc gcg tgg aaa cgt gct gcc gct gct gtg    1152
   Met Asn Pro Glu Ala Leu Thr Ala Trp Lys Arg Ala Ala Ala Ala Val
       370                 375                 380
   tac cgc aag gac aag gct cgc aag tct cgc cgt atc agc ctt gag ttc    1200
   Tyr Arg Lys Asp Lys Ala Arg Lys Ser Arg Arg Ile Ser Leu Glu Phe
   385                 390                 395                 400
   atg ctt gag caa gcc aat aag ttt gct aac cat aag gcc atc tgg ttc    1248
   Met Leu Glu Gln Ala Asn Lys Phe Ala Asn His Lys Ala Ile Trp Phe
                   405                 410                 415
   cct tac aac atg gac tgg cgc ggt cgt gtt tac gct gtg tca atg ttc    1296
   Pro Tyr Asn Met Asp Trp Arg Gly Arg Val Tyr Ala Val Ser Met Phe
               420                 425                 430
   aac ccg caa ggt aac gat atg acc aaa gga ctg ctt acg ctg gcg aaa    1344
   Asn Pro Gln Gly Asn Asp Met Thr Lys Gly Leu Leu Thr Leu Ala Lys
           435                 440                 445
   ggt aaa cca atc ggt aag gaa ggt tac tac tgg ctg aaa atc cac ggt    1392
   Gly Lys Pro Ile Gly Lys Glu Gly Tyr Tyr Trp Leu Lys Ile His Gly
       450                 455                 460
   gca aac tgt gcg ggt gtc gat aag gtt ccg ttc cct gag cgc atc aag    1440
   Ala Asn Cys Ala Gly Val Asp Lys Val Pro Phe Pro Glu Arg Ile Lys
   465                 470                 475                 480
   ttc att gag gaa aac cac gag aac atc atg gct tgc gct aag tct cca    1488
   Phe Ile Glu Glu Asn His Glu Asn Ile Met Ala Cys Ala Lys Ser Pro
                   485                 490                 495
   ctg gag aac act tgg tgg gct gag caa gat tct ccg ttc tgc ttc ctt    1536
   Leu Glu Asn Thr Trp Trp Ala Glu Gln Asp Ser Pro Phe Cys Phe Leu
               500                 505                 510
   gcg ttc tgc ttt gag tac gct ggg gta cag cac cac ggc ctg agc tat    1584
   Ala Phe Cys Phe Glu Tyr Ala Gly Val Gln His His Gly Leu Ser Tyr
           515                 520                 525
   aac tgc tcc ctt ccg ctg gcg ttt gac ggg tct tgc tct ggc atc cag    1632
   Asn Cys Ser Leu Pro Leu Ala Phe Asp Gly Ser Cys Ser Gly Ile Gln
       530                 535                 540
   cac ttc tcc gcg atg ctc cga gat gag gta ggt ggt cgc gcg gtt aac    1680
   His Phe Ser Ala Met Leu Arg Asp Glu Val Gly Gly Arg Ala Val Asn
   545                 550                 555                 560
   ttg ctt cct agt gaa acc gtt cag gac atc tac ggg att gtt gct aag    1728
   Leu Leu Pro Ser Glu Thr Val Gln Asp Ile Tyr Gly Ile Val Ala Lys
                   565                 570                 575
   aaa gtc aac gag att cta caa gca gac gca atc aat ggg acc gat aac    1776
   Lys Val Asn Glu Ile Leu Gln Ala Asp Ala Ile Asn Gly Thr Asp Asn
               580                 585                 590
   gaa gta gtt acc gtg acc gat gag aac act ggt gaa atc tct gag aaa    1824
   Glu Val Val Thr Val Thr Asp Glu Asn Thr Gly Glu Ile Ser Glu Lys
           595                 600                 605
   gtc aag ctg ggc act aag gca ctg gct ggt caa tgg ctg gct tac ggt    1872
   Val Lys Leu Gly Thr Lys Ala Leu Ala Gly Gln Trp Leu Ala Tyr Gly
       610                 615                 620
   gtt act cgc agt gtg act aag cgt tca gtc atg acg ctg gct tac ggg    1920
   Val Thr Arg Ser Val Thr Lys Arg Ser Val Met Thr Leu Ala Tyr Gly
   625                 630                 635                 640
   tcc aaa gag ttc ggc ttc cgt caa caa gtg ctg gaa gat acc att cag    1968
   Ser Lys Glu Phe Gly Phe Arg Gln Gln Val Leu Glu Asp Thr Ile Gln
                   645                 650                 655
   cca gct att gat tcc ggc aag ggt ctg atg ttc act cag ccg aat cag    2016
   Pro Ala Ile Asp Ser Gly Lys Gly Leu Met Phe Thr Gln Pro Asn Gln
               660                 665                 670
   gct gct gga tac atg gct aag ctg att tgg gaa tct gtg agc gtg acg    2064
   Ala Ala Gly Tyr Met Ala Lys Leu Ile Trp Glu Ser Val Ser Val Thr
           675                 680                 685
   gtg gta gct gcg gtt gaa gca atg aac tgg ctt aag tct gct gct aag    2112
   Val Val Ala Ala Val Glu Ala Met Asn Trp Leu Lys Ser Ala Ala Lys
       690                 695                 700
   ctg ctg gct gct gag gtc aaa gat aag aag act gga gag att ctt cgc    2160
   Leu Leu Ala Ala Glu Val Lys Asp Lys Lys Thr Gly Glu Ile Leu Arg
   705                 710                 715                 720
   aag cgt tgc gct gtg cat tgg gta act cct gat ggt ttc cct gtg tgg    2208
   Lys Arg Cys Ala Val His Trp Val Thr Pro Asp Gly Phe Pro Val Trp
                   725                 730                 735
   cag gaa tac aag aag cct att cag acg cgc ttg aac ctg atg ttc ctc    2256
   Gln Glu Tyr Lys Lys Pro Ile Gln Thr Arg Leu Asn Leu Met Phe Leu
               740                 745                 750
   ggt cag ttc cgc tta cag cct acc att aac acc aac aaa gat agc gag    2304
   Gly Gln Phe Arg Leu Gln Pro Thr Ile Asn Thr Asn Lys Asp Ser Glu
           755                 760                 765
   att gat gca cac aaa cag gag tct ggt atc gct cct aac ttt gta cac    2352
   Ile Asp Ala His Lys Gln Glu Ser Gly Ile Ala Pro Asn Phe Val His
       770                 775                 780
   agc caa gac ggt agc cac ctt cgt aag act gta gtg tgg gca cac gag    2400
   Ser Gln Asp Gly Ser His Leu Arg Lys Thr Val Val Trp Ala His Glu
   785                 790                 795                 800
   aag tac gga atc gaa tct ttt gca ctg att cac gac tcc ttc ggt acc    2448
   Lys Tyr Gly Ile Glu Ser Phe Ala Leu Ile His Asp Ser Phe Gly Thr
                   805                 810                 815
   att ccg gct gac gct gcg aac ctg ttc aaa gca gtg cgc gaa act atg    2496
   Ile Pro Ala Asp Ala Ala Asn Leu Phe Lys Ala Val Arg Glu Thr Met
               820                 825                 830
   gtt gac aca tat gag tct tgt gat gta ctg gct gat ttc tac gac cag    2544
   Val Asp Thr Tyr Glu Ser Cys Asp Val Leu Ala Asp Phe Tyr Asp Gln
           835                 840                 845
   ttc gct gac cag ttg cac gag tct caa ttg gac aaa atg cca gca ctt    2592
   Phe Ala Asp Gln Leu His Glu Ser Gln Leu Asp Lys Met Pro Ala Leu
       850                 855                 860
   ccg gct aaa ggt aac ttg aac ctc cgt gac atc tta gag tcg gac ttc    2640
   Pro Ala Lys Gly Asn Leu Asn Leu Arg Asp Ile Leu Glu Ser Asp Phe
   865                 870                 875                 880
   gcg ttc gcg taa                                                    2652
   Ala Phe Ala
<210> 2
   <211> 883
   <212> PRT
   <213> Séquence artificielle
   <223> Description de la séquence artificielle: séquences mutées de l'ARN polymérase du bactériophage T7
<400> 2
   Met Asn Thr Ile Asn Ile Ala Lys Asn Asp Phe Ser Asp Ile Glu Leu
     1               5                  10                  15
   Ala Ala Ile Pro Phe Asn Thr Leu Ala Asp His Tyr Gly Glu Arg Leu
                20                  25                  30
   Ala Arg Glu Gln Leu Ala Leu Glu His Glu Ser Tyr Glu Met Gly Glu
            35                  40                  45
   Ala Arg Phe Arg Lys Met Phe Glu Arg Gln Leu Lys Ala Gly Glu Val
        50                  55                  60
   Ala Asp Asn Ala Ala Ala Lys Pro Leu Ile Thr Thr Leu Leu Pro Lys
    65                  70                  75                      80
   Met Ile Ala Arg Ile Asn Asp Trp Phe Glu Glu Val Lys Ala Lys Arg
                    85                  90                  95
   Gly Lys Arg Pro Thr Ala Phe Gln Phe Leu Gln Glu Ile Lys Pro Glu
               100                 105                 110
   Ala Val Ala Tyr Ile Thr Ile Lys Thr Thr Leu Ala Cys Leu Thr Ser
           115                 120                 125
   Ala Asp Asn Thr Thr Val Gln Ala Val Ala Ser Ala Ile Gly Arg Ala
       130                 135                 140
   Ile Glu Asp Glu Ala Arg Phe Gly Arg Ile Arg Asp Leu Glu Ala Lys
   145                 150                 155                 160
   His Phe Lys Lys Asn Val Glu Glu Gln Leu Asn Lys Arg Val Gly His
                   165                 170                 175
   Val Tyr Lys Lys Ala Phe Met Gln Val Val Glu Ala Asp Met Leu Ser
               180                 185                 190
   Lys Gly Leu Leu Gly Gly Glu Ala Trp Ser Ser Trp His Lys Glu Asp
           195                 200                 205
   Ser Ile His Val Gly Val Arg Cys Ile Glu Met Leu Ile Glu Ser Thr
       210                 215                 220
   Gly Met Val Ser Leu His Arg Gln Asn Ala Gly Val Val Gly Gln Asp
   225                 230                 235                 240
   Ser Glu Thr Ile Glu Leu Ala Pro Glu Tyr Ala Glu Ala Ile Ala Thr
                   245                 250                 255
   Arg Ala Gly Ala Leu Ala Gly Ile Ser Leu Met Phe Gln Pro Cys Val
               260                 265                 270
   Val Pro Pro Lys Pro Trp Thr Gly Ile Thr Gly Gly Gly Tyr Trp Ala
           275                 280                 285
   Asn Gly Arg Arg Pro Leu Ala Leu Val Arg Thr His Ser Lys Lys Ala
       290                 295                 300
   Leu Met Arg Tyr Glu Asp Val Tyr Met Pro Glu Val Tyr Lys Ala Ile
   305                 310                 315                 320
   Asn Ile Ala Gln Asn Thr Ala Trp Lys Ile Asn Lys Lys Val Leu Ala
                   325                 330                 335
   Val Ala Asn Val Ile Thr Lys Trp Lys His Cys Pro Val Glu Asp Ile
               340                 345                 350
   Pro Ala Ile Glu Arg Glu Glu Leu Pro Met Lys Pro Glu Asp Ile Asp
           355                 360                 365
   Met Asn Pro Glu Ala Leu Thr Ala Trp Lys Arg Ala Ala Ala Ala Val
       370                 375                 380
   Tyr Arg Lys Asp Lys Ala Arg Lys Ser Arg Arg Ile Ser Leu Glu Phe
   385                 390                 395                 400
   Met Leu Glu Gln Ala Asn Lys Phe Ala Asn His Lys Ala Ile Trp Phe
                   405                 410                 415
   Pro Tyr Asn Met Asp Trp Arg Gly Arg Val Tyr Ala Val Ser Met Phe
               420                 425                 430
   Asn Pro Gln Gly Asn Asp Met Thr Lys Gly Leu Leu Thr Leu Ala Lys
           435                 440                 445
   Gly Lys Pro Ile Gly Lys Glu Gly Tyr Tyr Trp Leu Lys Ile His Gly
       450                 455                 460
   Ala Asn Cys Ala Gly Val Asp Lys Val Pro Phe Pro Glu Arg Ile Lys
   465                 470                 475                 480
   Phe Ile Glu Glu Asn His Glu Asn Ile Met Ala Cys Ala Lys Ser Pro
                   485                 490                 495
   Leu Glu Asn Thr Trp Trp Ala Glu Gln Asp Ser Pro Phe Cys Phe Leu
               500                 505                 510
   Ala Phe Cys Phe Glu Tyr Ala Gly Val Gln His His Gly Leu Ser Tyr
           515                 520                 525
   Asn Cys Ser Leu Pro Leu Ala Phe Asp Gly Ser Cys Ser Gly Ile Gln
       530                 535                 540
   His Phe Ser Ala Met Leu Arg Asp Glu Val Gly Gly Arg Ala Val Asn
   545                 550                 555                 560
   Leu Leu Pro Ser Glu Thr Val Gln Asp Ile Tyr Gly Ile Val Ala Lys
                   565                 570                 575
   Lys Val Asn Glu Ile Leu Gln Ala Asp Ala Ile Asn Gly Thr Asp Asn
               580                 585                 590
   Glu Val Val Thr Val Thr Asp Glu Asn Thr Gly Glu Ile Ser Glu Lys
           595                 600                 605
   Val Lys Leu Gly Thr Lys Ala Leu Ala Gly Gln Trp Leu Ala Tyr Gly
       610                 615                 620
   Val Thr Arg Ser Val Thr Lys Arg Ser Val Met Thr Leu Ala Tyr Gly
   625                 630                 635                 640
   Ser Lys Glu Phe Gly Phe Arg Gln Gln Val Leu Glu Asp Thr Ile Gln
                   645                 650                 655
   Pro Ala Ile Asp Ser Gly Lys Gly Leu Met Phe Thr Gln Pro Asn Gln
               660                 665                 670
   Ala Ala Gly Tyr Met Ala Lys Leu Ile Trp Glu Ser Val Ser Val Thr
           675                 680                 685
   Val Val Ala Ala Val Glu Ala Met Asn Trp Leu Lys Ser Ala Ala Lys
       690                 695                 700
   Leu Leu Ala Ala Glu Val Lys Asp Lys Lys Thr Gly Glu Ile Leu Arg
   705                 710                 715                 720
   Lys Arg Cys Ala Val His Trp Val Thr Pro Asp Gly Phe Pro Val Trp
                   725                 730                 735
   Gln Glu Tyr Lys Lys Pro Ile Gln Thr Arg Leu Asn Leu Met Phe Leu
               740                 745                 750
   Gly Gln Phe Arg Leu Gln Pro Thr Ile Asn Thr Asn Lys Asp Ser Glu
           755                 760                 765
   Ile Asp Ala His Lys Gln Glu Ser Gly Ile Ala Pro Asn Phe Val His
       770                 775                 780
   Ser Gln Asp Gly Ser His Leu Arg Lys Thr Val Val Trp Ala His Glu
   785                 790                 795                 800
   Lys Tyr Gly Ile Glu Ser Phe Ala Leu Ile His Asp Ser Phe Gly Thr
                   805                 810                 815
   Ile Pro Ala Asp Ala Ala Asn Leu Phe Lys Ala Val Arg Glu Thr Met
               820                 825                 830
   Val Asp Thr Tyr Glu Ser Cys Asp Val Leu Ala Asp Phe Tyr Asp Gln
           835                 840                 845
   Phe Ala Asp Gln Leu His Glu Ser Gln Leu Asp Lys Met Pro Ala Leu
       850                 855                 860
   Pro Ala Lys Gly Asn Leu Asn Leu Arg Asp Ile Leu Glu Ser Asp Phe
   865 870 875 880
   Ala Phe Ala
<210> 3
   <211> 2652
   <212> ADN
   <213> Séquence artificielle
<220>
   <223> Description de la séquence artificielle: séquences mutées de l'ARN polymérase du bactériophage T7
<220>
   <221> CDS
   <222> (1)..(2652)
<400> 3
   atg aac acg att aac atc gct aag aac gac ttc tct gac atc gaa ctg    48
   Met Asn Thr Ile Asn Ile Ala Lys Asn Asp Phe Ser Asp Ile Glu Leu
    1               5                  10                  15
   gct gct atc ccg ttc aac act ctg gct gac cat tac ggt gag cgt tta    96
   Ala Ala Ile Pro Phe Asn Thr Leu Ala Asp His Tyr Gly Glu Arg Leu
               20                  25                  30
   gct cgc gaa cag ttg gcc ctt gag cat gag tct tac gag atg ggt gaa   144
   Ala Arg Glu Gln Leu Ala Leu Glu His Glu Ser Tyr Glu Met Gly Glu
           35                  40                  45
   gca cgc ttc cgc aag atg ttt gag cgt caa ctt aaa gct ggt gag gtt   192
   Ala Arg Phe Arg Lys Met Phe Glu Arg Gln Leu Lys Ala Gly Glu Val
       50                  55                  60
   gcg gat aac gct gcc gcc aag cct ctc atc act acc cta ctc cct aag   240
   Ala Asp Asn Ala Ala Ala Lys Pro Leu Ile Thr Thr Leu Leu Pro Lys
   65                  70                  75                  80
   atg att gca cgc atc aac gac tgg ttt gag gaa gtg aaa gct aag cgc   288
   Met Ile Ala Arg Ile Asn Asp Trp Phe Glu Glu Val Lys Ala Lys Arg
                   85                  90                  95
   ggc aag cgc ccg aca gcc ttc cag ttc ctg caa gaa atc aag ccg gaa   336
   Gly Lys Arg Pro Thr Ala Phe Gln Phe Leu Gln Glu Ile Lys Pro Glu
               100                 105                 110
   gcc gta gcg tac gtc acc att aag acc act ctg gct tgc cta acc agt   384
   Ala Val Ala Tyr Val Thr Ile Lys Thr Thr Leu Ala Cys Leu Thr Ser
           115                 120                 125
   gct gac aat aca acc gct cag gct gta gca agc gca atc ggt cgg gcc   432
   Ala Asp Asn Thr Thr Ala Gln Ala Val Ala Ser Ala Ile Gly Arg Ala
       130                 135                 140
   att gag gac gag gct cgc ttc ggt cgt atc cgt gac ctt gaa gct aag   480
   Ile Glu Asp Glu Ala Arg Phe Gly Arg Ile Arg Asp Leu Glu Ala Lys
   145                 150                 155                 160
   cac ttc aag aaa aac gtt gag gaa caa ctc aac aag cgc gta ggg cac     528
   His Phe Lys Lys Asn Val Glu Glu Gln Leu Asn Lys Arg Val Gly His
                   165                 170                 175
   gtc tac aag aaa gca ttt atg caa gtt gtc gag gct gac atg ctc tct     576
   Val Tyr Lys Lys Ala Phe Met Gln Val Val Glu Ala Asp Met Leu Ser
               180                 185                 190
   aag ggt cta ctc ggt ggc gag gcg tgg tct tcg tgg cat aag gaa gac     624
   Lys Gly Leu Leu Gly Gly Glu Ala Trp Ser Ser Trp His Lys Glu Asp
           195                 200                 205
   tct att cat gta gga gta cgc tgc atc gag atg ctc att gag tca acc     672
   Ser Ile His Val Gly Val Arg Cys Ile Glu Met Leu Ile Glu Ser Thr
       210                 215                 220
   gga atg gtt agc tta cac cgc caa aat gct ggc gta gta ggt caa gac     720
   Gly Met Val Ser Leu His Arg Gln Asn Ala Gly Val Val Gly Gln Asp
   225                 230                 235                 240
   tct gag act atc gaa ctc gca cct gaa tac gct gag gct atc gca acc     768
   Ser Glu Thr Ile Glu Leu Ala Pro Glu Tyr Ala Glu Ala Ile Ala Thr
                   245                 250                 255
   cgt gca ggt gcg ctg gct ggc atc tct ccg atg ttc caa cct tgc gta     816
   Arg Ala Gly Ala Leu Ala Gly Ile Ser Pro Met Phe Gln Pro Cys Val
               260                 265                 270
   gtt cct cct aag ccg tgg act ggc att act ggt ggt ggc tat tgg gct     864
   Val Pro Pro Lys Pro Trp Thr Gly Ile Thr Gly Gly Gly Tyr Trp Ala
           275                 280                 285
   aac ggt cgt cgt cct ctg gcg ctg gtg cgt act cac agt aag aaa gca     912
   Asn Gly Arg Arg Pro Leu Ala Leu Val Arg Thr His Ser Lys Lys Ala
       290                 295                 300
   ctg atg cgc tac gaa gac gtt tac atg cct gag gtg tac aaa gcg att     960
   Leu Met Arg Tyr Glu Asp Val Tyr Met Pro Glu Val Tyr Lys Ala Ile
   305                 310                 315                 320
   aac att gcg caa aac acc gca tgg aaa atc aac aag aaa gtc cta gcg     1008
   Asn Ile Ala Gln Asn Thr Ala Trp Lys Ile Asn Lys Lys Val Leu Ala
                   325                 330                 335
   gtc gcc aac gta atc acc aag tgg aag cat tgt ccg gtc gag gac atc     1056
   Val Ala Asn Val Ile Thr Lys Trp Lys His Cys Pro Val Glu Asp Ile
               340                 345                 350
   cct gcg att gag cgt gaa gaa ctc ccg atg aaa ccg gaa gac atc gac     1104
   Pro Ala Ile Glu Arg Glu Glu Leu Pro Met Lys Pro Glu Asp Ile Asp
           355                 360                 365
   atg aat cct gag gct ctc acc gcg tgg aaa cgt gct gcc gct gct gtg     1152
   Met Asn Pro Glu Ala Leu Thr Ala Trp Lys Arg Ala Ala Ala Ala Val
       370                 375                 380
   tac cgc aag gac aag gct cgc aag tct cgc cgt atc agc ctt gag ttc     1200
   Tyr Arg Lys Asp Lys Ala Arg Lys Ser Arg Arg Ile Ser Leu Glu Phe
   385                 390                 395                 400
   atg ctt gag caa gcc aat aag ttt gct aac cat aag gcc atc tgg ttc    1248
   Met Leu Glu Gln Ala Asn Lys Phe Ala Asn His Lys Ala Ile Trp Phe
                   405                 410                 415
   cct tac aac atg gac tgg cgc ggt cgt gtt tac gct gtg tca atg ttc    1296
   Pro Tyr Asn Met Asp Trp Arg Gly Arg Val Tyr Ala Val Ser Met Phe
               420                 425                 430
   aac ccg caa ggt aac gat atg acc aaa gga ctg ctt acg ctg gcg aaa    1344
   Asn Pro Gln Gly Asn Asp Met Thr Lys Gly Leu Leu Thr Leu Ala Lys
           435                 440                 445
   ggt aaa cca atc ggt aag gaa ggt tac tac tgg ctg aaa atc cac ggt    1392
   Gly Lys Pro Ile Gly Lys Glu Gly Tyr Tyr Trp Leu Lys Ile His Gly
       450                 455                 460
   gca aac tgt gcg ggt gtc gat aag gtt ccg ttc cct gag cgc atc aag    1440
   Ala Asn Cys Ala Gly Val Asp Lys Val Pro Phe Pro Glu Arg Ile Lys
   465                 470                 475                 480
   ttc att gag gaa aac cac gag aac atc atg gct tgc gct aag tct cca    1488
   Phe Ile Glu Glu Asn His Glu Asn Ile Met Ala Cys Ala Lys Ser Pro
                   485                 490                 495
   ctg gag aac act tgg tgg gct gag caa gat tct ccg ttc tgc ttc ctt    1536
   Leu Glu Asn Thr Trp Trp Ala Glu Gln Asp Ser Pro Phe Cys Phe Leu
               500                 505                 510
   gcg ttc tgc ttt gag tac gct ggg gta cag cac cac ggc ctg agc tat    1584
   Ala Phe Cys Phe Glu Tyr Ala Gly Val Gln His His Gly Leu Ser Tyr
           515                 520                 525
   aac tgc tcc ctt ccg ctg gcg ttt gac ggg tct tgc tct ggc atc cag    1632
   Asn Cys Ser Leu Pro Leu Ala Phe Asp Gly Ser Cys Ser Gly Ile Gln
       530                 535                 540
   cac ttc tcc gcg atg ctc cga gat gag gta ggt ggt cgc gcg gtt aac    1680
   His Phe Ser Ala Met Leu Arg Asp Glu Val Gly Gly Arg Ala Val Asn
   545                 550                 555                 560
   ttg ctt cct agt gaa acc gtt cag gac atc tac ggg att gtt gct aag    1728
   Leu Leu Pro Ser Glu Thr Val Gln Asp Ile Tyr Gly Ile Val Ala Lys
                   565                 570                 575
   aaa gtc aac gag att cta caa gca gac gca atc aat ggg acc gat aac    1776
   Lys Val Asn Glu Ile Leu Gln Ala Asp Ala Ile Asn Gly Thr Asp Asn
               580                 585                 590
   gaa gta gtt acc gtg acc gat gag aac act ggt gaa atc tct gag aaa    1824
   Glu Val Val Thr Val Thr Asp Glu Asn Thr Gly Glu Ile Ser Glu Lys
           595                 600                 605
   gtc aag ctg ggc act aag gca ctg gct ggt caa tgg ctg gct tac ggt    1872
   Val Lys Leu Gly Thr Lys Ala Leu Ala Gly Gln Trp Leu Ala Tyr Gly
       610                 615                 620
   gtt act cgc agt gtg act aag cgt tca gtc atg acg ctg gct tac ggg    1920
   Val Thr Arg Ser Val Thr Lys Arg Ser Val Met Thr Leu Ala Tyr Gly
   625                 630                 635                 640
   tcc aaa gag ttc ggc ttc cgt caa caa gtg ctg gaa gat acc att cag    1968
   Ser Lys Glu Phe Gly Phe Arg Gln Gln Val Leu Glu Asp Thr Ile Gln
                   645                 650                 655
   cca gct att gat tcc ggc aag ggt ctg atg ttc act cag ccg aat cag    2016
   Pro Ala Ile Asp Ser Gly Lys Gly Leu Met Phe Thr Gln Pro Asn Gln
               660                 665                 670
   gct gct gga tac atg gct aag ctg att tgg gaa tct gtg agc gtg acg    2064
   Ala Ala Gly Tyr Met Ala Lys Leu Ile Trp Glu Ser Val Ser Val Thr
           675                 680                 685
   gtg gta gct gcg gtt gaa gca atg aac tgg ctt aag tct gct gct aag    2112
   Val Val Ala Ala Val Glu Ala Met Asn Trp Leu Lys Ser Ala Ala Lys
       690                 695                 700
   ctg ctg gct gct gag gtc aaa gat aag aag act gga gag att ctt cgc    2160
   Leu Leu Ala Ala Glu Val Lys Asp Lys Lys Thr Gly Glu Ile Leu Arg
   705                 710                 715                 720
   aag cgt tgc gct gtg cat tgg gta act cct gat ggt ttc cct gtg tgg    2208
   Lys Arg Cys Ala Val His Trp Val Thr Pro Asp Gly Phe Pro Val Trp
                   725                 730                 735
   cag gaa tac aag aag cct att cag acg cgc ttg aac ctg atg ttc ctc    2256
   Gln Glu Tyr Lys Lys Pro Ile Gln Thr Arg Leu Asn Leu Met Phe Leu
               740                 745                 750
   ggt cag ttc cgc tta cag cct acc att aac acc aac aaa gat agc gag    2304
   Gly Gln Phe Arg Leu Gln Pro Thr Ile Asn Thr Asn Lys Asp Ser Glu
           755                 760                 765
   att gat gca cac aaa cag gag tct ggt atc gct cct aac ttt gta cac    2352
   Ile Asp Ala His Lys Gln Glu Ser Gly Ile Ala Pro Asn Phe Val His
       770                 775                 780
   agc caa gac ggt agc cac ctt cgt aag act gta gtg tgg gca cac gag    2400
   Ser Gln Asp Gly Ser His Leu Arg Lys Thr Val Val Trp Ala His Glu
   785                 790                 795                 800
   aag tac gga atc gaa tct ttt gca ctg att cac gac tcc ttc ggt acc    2448
   Lys Tyr Gly Ile Glu Ser Phe Ala Leu Ile His Asp Ser Phe Gly Thr
                   805                 810                 815
   att ccg gct gac gct gcg aac ctg ttc aaa gca gtg cgc gaa act atg    2496
   Ile Pro Ala Asp Ala Ala Asn Leu Phe Lys Ala Val Arg Glu Thr Met
               820                 825                 830
   gtt gac aca tat gag tct tgt gat gta ctg gct gat ttc tac gac cag    2544
   Val Asp Thr Tyr Glu Ser Cys Asp Val Leu Ala Asp Phe Tyr Asp Gln
           835                 840                 845
   ttc gct gac cag ttg cac gag tct caa ttg gac aaa atg cca gca ctt    2592
   Phe Ala Asp Gln Leu His Glu Ser Gln Leu Asp Lys Met Pro Ala Leu
       850                 855                 860
   ccg gct aaa ggt aac ttg aac ctc cgt gac atc tta gag tcg gac ttc    2640
   Pro Ala Lys Gly Asn Leu Asn Leu Arg Asp Ile Leu Glu Ser Asp Phe
   865                 870                 875                 880
   gcg ttc gcg taa 2652
   Ala Phe Ala
<210> 4
   <211> 883
   <212> PRT
   <213> Séquence artificielle
   <223> Description de la séquence artificielle: séquences mutées de l'ARN polymérase du bactériophage T7
<400> 4
   Met Asn Thr Ile Asn Ile Ala Lys Asn Asp Phe Ser Asp Ile Glu Leu
    1               5                  10                  15
   Ala Ala Ile Pro Phe Asn Thr Leu Ala Asp His Tyr Gly Glu Arg Leu
               20                  25                  30
   Ala Arg Glu Gln Leu Ala Leu Glu His Glu Ser Tyr Glu Met Gly Glu
           35                  40                  45
   Ala Arg Phe Arg Lys Met Phe Glu Arg Gln Leu Lys Ala Gly Glu Val
       50                  55                  60
   Ala Asp Asn Ala Ala Ala Lys Pro Leu Ile Thr Thr Leu Leu Pro Lys
   65                  70                  75                  80
   Met Ile Ala Arg Ile Asn Asp Trp Phe Glu Glu Val Lys Ala Lys Arg
                   85                  90                  95
   Gly Lys Arg Pro Thr Ala Phe Gln Phe Leu Gln Glu Ile Lys Pro Glu
               100                 105                 110
   Ala Val Ala Tyr Val Thr Ile Lys Thr Thr Leu Ala Cys Leu Thr Ser
           115                 120                 125
   Ala Asp Asn Thr Thr Ala Gln Ala Val Ala Ser Ala Ile Gly Arg Ala
       130                 135                 140
   Ile Glu Asp Glu Ala Arg Phe Gly Arg Ile Arg Asp Leu Glu Ala Lys
   145                 150                 155                 160
   His Phe Lys Lys Asn Val Glu Glu Gln Leu Asn Lys Arg Val Gly His
                   165                 170                 175
   Val Tyr Lys Lys Ala Phe Met Gln Val Val Glu Ala Asp Met Leu Ser
               180                 185                 190
   Lys Gly Leu Leu Gly Gly Glu Ala Trp Ser Ser Trp His Lys Glu Asp
           195                 200                 205
   Ser Ile His Val Gly Val Arg Cys Ile Glu Met Leu Ile Glu Ser Thr
       210                 215                 220
   Gly Met Val Ser Leu His Arg Gln Asn Ala Gly Val Val Gly Gln Asp
   225                 230                 235                 240
   Ser Glu Thr Ile Glu Leu Ala Pro Glu Tyr Ala Glu Ala Ile Ala Thr
                   245                 250                 255
   Arg Ala Gly Ala Leu Ala Gly Ile Ser Pro Met Phe Gln Pro Cys Val
               260                 265                 270
   Val Pro Pro Lys Pro Trp Thr Gly Ile Thr Gly Gly Gly Tyr Trp Ala
           275                 280                 285
   Asn Gly Arg Arg Pro Leu Ala Leu Val Arg Thr His Ser Lys Lys Ala
       290                 295                 300
   Leu Met Arg Tyr Glu Asp Val Tyr Met Pro Glu Val Tyr Lys Ala Ile
   305                 310                 315                 320
   Asn Ile Ala Gln Asn Thr Ala Trp Lys Ile Asn Lys Lys Val Leu Ala
                   325                 330                 335
   Val Ala Asn Val Ile Thr Lys Trp Lys His Cys Pro Val Glu Asp Ile
               340                 345                 350
   Pro Ala Ile Glu Arg Glu Glu Leu Pro Met Lys Pro Glu Asp Ile Asp
           355                 360                 365
   Met Asn Pro Glu Ala Leu Thr Ala Trp Lys Arg Ala Ala Ala Ala Val
       370                 375                 380
   Tyr Arg Lys Asp Lys Ala Arg Lys Ser Arg Arg Ile Ser Leu Glu Phe
   385                 390                 395                 400
   Met Leu Glu Gln Ala Asn Lys Phe Ala Asn His Lys Ala Ile Trp Phe
                   405                 410                 415
   Pro Tyr Asn Met Asp Trp Arg Gly Arg Val Tyr Ala Val Ser Met Phe
               420                 425                 430
   Asn Pro Gln Gly Asn Asp Met Thr Lys Gly Leu Leu Thr Leu Ala Lys
           435                 440                 445
   Gly Lys Pro Ile Gly Lys Glu Gly Tyr Tyr Trp Leu Lys Ile His Gly
       450                 455                 460
   Ala Asn Cys Ala Gly Val Asp Lys Val Pro Phe Pro Glu Arg Ile Lys
   465                 470                 475                 480
   Phe Ile Glu Glu Asn His Glu Asn Ile Met Ala Cys Ala Lys Ser Pro
                   485                 490                 495
   Leu Glu Asn Thr Trp Trp Ala Glu Gln Asp Ser Pro Phe Cys Phe Leu
               500                 505                 510
   Ala Phe Cys Phe Glu Tyr Ala Gly Val Gln His His Gly Leu Ser Tyr
           515                 520                 525
   Asn Cys Ser Leu Pro Leu Ala Phe Asp Gly Ser Cys Ser Gly Ile Gln
       530                 535                 540
   His Phe Ser Ala Met Leu Arg Asp Glu Val Gly Gly Arg Ala Val Asn
   545                 550                 555                 560
   Leu Leu Pro Ser Glu Thr Val Gln Asp Ile Tyr Gly Ile Val Ala Lys
                   565                 570                 575
   Lys Val Asn Glu Ile Leu Gln Ala Asp Ala Ile Asn Gly Thr Asp Asn
               580                 585                 590
   Glu Val Val Thr Val Thr Asp Glu Asn Thr Gly Glu Ile Ser Glu Lys
           595                 600                 605
   Val Lys Leu Gly Thr Lys Ala Leu Ala Gly Gln Trp Leu Ala Tyr Gly
       610                 615                 620
   Val Thr Arg Ser Val Thr Lys Arg Ser Val Met Thr Leu Ala Tyr Gly
   625                 630                 635                 640
   Ser Lys Glu Phe Gly Phe Arg Gln Gln Val Leu Glu Asp Thr Ile Gln
                   645                 650                 655
   Pro Ala Ile Asp Ser Gly Lys Gly Leu Met Phe Thr Gln Pro Asn Gln
               660                 665                 670
   Ala Ala Gly Tyr Met Ala Lys Leu Ile Trp Glu Ser Val Ser Val Thr
           675                 680                 685
   Val Val Ala Ala Val Glu Ala Met Asn Trp Leu Lys Ser Ala Ala Lys
       690                 695                 700
   Leu Leu Ala Ala Glu Val Lys Asp Lys Lys Thr Gly Glu Ile Leu Arg
   705                 710                 715                 720
   Lys Arg Cys Ala Val His Trp Val Thr Pro Asp Gly Phe Pro Val Trp
                   725                 730                 735
   Gln Glu Tyr Lys Lys Pro Ile Gln Thr Arg Leu Asn Leu Met Phe Leu
               740                 745                 750
   Gly Gln Phe Arg Leu Gln Pro Thr Ile Asn Thr Asn Lys Asp Ser Glu
           755                 760                 765
   Ile Asp Ala His Lys Gln Glu Ser Gly Ile Ala Pro Asn Phe Val His
       770                 775                 780
   Ser Gln Asp Gly Ser His Leu Arg Lys Thr Val Val Trp Ala His Glu
   785                 790                 795                 800
   Lys Tyr Gly Ile Glu Ser Phe Ala Leu Ile His Asp Ser Phe Gly Thr
                   805                 810                 815
   Ile Pro Ala Asp Ala Ala Asn Leu Phe Lys Ala Val Arg Glu Thr Met
               820                 825                 830
   Val Asp Thr Tyr Glu Ser Cys Asp Val Leu Ala Asp Phe Tyr Asp Gln
           835                 840                 845
   Phe Ala Asp Gln Leu His Glu Ser Gln Leu Asp Lys Met Pro Ala Leu
       850                 855                 860
   Pro Ala Lys Gly Asn Leu Asn Leu Arg Asp Ile Leu Glu Ser Asp Phe
   865                 870                 875                 880
   Ala Phe Ala
<210> 5
   <211> 2652
   <212> ADN
   <213> Séquence artificielle
<220>
   <223> Description de la séquence artificielle: séquences mutées de l'ARN polymérase du bactériophage T7
<220>
   <221> CDS
   <222> (1)..(2652)
<400> 5
   atg aac acg att aac atc gct aag aac gac ttc tct gac atc gaa ctg      48
   Met Asn Thr Ile Asn Ile Ala Lys Asn Asp Phe Ser Asp Ile Glu Leu
     1               5                  10                  15
   gct gct atc ccg ttc aac act ctg gct gac cat tac ggt gag cgt tta      96
   Ala Ala Ile Pro Phe Asn Thr Leu Ala Asp His Tyr Gly Glu Arg Leu
                20                  25                  30
   gct cgc gaa cag ttg gcc ctt gag cat gag tct tac gag atg ggt gaa     144
   Ala Arg Glu Gln Leu Ala Leu Glu His Glu Ser Tyr Glu Met Gly Glu
            35                  40                  45
   gca cgc ttc cgc aag atg ttt gag cgt caa ctt aaa gct ggt gag gtt     192
   Ala Arg Phe Arg Lys Met Phe Glu Arg Gln Leu Lys Ala Gly Glu Val
        50                  55                  60
   gcg gat aac gct gcc gcc aag cct ctc atc act acc cta ctc cct aag     240
   Ala Asp Asn Ala Ala Ala Lys Pro Leu Ile Thr Thr Leu Leu Pro Lys
    65                  70                  75                  80
   atg att gca cgc atc aac gac tgg ttt gag gaa gtg aaa gct aag cgc     288
   Met Ile Ala Arg Ile Asn Asp Trp Phe Glu Glu Val Lys Ala Lys Arg
                    85                  90                  95
   ggc aag cgc ccg aca gcc ttc cag ttc ctg caa gaa atc aag ccg gaa     336
   Gly Lys Arg Pro Thr Ala Phe Gln Phe Leu Gln Glu Ile Lys Pro Glu
               100                 105                 110
   gcc gta gcg tac atc acc gtt aag acc act ctg gct tgc cta acc agt     384
   Ala Val Ala Tyr Ile Thr Val Lys Thr Thr Leu Ala Cys Leu Thr Ser
           115                 120                 125
   gct gac aat aca acc gtt cag gct gta gca agc gca atc ggt cgg gcc     432
   Ala Asp Asn Thr Thr Val Gln Ala Val Ala Ser Ala Ile Gly Arg Ala
       130                 135                 140
   att gag aac gag gct cgc ttc ggt cgt atc cgt gac ctt gaa gct aag     480
   Ile Glu Asn Glu Ala Arg Phe Gly Arg Ile Arg Asp Leu Glu Ala Lys
   145                 150                 155                 160
   cac ttc aag aaa aac gtt gag gaa caa ctc aac aag cgc gta ggg cac     528
   His Phe Lys Lys Asn Val Glu Glu Gln Leu Asn Lys Arg Val Gly His
                   165                 170                 175
   gtc tac aag aaa gca ttt atg caa gtt gtc gag gct gac atg ctc tct     576
   Val Tyr Lys Lys Ala Phe Met Gln Val Val Glu Ala Asp Met Leu Ser
               180                 185                 190
   aag ggt cta ctc ggt ggc gag gcg tgg tct tcg tgg cat aag gaa gac     624
   Lys Gly Leu Leu Gly Gly Glu Ala Trp Ser Ser Trp His Lys Glu Asp
           195                 200                 205
   tct att cat gta gga gta cgc tgc atc gag atg ctc att gag tca acc     672
   Ser Ile His Val Gly Val Arg Cys Ile Glu Met Leu Ile Glu Ser Thr
       210                 215                 220
   gga atg gtt agc tta cac cgc caa aat gct ggc gta gta ggt caa gac     720
   Gly Met Val Ser Leu His Arg Gln Asn Ala Gly Val Val Gly Gln Asp
   225                 230                 235                 240
   tct gag act atc gaa ctc gca cct gaa tac gct gag gct atc gca acc     768
   Ser Glu Thr Ile Glu Leu Ala Pro Glu Tyr Ala Glu Ala Ile Ala Thr
                   245                 250                 255
   cgt gca ggt gcg ctg gct ggc atc tct ccg atg ttc caa cct tgc gta     816
   Arg Ala Gly Ala Leu Ala Gly Ile Ser Pro Met Phe Gln Pro Cys Val
               260                 265                 270
   gtt cct cct aag ccg tgg act ggc att act ggt ggt ggc tat tgg gct     864
   Val Pro Pro Lys Pro Trp Thr Gly Ile Thr Gly Gly Gly Tyr Trp Ala
           275                 280                 285
   aac ggt cgt cgt cct ctg gcg ctg gtg cgt act cac agt aag aaa gca     912
   Asn Gly Arg Arg Pro Leu Ala Leu Val Arg Thr His Ser Lys Lys Ala
       290                 295                 300
   ctg atg cgc tac gaa gac gtt tac atg cct gag gtg tac aaa gcg att     960
   Leu Met Arg Tyr Glu Asp Val Tyr Met Pro Glu Val Tyr Lys Ala Ile
   305                 310                 315                 320
   aac att gcg caa aac acc gca tgg aaa atc aac aag aaa gtc cta gcg    1008
   Asn Ile Ala Gln Asn Thr Ala Trp Lys Ile Asn Lys Lys Val Leu Ala
                   325                 330                 335
   gtc gcc aac gta atc acc aag tgg aag cat tgt ccg gtc gag gac atc    1056
   Val Ala Asn Val Ile Thr Lys Trp Lys His Cys Pro Val Glu Asp Ile
               340                 345                 350
   cct gcg att gag cgt gaa gaa ctc ccg atg aaa ccg gaa gac atc gac    1104
   Pro Ala Ile Glu Arg Glu Glu Leu Pro Met Lys Pro Glu Asp Ile Asp
           355                 360                 365
   atg aat cct gag gct ctc acc gcg tgg aaa cgt gct gcc gct gct gtg    1152
   Met Asn Pro Glu Ala Leu Thr Ala Trp Lys Arg Ala Ala Ala Ala Val
       370                 375                 380
   tac cgc aag gac aag gct cgc aag tct cgc cgt atc agc ctt gag ttc    1200
   Tyr Arg Lys Asp Lys Ala Arg Lys Ser Arg Arg Ile Ser Leu Glu Phe
   385                 390                 395                 400
   atg ctt gag caa gcc aat aag ttt gct aac cat aag gcc atc tgg ttc    1248
   Met Leu Glu Gln Ala Asn Lys Phe Ala Asn His Lys Ala Ile Trp Phe
                   405                 410                 415
   cct tac aac atg gac tgg cgc ggt cgt gtt tac gct gtg tca atg ttc    1296
   Pro Tyr Asn Met Asp Trp Arg Gly Arg Val Tyr Ala Val Ser Met Phe
               420                 425                 430
   aac ccg caa ggt aac gat atg acc aaa gga ctg ctt acg ctg gcg aaa    1344
   Asn Pro Gln Gly Asn Asp Met Thr Lys Gly Leu Leu Thr Leu Ala Lys
           435                 440                 445
   ggt aaa cca atc ggt aag gaa ggt tac tac tgg ctg aaa atc cac ggt    1392
   Gly Lys Pro Ile Gly Lys Glu Gly Tyr Tyr Trp Leu Lys Ile His Gly
       450                 455                 460
   gca aac tgt gcg ggt gtc gat aag gtt ccg ttc cct gag cgc atc aag    1440
   Ala Asn Cys Ala Gly Val Asp Lys Val Pro Phe Pro Glu Arg Ile Lys
   465                 470                 475                 480
   ttc att gag gaa aac cac gag aac atc atg gct tgc gct aag tct cca    1488
   Phe Ile Glu Glu Asn His Glu Asn Ile Met Ala Cys Ala Lys Ser Pro
                   485                 490                 495
   ctg gag aac act tgg tgg gct gag caa gat tct ccg ttc tgc ttc ctt    1536
   Leu Glu Asn Thr Trp Trp Ala Glu Gln Asp Ser Pro Phe Cys Phe Leu
               500                 505                 510
   gcg ttc tgc ttt gag tac gct ggg gta cag cac cac ggc ctg agc tat    1584
   Ala Phe Cys Phe Glu Tyr Ala Gly Val Gln His His Gly Leu Ser Tyr
           515                 520                 525
   aac tgc tcc ctt ccg ctg gcg ttt gac ggg tct tgc tct ggc atc cag    1632
   Asn Cys Ser Leu Pro Leu Ala Phe Asp Gly Ser Cys Ser Gly Ile Gln
       530                 535                 540
   cac ttc tcc gcg atg ctc cga gat gag gta ggt ggt cgc gcg gtt aac    1680
   His Phe Ser Ala Met Leu Arg Asp Glu Val Gly Gly Arg Ala Val Asn
   545                 550                 555                 560
   ttg ctt cct agt gaa acc gtt cag gac atc tac ggg att gtt gct aag    1728
   Leu Leu Pro Ser Glu Thr Val Gln Asp Ile Tyr Gly Ile Val Ala Lys
                   565                 570                 575
   aaa gtc aac gag att cta caa gca gac gca atc aat ggg acc gat aac    1776
   Lys Val Asn Glu Ile Leu Gln Ala Asp Ala Ile Asn Gly Thr Asp Asn
               580                 585                 590
   gaa gta gtt acc gtg acc gat gag aac act ggt gaa atc tct gag aaa    1824
   Glu Val Val Thr Val Thr Asp Glu Asn Thr Gly Glu Ile Ser Glu Lys
           595                 600                 605
   gtc aag ctg ggc act aag gca ctg gct ggt caa tgg ctg gct tac ggt    1872
   Val Lys Leu Gly Thr Lys Ala Leu Ala Gly Gln Trp Leu Ala Tyr Gly
       610                 615                 620
   gtt act cgc agt gtg act aag cgt tca gtc atg acg ctg gct tac ggg    1920
   Val Thr Arg Ser Val Thr Lys Arg Ser Val Met Thr Leu Ala Tyr Gly
   625                 630                 635                 640
   tcc aaa gag ttc ggc ttc cgt caa caa gtg ctg gaa gat acc att cag    1968
   Ser Lys Glu Phe Gly Phe Arg Gln Gln Val Leu Glu Asp Thr Ile Gln
                   645                 650                 655
   cca gct att gat tcc ggc aag ggt ctg atg ttc act cag ccg aat cag    2016
   Pro Ala Ile Asp Ser Gly Lys Gly Leu Met Phe Thr Gln Pro Asn Gln
               660                 665                 670
   gct gct gga tac atg gct aag ctg att tgg gaa tct gtg agc gtg acg    2064
   Ala Ala Gly Tyr Met Ala Lys Leu Ile Trp Glu Ser Val Ser Val Thr
           675                 680                 685
   gtg gta gct gcg gtt gaa gca atg aac tgg ctt aag tct gct gct aag    2112
   Val Val Ala Ala Val Glu Ala Met Asn Trp Leu Lys Ser Ala Ala Lys
       690                 695                 700
   ctg ctg gct gct gag gtc aaa gat aag aag act gga gag att ctt cgc    2160
   Leu Leu Ala Ala Glu Val Lys Asp Lys Lys Thr Gly Glu Ile Leu Arg
   705                 710                 715                 720
   aag cgt tgc gct gtg cat tgg gta act cct gat ggt ttc cct gtg tgg    2208
   Lys Arg Cys Ala Val His Trp Val Thr Pro Asp Gly Phe Pro Val Trp
                   725                 730                 735
   cag gaa tac aag aag cct att cag acg cgc ttg aac ctg atg ttc ctc    2256
   Gln Glu Tyr Lys Lys Pro Ile Gln Thr Arg Leu Asn Leu Met Phe Leu
               740                 745                 750
   ggt cag ttc cgc tta cag cct acc att aac acc aac aaa gat agc gag    2304
   Gly Gln Phe Arg Leu Gln Pro Thr Ile Asn Thr Asn Lys Asp Ser Glu
           755                 760                 765
   att gat gca cac aaa cag gag tct ggt atc gct cct aac ttt gta cac    2352
   Ile Asp Ala His Lys Gln Glu Ser Gly Ile Ala Pro Asn Phe Val His
       770                 775                 780
   agc caa gac ggt agc cac ctt cgt aag act gta gtg tgg gca cac gag    2400
   Ser Gln Asp Gly Ser His Leu Arg Lys Thr Val Val Trp Ala His Glu
   785                 790                 795                 800
   aag tac gga atc gaa tct ttt gca ctg att cac gac tcc ttc ggt acc    2448
   Lys Tyr Gly Ile Glu Ser Phe Ala Leu Ile His Asp Ser Phe Gly Thr
                   805                 810                 815
   att ccg gct gac gct gcg aac ctg ttc aaa gca gtg cgc gaa act atg    2496
   Ile Pro Ala Asp Ala Ala Asn Leu Phe Lys Ala Val Arg Glu Thr Met
               820                 825                 830
   gtt gac aca tat gag tct tgt gat gta ctg gct gat ttc tac gac cag    2544
   Val Asp Thr Tyr Glu Ser Cys Asp Val Leu Ala Asp Phe Tyr Asp Gln
           835                 840                 845
   ttc gct gac cag ttg cac gag tct caa ttg gac aaa atg cca gca ctt    2592
   Phe Ala Asp Gln Leu His Glu Ser Gln Leu Asp Lys Met Pro Ala Leu
       850                 855                 860
   ccg gct aaa ggt aac ttg aac ctc cgt gac atc tta gag tcg gac ttc    2640
   Pro Ala Lys Gly Asn Leu Asn Leu Arg Asp Ile Leu Glu Ser Asp Phe
   865                 870                 875                 880
   gcg ttc gcg taa                                                    2652
   Ala Phe Ala
<210> 6
   <211> 883
   <212> PRT
   <213> Séquence artificielle '
   <223> Description de la séquence artificielle: séquences mutées de l'ARN polymérase du bactériophage T7
<400> 6
   Met Asn Thr Ile Asn Ile Ala Lys Asn Asp Phe Ser Asp Ile Glu Leu
   1               5                   10                  15
   Ala Ala Ile Pro Phe Asn Thr Leu Ala Asp His Tyr Gly Glu Arg Leu
               20                  25                  30
   Ala Arg Glu Gln Leu Ala Leu Glu His Glu Ser Tyr Glu Met Gly Glu
           35                  40                  45
   Ala Arg Phe Arg Lys Met Phe Glu Arg Gln Leu Lys Ala Gly Glu Val
       50                  55                  60
   Ala Asp Asn Ala Ala Ala Lys Pro Leu Ile Thr Thr Leu Leu Pro Lys
   65                  70                  75                  80
   Met Ile Ala Arg Ile Asn Asp Trp Phe Glu Glu Val Lys Ala Lys Arg
                   85                  90                  95
   Gly Lys Arg Pro Thr Ala Phe Gln Phe Leu Gln Glu Ile Lys Pro Glu
               100                 105                 110
   Ala Val Ala Tyr Ile Thr Val Lys Thr Thr Leu Ala Cys Leu Thr Ser
           115                 120                 125
   Ala Asp Asn Thr Thr Val Gln Ala Val Ala Ser Ala Ile Gly Arg Ala
       130                 135                 140
   Ile Glu Asn Glu Ala Arg Phe Gly Arg Ile Arg Asp Leu Glu Ala Lys
   145                 150                 155                 160
   His Phe Lys Lys Asn Val Glu Glu Gln Leu Asn Lys Arg Val Gly His
                   165                 170                 175
   Val Tyr Lys Lys Ala Phe Met Gln Val Val Glu Ala Asp Met Leu Ser
               180                 185                 190
   Lys Gly Leu Leu Gly Gly Glu Ala Trp Ser Ser Trp His Lys Glu Asp
           195                 200                 205
   Ser Ile His Val Gly Val Arg Cys Ile Glu Met Leu Ile Glu Ser Thr
       210                 215                 220
   Gly Met Val Ser Leu His Arg Gln Asn Ala Gly Val Val Gly Gln Asp
   225                 230                 235                 240
   Ser Glu Thr Ile Glu Leu Ala Pro Glu Tyr Ala Glu Ala Ile Ala Thr
                   245                 250                 255
   Arg Ala Gly Ala Leu Ala Gly Ile Ser Pro Met Phe Gln Pro Cys Val
               260                 265                 270
   Val Pro Pro Lys Pro Trp Thr Gly Ile Thr Gly Gly Gly Tyr Trp Ala
           275                 280                 285
   Asn Gly Arg Arg Pro Leu Ala Leu Val Arg Thr His Ser Lys Lys Ala
       290                 295                 300
   Leu Met Arg Tyr Glu Asp Val Tyr Met Pro Glu Val Tyr Lys Ala Ile
   305                 310                 315                 320
   Asn Ile Ala Gln Asn Thr Ala Trp Lys Ile Asn Lys Lys Val Leu Ala
                   325                 330                 335
   Val Ala Asn Val Ile Thr Lys Trp Lys His Cys Pro Val Glu Asp Ile
               340                 345                 350
   Pro Ala Ile Glu Arg Glu Glu Leu Pro Met Lys Pro Glu Asp Ile Asp
           355                 360                 365
   Met Asn Pro Glu Ala Leu Thr Ala Trp Lys Arg Ala Ala Ala Ala Val
       370                 375                 380
   Tyr Arg Lys Asp Lys Ala Arg Lys Ser Arg Arg Ile Ser Leu Glu Phe
   385                 390                 395                 400
   Met Leu Glu Gln Ala Asn Lys Phe Ala Asn His Lys Ala Ile Trp Phe
                   405                 410                 415
   Pro Tyr Asn Met Asp Trp Arg Gly Arg Val Tyr Ala Val Ser Met Phe
               420                 425                 430
   Asn Pro Gln Gly Asn Asp Met Thr Lys Gly Leu Leu Thr Leu Ala Lys
           435                 440                 445
   Gly Lys Pro Ile Gly Lys Glu Gly Tyr Tyr Trp Leu Lys Ile His Gly
       450                 455                 460
   Ala Asn Cys Ala Gly Val Asp Lys Val Pro Phe Pro Glu Arg Ile Lys
   465                 470                 475                 480
   Phe Ile Glu Glu Asn His Glu Asn Ile Met Ala Cys Ala Lys Ser Pro
                   485                 490                 495
   Leu Glu Asn Thr Trp Trp Ala Glu Gln Asp Ser Pro Phe Cys Phe Leu
               500                 505                 510
   Ala Phe Cys Phe Glu Tyr Ala Gly Val Gln His His Gly Leu Ser Tyr
           515                 520                 525
   Asn Cys Ser Leu Pro Leu Ala Phe Asp Gly Ser Cys Ser Gly Ile Gln
       530                 535                 540
   His Phe Ser Ala Met Leu Arg Asp Glu Val Gly Gly Arg Ala Val Asn
   545                 550                 555                 560
   Leu Leu Pro Ser Glu Thr Val Gln Asp Ile Tyr Gly Ile Val Ala Lys
                   565                 570                 575
   Lys Val Asn Glu Ile Leu Gln Ala Asp Ala Ile Asn Gly Thr Asp Asn
               580                 585                 590
   Glu Val Val Thr Val Thr Asp Glu Asn Thr Gly Glu Ile Ser Glu Lys
           595                 600                 605
   Val Lys Leu Gly Thr Lys Ala Leu Ala Gly Gln Trp Leu Ala Tyr Gly
       610                 615                 620
   Val Thr Arg Ser Val Thr Lys Arg Ser Val Met Thr Leu Ala Tyr Gly
   625 630 635 640
   Ser Lys Glu Phe Gly Phe Arg Gln Gln Val Leu Glu Asp Thr Ile Gln
   645 650 655
   Pro Ala Ile Asp Ser Gly Lys Gly Leu Met Phe Thr Gln Pro Asn Gln
   660 665 670
   Ala Ala Gly Tyr Met Ala Lys Leu Ile Trp Glu Ser Val Ser Val Thr
   675 680 685
   Val Val Ala Ala Val Glu Ala Met Asn Trp Leu Lys Ser Ala Ala Lys
   690 695 700
   Leu Leu Ala Ala Glu Val Lys Asp Lys Lys Thr Gly Glu Ile Leu Arg
   705 710 715 720
   Lys Arg Cys Ala Val His Trp Val Thr Pro Asp Gly Phe Pro Val Trp
   725 730 735
   Gln Glu Tyr Lys Lys Pro Ile Gln Thr Arg Leu Asn Leu Met Phe Leu
   740 745 750
   Gly Gln Phe Arg Leu Gln Pro Thr Ile Asn Thr Asn Lys Asp Ser Glu
   755 760 765
   Ile Asp Ala His Lys Gln Glu Ser Gly Ile Ala Pro Asn Phe Val His
   770 775 780
   Ser Gln Asp Gly Ser His Leu Arg Lys Thr Val Val Trp Ala His Glu
   785 790 795 800
   Lys Tyr Gly Ile Glu Ser Phe Ala Leu Ile His Asp Ser Phe Gly Thr
   805 810 815
   Ile Pro Ala Asp Ala Ala Asn Leu Phe Lys Ala Val Arg Glu Thr Met
   820 825 830
   Val Asp Thr Tyr Glu Ser Cys Asp Val Leu Ala Asp Phe Tyr Asp Gln
   835 840 845
   Phe Ala Asp Gln Leu His Glu Ser Gln Leu Asp Lys Met Pro Ala Leu
   850 855 860
   Pro Ala Lys Gly Asn Leu Asn Leu Arg Asp Ile Leu Glu Ser Asp Phe
   865 870 875 880
   Ala Phe Ala
<210> 7
   <211> 2652
   <212> ADN
   <213> Séquence artificielle
<220>
   <223> Description de la séquence artificielle: séquences mutées de l'ARN polymérase du bactériophage T7
<220>
   <221> CDS
   <222> (1)..(2652)
<400> 7
   atg aac acg att aac atc gct aag aac gac ttc tct gac atc gaa ctg     48
   Met Asn Thr Ile Asn Ile Ala Lys Asn Asp Phe Ser Asp Ile Glu Leu
    1               5                  10                  15
   gct gct atc ccg ttc aac act ctg gct gac cat tac ggt gag cgt tta     96
   Ala Ala Ile Pro Phe Asn Thr Leu Ala Asp His Tyr Gly Glu Arg Leu
               20                  25                  30
   gct cgc gaa cag ttg gcc ctt gag cat gag tct tac gag atg ggt gaa     144
   Ala Arg Glu Gln Leu Ala Leu Glu His Glu Ser Tyr Glu Met Gly Glu
           35                  40                  45
   gca cgc ttc cgc aag atg ttt gag cgt caa ctt aaa gct ggt gag gtt     192
   Ala Arg Phe Arg Lys Met Phe Glu Arg Gln Leu Lys Ala Gly Glu Val
       50                  55                  60
   gcg gat aac gct gcc gcc aag cct ctc atc act acc cta ctc cct aag     240
   Ala Asp Asn Ala Ala Ala Lys Pro Leu Ile Thr Thr Leu Leu Pro Lys
   65                  70                  75                  80
   atg att gca cgc atc aac gac tgg ttt gag gaa gtg aaa gct aag cgc     288
   Met Ile Ala Arg Ile Asn Asp Trp Phe Glu Glu Val Lys Ala Lys Arg
                   85                  90                  95
   ggc aag cgc ccg aca gcc ttc cag ttc ctg caa gaa atc aag ccg gaa     336
   Gly Lys Arg Pro Thr Ala Phe Gln Phe Leu Gln Glu Ile Lys Pro Glu
               100                 105                 110
   gcc gta gcg tac atc acc att aag acc act ctg gct tgc cta acc agt     384
   Ala Val Ala Tyr Ile Thr Ile Lys Thr Thr Leu Ala Cys Leu Thr Ser
           115                 120                 125
   gct gac aat aca acc gtt cag gct gta gca agc gca atc ggt cgg gcc     432
   Ala Asp Asn Thr Thr Val Gln Ala Val Ala Ser Ala Ile Gly Arg Ala
       130                 135                 140
   att gag gac gag gct cgc ttc ggt cgt atc cgt gac ctt gaa gct aag     480
   Ile Glu Asp Glu Ala Arg Phe Gly Arg Ile Arg Asp Leu Glu Ala Lys
   145                 150                 155                 160
   cac ttc aag aaa aac gtt gag gaa caa ctc aac aag cgc gta ggg cac     528
   His Phe Lys Lys Asn Val Glu Glu Gln Leu Asn Lys Arg Val Gly His
                   165                 170                 175
   gtc tac aag aaa gca ttt atg caa gtt gtc gag gct gac atg ctc tct     576
   Val Tyr Lys Lys Ala Phe Met Gln Val Val Glu Ala Asp Met Leu Ser
               180                 185                 190
   aag ggt cta ctc ggt ggc gag gcg tgg tct tcg tgg cat aag gaa gac     624
   Lys Gly Leu Leu Gly Gly Glu Ala Trp Ser Ser Trp His Lys Glu Asp
           195                 200                 205
   tct att cat gta gga gta cgc tgc atc gag atg ctc att gag tca acc     672
   Ser Ile His Val Gly Val Arg Cys Ile Glu Met Leu Ile Glu Ser Thr
       210                 215                 220
   gga atg gtt agc tta cgc cgc caa aat gct ggc gta gta ggt caa gac    720
   Gly Met Val Ser Leu Arg Arg Gln Asn Ala Gly Val Val Gly Gln Asp
   225                 230                 235                 240
   tct gag act atc gaa ctc gca cct gaa tac gct gag gct atc gca acc    768
   Ser Glu Thr Ile Glu Leu Ala Pro Glu Tyr Ala Glu Ala Ile Ala Thr
                   245                 250                 255
   cgt gca ggt gcg ctg gct ggc atc tct ccg atg ttc caa cct tgc gta    816
   Arg Ala Gly Ala Leu Ala Gly Ile Ser Pro Met Phe Gln Pro Cys Val
               260                 265                 270
   gtt cct cct aag ccg tgg act ggc att act ggt ggt ggc tat tgg gct    864
   Val Pro Pro Lys Pro Trp Thr Gly Ile Thr Gly Gly Gly Tyr Trp Ala
           275                 280                 285
   aac ggt tgt cgt cct ctg gcg ctg gtg cgt act cac agt aag aaa gca    912
   Asn Gly Cys Arg Pro Leu Ala Leu Val Arg Thr His Ser Lys Lys Ala
       290                 295                 300
   ctg atg cgc tac gaa gac gtt tac atg cct gag gtg tac aaa gcg att    960
   Leu Met Arg Tyr Glu Asp Val Tyr Met Pro Glu Val Tyr Lys Ala Ile
   305                 310                 315                 320
   aac att gcg caa aac acc gca tgg aaa atc aac aag aaa gtc cta gcg    1008
   Asn Ile Ala Gln Asn Thr Ala Trp Lys Ile Asn Lys Lys Val Leu Ala
                   325                 330                 335
   gtc gcc aac gta atc acc aag tgg aag cat tgt ccg gtc gag gac atc    1056
   Val Ala Asn Val Ile Thr Lys Trp Lys His Cys Pro Val Glu Asp Ile
               340                 345                 350
   cct gcg att gag cgt gaa gaa ctc ccg atg aaa ccg gaa gac atc gac    1104
   Pro Ala Ile Glu Arg Glu Glu Leu Pro Met Lys Pro Glu Asp Ile Asp
           355                 360                 365
   atg aat cct gag gct ctc acc gcg tgg aaa cgt gct gcc gct gct gtg    1152
   Met Asn Pro Glu Ala Leu Thr Ala Trp Lys Arg Ala Ala Ala Ala Val
       370                 375                 380
   tac cgc aag gac aag gct cgc aag tct cgc cgt atc agc ctt gag ttc    1200
   Tyr Arg Lys Asp Lys Ala Arg Lys Ser Arg Arg Ile Ser Leu Glu Phe
   385                 390                 395                 400
   atg ctt gag caa gcc aat aag ttt gct aac cat aag gcc atc tgg ttc    1248
   Met Leu Glu Gln Ala Asn Lys Phe Ala Asn His Lys Ala Ile Trp Phe
                   405                 410                 415
   cct tac aac atg gac tgg cgc ggt cgt gtt tac gct gtg tca atg ttc    1296
   Pro Tyr Asn Met Asp Trp Arg Gly Arg Val Tyr Ala Val Ser Met Phe
               420                 425                 430
   aac ccg caa ggt aac gat atg acc aaa gga ctg ctt acg ctg gcg aaa    1344
   Asn Pro Gln Gly Asn Asp Met Thr Lys Gly Leu Leu Thr Leu Ala Lys
           435                 440                 445
   ggt aaa cca atc ggt aag gaa ggt tac tac tgg ctg aaa atc cac ggt    1392
   Gly Lys Pro Ile Gly Lys Glu Gly Tyr Tyr Trp Leu Lys Ile His Gly
       450                 455                 460
   gca aac tgt gcg ggt gtc gat aag gtt ccg ttc cct gag cgc atc aag    1440
   Ala Asn Cys Ala Gly Val Asp Lys Val Pro Phe Pro Glu Arg Ile Lys
   465                 470                 475                 480
   ttc att gag gaa aac cac gag aac atc atg gct tgc gct aag tct cca    1488
   Phe Ile Glu Glu Asn His Glu Asn Ile Met Ala Cys Ala Lys Ser Pro
                   485                 490                 495
   ctg gag aac act tgg tgg gct gag caa gat tct ccg ttc tgc ttc ctt    1536
   Leu Glu Asn Thr Trp Trp Ala Glu Gln Asp Ser Pro Phe Cys Phe Leu
               500                 505                 510
   gcg ttc tgc ttt gag tac gct ggg gta cag cac cac ggc ctg agc tat    1584
   Ala Phe Cys Phe Glu Tyr Ala Gly Val Gln His His Gly Leu Ser Tyr
           515                 520                 525
   aac tgc tcc ctt ccg ctg gcg ttt gac ggg tct tgc tct ggc atc cag    1632
   Asn Cys Ser Leu Pro Leu Ala Phe Asp Gly Ser Cys Ser Gly Ile Gln
       530                 535                 540
   cac ttc tcc gcg atg ctc cga gat gag gta ggt ggt cgc gcg gtt aac    1680
   His Phe Ser Ala Met Leu Arg Asp Glu Val Gly Gly Arg Ala Val Asn
   545                 550                 555                 560
   ttg ctt cct agt gaa acc gtt cag gac atc tac ggg att gtt gct aag    1728
   Leu Leu Pro Ser Glu Thr Val Gln Asp Ile Tyr Gly Ile Val Ala Lys
                   565                 570                 575
   aaa gtc aac gag att cta caa gca gac gca atc aat ggg acc gat aac    1776
   Lys Val Asn Glu Ile Leu Gln Ala Asp Ala Ile Asn Gly Thr Asp Asn
               580                 585                 590
   gaa gta gtt acc gtg acc gat gag aac act ggt gaa atc tct gag aaa    1824
   Glu Val Val Thr Val Thr Asp Glu Asn Thr Gly Glu Ile Ser Glu Lys
           595                 600                 605
   gtc aag ctg ggc act aag gca ctg gct ggt caa tgg ctg gct tac ggt    1872
   Val Lys Leu Gly Thr Lys Ala Leu Ala Gly Gln Trp Leu Ala Tyr Gly
       610                 615                 620
   gtt act cgc agt gtg act aag cgt tca gtc atg acg ctg gct tac ggg    1920
   Val Thr Arg Ser Val Thr Lys Arg Ser Val Met Thr Leu Ala Tyr Gly
   625                 630                 635                 640
   tcc aaa gag ttc ggc ttc cgt caa caa gtg ctg gaa gat acc att cag    1968
   Ser Lys Glu Phe Gly Phe Arg Gln Gln Val Leu Glu Asp Thr Ile Gln
                   645                 650                 655
   cca gct att gat tcc ggc aag ggt ctg atg ttc act cag ccg aat cag    2016
   Pro Ala Ile Asp Ser Gly Lys Gly Leu Met Phe Thr Gln Pro Asn Gln
               660                 665                 670
   gct gct gga tac atg gct aag ctg att tgg gaa tct gtg agc gtg acg    2064
   Ala Ala Gly Tyr Met Ala Lys Leu Ile Trp Glu Ser Val Ser Val Thr
           675                 680                 685
   gtg gta gct gcg gtt gaa gca atg aac tgg ctt aag tct gct gct aag    2112
   Val Val Ala Ala Val Glu Ala Met Asn Trp Leu Lys Ser Ala Ala Lys
       690                 695                 700
   ctg ctg gct gct gag gtc aaa gat aag aag act gga gag att ctt cgc   2160
   Leu Leu Ala Ala Glu Val Lys Asp Lys Lys Thr Gly Glu Ile Leu Arg
   705                 710                 715                 720
   aag cgt tgc gct gtg cat tgg gta act cct gat ggt ttc cct gtg tgg   2208
   Lys Arg Cys Ala Val His Trp Val Thr Pro Asp Gly Phe Pro Val Trp
                   725                 730                 735
   cag gaa tac aag aag cct att cag acg cgc ttg aac ctg atg ttc ctc   2256
   Gln Glu Tyr Lys Lys Pro Ile Gln Thr Arg Leu Asn Leu Met Phe Leu
               740                 745                 750
   ggt cag ttc cgc tta cag cct acc att aac acc aac aaa gat agc gag   2304
   Gly Gln Phe Arg Leu Gln Pro Thr Ile Asn Thr Asn Lys Asp Ser Glu
           755                 760                 765
   att gat gca cac aaa cag gag tct ggt atc gct cct aac ttt gta cac   2352
   Ile Asp Ala His Lys Gln Glu Ser Gly Ile Ala Pro Asn Phe Val His
       770                 775                 780
   agc caa gac ggt agc cac ctt cgt aag act gta gtg tgg gca cac gag   2400
   Ser Gln Asp Gly Ser His Leu Arg Lys Thr Val Val Trp Ala His Glu
   785                 790                 795                 800
   aag tac gga atc gaa tct ttt gca ctg att cac gac tcc ttc ggt acc   2448
   Lys Tyr Gly Ile Glu Ser Phe Ala Leu Ile His Asp Ser Phe Gly Thr
                   805                 810                 815
   att ccg gct gac gct gcg aac ctg ttc aaa gca gtg cgc gaa act atg   2496
   Ile Pro Ala Asp Ala Ala Asn Leu Phe Lys Ala Val Arg Glu Thr Met
               820                 825                 830
   gtt gac aca tat gag tct tgt gat gta ctg gct gat ttc tac gac cag    2544
   Val Asp Thr Tyr Glu Ser Cys Asp Val Leu Ala Asp Phe Tyr Asp Gln
           835                 840                 845
   ttc gct gac cag ttg cac gag tct caa ttg gac aaa atg cca gca ctt   2592
   Phe Ala Asp Gln Leu His Glu Ser Gln Leu Asp Lys Met Pro Ala Leu
       850                 855                 860
   ccg gct aaa ggt aac ttg aac ctc cgt gac atc tta gag tcg gac ttc   2640
   Pro Ala Lys Gly Asn Leu Asn Leu Arg Asp Ile Leu Glu Ser Asp Phe
   865                 870                 875                 880
   gcg ttc gcg taa                                                   2652
   Ala Phe Ala
<210> 8
   <211> 883
   <212> PRT
   <213> Séquence artificielle
   <223> Description de la séquence artificielle: séquences mutées de l'ARN polymérase du bactériophage T7
<400> 8
   Met Asn Thr Ile Asn Ile Ala Lys Asn Asp Phe Ser Asp Ile Glu Leu
    1               5                  10                  15
   Ala Ala Ile Pro Phe Asn Thr Leu Ala Asp His Tyr Gly Glu Arg Leu
               20                  25                  30
   Ala Arg Glu Gln Leu Ala Leu Glu His Glu Ser Tyr Glu Met Gly Glu
   35 40 45
   Ala Arg Phe Arg Lys Met Phe Glu Arg Gln Leu Lys Ala Gly Glu Val
   50 55 60
   Ala Asp Asn Ala Ala Ala Lys Pro Leu Ile Thr Thr Leu Leu Pro Lys
   65 70 75 80
   Met Ile Ala Arg Ile Asn Asp Trp Phe Glu Glu Val Lys Ala Lys Arg
   85 90 95
   Gly Lys Arg Pro Thr Ala Phe Gln Phe Leu Gln Glu Ile Lys Pro Glu
   100 105 110
   Ala Val Ala Tyr Ile Thr Ile Lys Thr Thr Leu Ala Cys Leu Thr Ser
   115 120 125
   Ala Asp Asn Thr Thr Val Gln Ala Val Ala Ser Ala Ile Gly Arg Ala
   130 135 140
   Ile Glu Asp Glu Ala Arg Phe Gly Arg Ile Arg Asp Leu Glu Ala Lys
   145 150 155 160
   His Phe Lys Lys Asn Val Glu Glu Gln Leu Asn Lys Arg Val Gly His
   165 170 175
   Val Tyr Lys Lys Ala Phe Met Gln Val Val Glu Ala Asp Met Leu Ser
   180 185 190
   Lys Gly Leu Leu Gly Gly Glu Ala Trp Ser Ser Trp His Lys Glu Asp
   195 200 205
   Ser Ile His Val Gly Val Arg Cys Ile Glu Met Leu Ile Glu Ser Thr
   210 215 220
   Gly Met Val Ser Leu Arg Arg Gln Asn Ala Gly Val Val Gly Gln Asp
   225 230 235 240
   Ser Glu Thr Ile Glu Leu Ala Pro Glu Tyr Ala Glu Ala Ile Ala Thr
   245 250 255
   Arg Ala Gly Ala Leu Ala Gly Ile Ser Pro Met Phe Gln Pro Cys Val
   260 265 270
   Val Pro Pro Lys Pro Trp Thr Gly Ile Thr Gly Gly Gly Tyr Trp Ala
   275 280 285
   Asn Gly Cys Arg Pro Leu Ala Leu Val Arg Thr His Ser Lys Lys Ala
   290 295 300
   Leu Met Arg Tyr Glu Asp Val Tyr Met Pro Glu Val Tyr Lys Ala Ile
   305 310 315 320
   Asn Ile Ala Gln Asn Thr Ala Trp Lys Ile Asn Lys Lys Val Leu Ala
   325 330 335
   Val Ala Asn Val Ile Thr Lys Trp Lys His Cys Pro Val Glu Asp Ile
   340 345 350
   Pro Ala Ile Glu Arg Glu Glu Leu Pro Met Lys Pro Glu Asp Ile Asp
           355                 360                 365
   Met Asn Pro Glu Ala Leu Thr Ala Trp Lys Arg Ala Ala Ala Ala Val
       370                 375                 380
   Tyr Arg Lys Asp Lys Ala Arg Lys Ser Arg Arg Ile Ser Leu Glu Phe
   385                 390                 395                 400
   Met Leu Glu Gln Ala Asn Lys Phe Ala Asn His Lys Ala Ile Trp Phe
                   405                 410                 415
   Pro Tyr Asn Met Asp Trp Arg Gly Arg Val Tyr Ala Val Ser Met Phe
               420                 425                 430
   Asn Pro Gln Gly Asn Asp Met Thr Lys Gly Leu Leu Thr Leu Ala Lys
           435                 440                 445
   Gly Lys Pro Ile Gly Lys Glu Gly Tyr Tyr Trp Leu Lys Ile His Gly
       450                 455                 460
   Ala Asn Cys Ala Gly Val Asp Lys Val Pro Phe Pro Glu Arg Ile Lys
   465                 470                 475                 480
   Phe Ile Glu Glu Asn His Glu Asn Ile Met Ala Cys Ala Lys Ser Pro
                   485                 490                 495
   Leu Glu Asn Thr Trp Trp Ala Glu Gln Asp Ser Pro Phe Cys Phe Leu
               500                 505                 510
   Ala Phe Cys Phe Glu Tyr Ala Gly Val Gln His His Gly Leu Ser Tyr
           515                 520                 525
   Asn Cys Ser Leu Pro Leu Ala Phe Asp Gly Ser Cys Ser Gly Ile Gln
       530                 535                 540
   His Phe Ser Ala Met Leu Arg Asp Glu Val Gly Gly Arg Ala Val Asn
   545                 550                 555                 560
   Leu Leu Pro Ser Glu Thr Val Gln Asp Ile Tyr Gly Ile Val Ala Lys
                   565                 570                 575
   Lys Val Asn Glu Ile Leu Gln Ala Asp Ala Ile Asn Gly Thr Asp Asn
               580                 585                 590
   Glu Val Val Thr Val Thr Asp Glu Asn Thr Gly Glu Ile Ser Glu Lys
           595                 600                 605
   Val Lys Leu Gly Thr Lys Ala Leu Ala Gly Gln Trp Leu Ala Tyr Gly
       610                 615                 620
   Val Thr Arg Ser Val Thr Lys Arg Ser Val Met Thr Leu Ala Tyr Gly
   625                 630                 635                 640
   Ser Lys Glu Phe Gly Phe Arg Gln Gln Val Leu Glu Asp Thr Ile Gln
                   645                 650                 655
   Pro Ala Ile Asp Ser Gly Lys Gly Leu Met Phe Thr Gln Pro Asn Gln
               660                 665                 670
   Ala Ala Gly Tyr Met Ala Lys Leu Ile Trp Glu Ser Val Ser Val Thr
           675                 680                 685
   Val Val Ala Ala Val Glu Ala Met Asn Trp Leu Lys Ser Ala Ala Lys
       690                 695                 700
   Leu Leu Ala Ala Glu Val Lys Asp Lys Lys Thr Gly Glu Ile Leu Arg
   705                 710                 715                 720
   Lys Arg Cys Ala Val His Trp Val Thr Pro Asp Gly Phe Pro Val Trp
                   725                 730                 735
   Gln Glu Tyr Lys Lys Pro Ile Gln Thr Arg Leu Asn Leu Met Phe Leu
               740                 745                 750
   Gly Gln Phe Arg Leu Gln Pro Thr Ile Asn Thr Asn Lys Asp Ser Glu
           755                 760                 765
   Ile Asp Ala His Lys Gln Glu Ser Gly Ile Ala Pro Asn Phe Val His
       770                 775                 780
   Ser Gln Asp Gly Ser His Leu Arg Lys Thr Val Val Trp Ala His Glu
   785                 790                 795                 800
   Lys Tyr Gly Ile Glu Ser Phe Ala Leu Ile His Asp Ser Phe Gly Thr
                   805                 810                 815
   Ile Pro Ala Asp Ala Ala Asn Leu Phe Lys Ala Val Arg Glu Thr Met
               820                 825                 830
   Val Asp Thr Tyr Glu Ser Cys Asp Val Leu Ala Asp Phe Tyr Asp Gln
           835                 840                 845
   Phe Ala Asp Gln Leu His Glu Ser Gln Leu Asp Lys Met Pro Ala Leu
       850                 855                 860
   Pro Ala Lys Gly Asn Leu Asn Leu Arg Asp Ile Leu Glu Ser Asp Phe
   865                 870                 875                 880
   Ala Phe Ala
<210> 9
   <211> 2652
   <212> ADN
   <213> Séquence artificielle
<220>
   <223> Description de la séquence artificielle: séquences mutées de l'ARN polymérase du bactériophage T7
<220>
   <221> CDS
   <222> (1)..(2652)
<400> 9
   atg aac acg att aac atc gct aag aac gac ttc tct gac atc gaa ctg    48
   Met Asn Thr Ile Asn Ile Ala Lys Asn Asp Phe Ser Asp Ile Glu Leu
    1               5                  10                  15
   gct gct atc ccg ttc aac act ctg gct gac cat tac ggt gag cgt tta      96
   Ala Ala Ile Pro Phe Asn Thr Leu Ala Asp His Tyr Gly Glu Arg Leu
               20                  25                  30
   gct cgc gaa cag ttg gcc ctt gag cat gag tct tac gag atg ggt gaa     144
   Ala Arg Glu Gln Leu Ala Leu Glu His Glu Ser Tyr Glu Met Gly Glu
           35                  40                  45
   gca cgc ttc cgc aag atg ttt gag cgt caa ctt aaa gct ggt gag gtt     192
   Ala Arg Phe Arg Lys Met Phe Glu Arg Gln Leu Lys Ala Gly Glu Val
       50                  55                  60
   gcg gat aac gct gcc gcc aag cct ctc atc act acc cta ctc cct aag     240
   Ala Asp Asn Ala Ala Ala Lys Pro Leu Ile Thr Thr Leu Leu Pro Lys
   65                  70                  75                  80
   atg att gca cgc atc aac gac tgg ttt gag gaa gtg aaa gct aag cgc     288
   Met Ile Ala Arg Ile Asn Asp Trp Phe Glu Glu Val Lys Ala Lys Arg
                   85                  90                  95
   ggc aag cgc ccg aca gcc ttc cag ttc ctg caa gaa atc aag ccg gaa     336
   Gly Lys Arg Pro Thr Ala Phe Gln Phe Leu Gln Glu Ile Lys Pro Glu
               100                 105                 110
   gcc gta gcg tac atc acc att aag acc act ctg gct tgc cta acc agt     384
   Ala Val Ala Tyr Ile Thr Ile Lys Thr Thr Leu Ala Cys Leu Thr Ser
           115                 120                 125
   gct gac aat aca acc gtt cag gct gta gca agc gca atc ggt cgg gcc     432
   Ala Asp Asn Thr Thr Val Gln Ala Val Ala Ser Ala Ile Gly Arg Ala
       130                 135                 140
   att gag gac gag gct cgc ttc ggt cgt atc cgt gac ctt gaa gct aag     480
   Ile Glu Asp Glu Ala Arg Phe Gly Arg Ile Arg Asp Leu Glu Ala Lys
   145                 150                 155                 160
   cac ttc aag aaa aac gtt gag gaa caa ctc aac aag cgc gta ggg cac     528
   His Phe Lys Lys Asn Val Glu Glu Gln Leu Asn Lys Arg Val Gly His
                   165                 170                 175
   gtc tac aag aaa gca ttt atg caa gtt gtc gag gct gac atg ctc tct     576
   Val Tyr Lys Lys Ala Phe Met Gln Val Val Glu Ala Asp Met Leu Ser
               180                 185                 190
   aag ggt cta ctc ggt ggc gag gcg tgg tct tcg tgg cat aag gaa gac     624
   Lys Gly Leu Leu Gly Gly Glu Ala Trp Ser Ser Trp His Lys Glu Asp
           195                 200                 205
   tct att cat gta gga gta cgc tgc atc gag atg ctc att gag tca acc     672
   Ser Ile His Val Gly Val Arg Cys Ile Glu Met Leu Ile Glu Ser Thr
       210                 215                 220
   gga atg gtt agc tta cac cgc caa aat gct ggc gta gta ggt caa gac     720
   Gly Met Val Ser Leu His Arg Gln Asn Ala Gly Val Val Gly Gln Asp
   225                 230                 235                 240
   tct gag act atc gaa ctc gca cct gaa tac gct gag gct atc gca acc     768
   Ser Glu Thr Ile Glu Leu Ala Pro Glu Tyr Ala Glu Ala Ile Ala Thr
                   245                 250                 255
   cgt gca ggt gcg ctg gct ggc atc tct ctg atg ttc caa cct tgc gta    816
   Arg Ala Gly Ala Leu Ala Gly Ile Ser Leu Met Phe Gln Pro Cys Val
               260                 265                 270
   gtt cct cct aag ccg tgg act ggc att act ggt ggt ggc tat tgg gct    864
   Val Pro Pro Lys Pro Trp Thr Gly Ile Thr Gly Gly Gly Tyr Trp Ala
           275                 280                 285
   aac ggt cgt cgt cct ctg gcg ctg gtg cgt act cac agt aag aaa gca    912
   Asn Gly Arg Arg Pro Leu Ala Leu Val Arg Thr His Ser Lys Lys Ala
       290                 295                 300
   ctg atg cgc tac gaa gac gtt tac atg cct gag gtg tac aaa gcg att    960
   Leu Met Arg Tyr Glu Asp Val Tyr Met Pro Glu Val Tyr Lys Ala Ile
   305                 310                 315                 320
   aac att gcg caa aac acc gca tgg aaa atc aac aag aaa gtc cta gcg    1008
   Asn Ile Ala Gln Asn Thr Ala Trp Lys Ile Asn Lys Lys Val Leu Ala
                   325                 330                 335
   gtc gcc aac gta atc acc aag tgg aag cat tgt ccg gtc gag gac atc    1056
   Val Ala Asn Val Ile Thr Lys Trp Lys His Cys Pro Val Glu Asp Ile
               340                 345                 350
   cct gcg att gag cgt gaa gaa ctc ccg atg aaa ccg gaa gac atc gac    1104
   Pro Ala Ile Glu Arg Glu Glu Leu Pro Met Lys Pro Glu Asp Ile Asp
           355                 360                 365
   atg aat cct gag gct ctc acc gcg tgg aaa cgt gct gcc gct gct gtg    1152
   Met Asn Pro Glu Ala Leu Thr Ala Trp Lys Arg Ala Ala Ala Ala Val
       370                 375                 380
   tac cgc aag gac aag gct cgc aag tct cgc cgt atc agc ctt gag ttc    1200
   Tyr Arg Lys Asp Lys Ala Arg Lys Ser Arg Arg Ile Ser Leu Glu Phe
   385                 390                 395                 400
   atg ctt gag caa gcc aat aag ttt gct aac cat aag gcc atc tgg ttc    1248
   Met Leu Glu Gln Ala Asn Lys Phe Ala Asn His Lys Ala Ile Trp Phe
                   405                 410                 415
   cct tac aac atg gac tgg cgc ggt cgt gtt tac gct gtg tca atg ttc    1296
   Pro Tyr Asn Met Asp Trp Arg Gly Arg Val Tyr Ala Val Ser Met Phe
               420                 425                 430
   aac ccg caa ggt aac gat atg acc aaa gga ctg ctt acg ctg gcg aaa    1344
   Asn Pro Gln Gly Asn Asp Met Thr Lys Gly Leu Leu Thr Leu Ala Lys
           435                 440                 445
   ggt aaa cca atc ggt aag gaa ggt tac tac tgg ctg aaa atc cac ggt    1392
   Gly Lys Pro Ile Gly Lys Glu Gly Tyr Tyr Trp Leu Lys Ile His Gly
       450                 455                 460
   gca aac tgt gcg ggt gtc gat aag gtt ccg ttc cct gag cgc atc aag    1440
   Ala Asn Cys Ala Gly Val Asp Lys Val Pro Phe Pro Glu Arg Ile Lys
   465                 470                 475                 480
   ttc att gag gaa aac cac gag aac atc atg gct tgc gct aag tct cca    1488
   Phe Ile Glu Glu Asn His Glu Asn Ile Met Ala Cys Ala Lys Ser Pro
                   485                 490                 495
   ctg gag aac act tgg tgg gct gag caa gat tct ccg ttc tgc ttc ctt    1536
   Leu Glu Asn Thr Trp Trp Ala Glu Gln Asp Ser Pro Phe Cys Phe Leu
               500                 505                 510
   gcg ttc tgc ttt gag tac gct ggg gta cag cac cac ggc ctg agc tat    1584
   Ala Phe Cys Phe Glu Tyr Ala Gly Val Gln His His Gly Leu Ser Tyr
           515                 520                 525
   aac tgc tcc ctt ccg ctg gcg ttt gac ggg tct tgc tct ggc atc cag    1632
   Asn Cys Ser Leu Pro Leu Ala Phe Asp Gly Ser Cys Ser Gly Ile Gln
       530                 535                 540
   cac ttc tcc gcg atg ctc cga gat gag gta ggt ggt cgc gcg gtt aac    1680
   His Phe Ser Ala Met Leu Arg Asp Glu Val Gly Gly Arg Ala Val Asn
   545                 550                 555                 560
   ttg ctt cct agt gaa acc gtt cag gac atc tac ggg att gtt gct aag    1728
   Leu Leu Pro Ser Glu Thr Val Gln Asp Ile Tyr Gly Ile Val Ala Lys
                   565                 570                 575
   aaa gtc aac gag att cta caa gca gac gca atc aat ggg acc gat aac    1776
   Lys Val Asn Glu Ile Leu Gln Ala Asp Ala Ile Asn Gly Thr Asp Asn
               580                 585                 590
   gaa gta gtt acc gtg acc gat gag aac act ggt gaa atc tct gag aaa    1824
   Glu Val Val Thr Val Thr Asp Glu Asn Thr Gly Glu Ile Ser Glu Lys
           595                 600                 605
   gtc aag ctg ggc act aag gca ctg gct ggt caa tgg ctg gct tac ggt    1872
   Val Lys Leu Gly Thr Lys Ala Leu Ala Gly Gln Trp Leu Ala Tyr Gly
       610                 615                 620
   gtt act cgc agt gtg act aag cgt tca gtc atg acg ctg gct ttc ggg    1920
   Val Thr Arg Ser Val Thr Lys Arg Ser Val Met Thr Leu Ala Phe Gly
   625                 630                 635                 640
   tcc aaa gag ttc ggc ttc cgt caa caa gtg ctg gaa gat acc att cag    1968
   Ser Lys Glu Phe Gly Phe Arg Gln Gln Val Leu Glu Asp Thr Ile Gln
                   645                 650                 655
   cca gct att gat tcc ggc aag ggt ctg atg ttc act cag ccg aat cag    2016
   Pro Ala Ile Asp Ser Gly Lys Gly Leu Met Phe Thr Gln Pro Asn Gln
               660                 665                 670
   gct gct gga tac atg gct aag ctg att tgg gaa tct gtg agc gtg acg    2064
   Ala Ala Gly Tyr Met Ala Lys Leu Ile Trp Glu Ser Val Ser Val Thr
           675                 680                 685
   gtg gta gct gcg gtt gaa gca atg aac tgg ctt aag tct gct gct aag    2112
   Val Val Ala Ala Val Glu Ala Met Asn Trp Leu Lys Ser Ala Ala Lys
       690                 695                 700
   ctg ctg gct gct gag gtc aaa gat aag aag act gga gag att ctt cgc    2160
   Leu Leu Ala Ala Glu Val Lys Asp Lys Lys Thr Gly Glu Ile Leu Arg
   705                 710                 715                 720
   aag cgt tgc gct gtg cat tgg gta act cct gat ggt ttc cct gtg tgg    2208
   Lys Arg Cys Ala Val His Trp Val Thr Pro Asp Gly Phe Pro Val Trp
                   725                 730                 735
   cag gaa tac aag aag cct att cag acg cgc ttg aac ctg atg ttc ctc     2256
   Gln Glu Tyr Lys Lys Pro Ile Gln Thr Arg Leu Asn Leu Met Phe Leu
               740                 745                 750
   ggt cag ttc cgc tta cag cct acc att aac acc aac aaa gat agc gag     2304
   Gly Gln Phe Arg Leu Gln Pro Thr Ile Asn Thr Asn Lys Asp Ser Glu
           755                 760                 765
   att gat gca cac aaa cag gag tct ggt atc gct cct aac ttt gta cac     2352
   Ile Asp Ala His Lys Gln Glu Ser Gly Ile Ala Pro Asn Phe Val His
       770                 775                 780
   agc caa gac ggt agc cac ctt cgt aag act gta gtg tgg gca cac gag     2400
   Ser Gln Asp Gly Ser His Leu Arg Lys Thr Val Val Trp Ala His Glu
   785                 790                 795                 800
   aag tac gga atc gaa tct ttt gca ctg att cac gac tcc ttc ggt acc     2448
   Lys Tyr Gly Ile Glu Ser Phe Ala Leu Ile His Asp Ser Phe Gly Thr
                   805                 810                 815
   att ccg gct gac gct gcg aac ctg ttc aaa gca gtg cgc gaa act atg     2496
   Ile Pro Ala Asp Ala Ala Asn Leu Phe Lys Ala Val Arg Glu Thr Met
               820                 825                 830
   gtt gac aca tat gag tct tgt gat gta ctg gct gat ttc tac gac cag     2544
   Val Asp Thr Tyr Glu Ser Cys Asp Val Leu Ala Asp Phe Tyr Asp Gln
           835                 840                 845
   ttc gct gac cag ttg cac gag tct caa ttg gac aaa atg cca gca ctt     2592
   Phe Ala Asp Gln Leu His Glu Ser Gln Leu Asp Lys Met Pro Ala Leu
       850                 855                 860
   ccg gct aaa ggt aac ttg aac ctc cgt gac atc tta gag tcg gac ttc     2640
   Pro Ala Lys Gly Asn Leu Asn Leu Arg Asp Ile Leu Glu Ser Asp Phe
   865                 870                 875                 880
   gcg ttc gcg taa                                                     2652
   Ala Phe Ala
<210> 10
   <211> 883
   <212> PRT
   <213> Séquence artificielle
   <223> Description de la séquence artificielle: séquences mutées de l'ARN polymérase du bactériophage T7
<400> 10
   Met Asn Thr Ile Asn Ile Ala Lys Asn Asp Phe Ser Asp Ile Glu Leu
    1               5                  10                  15
   Ala Ala Ile Pro Phe Asn Thr Leu Ala Asp His Tyr Gly Glu Arg Leu
               20                  25                  30
   Ala Arg Glu Gln Leu Ala Leu Glu His Glu Ser Tyr Glu Met Gly Glu
           35                  40                  45
   Ala Arg Phe Arg Lys Met Phe Glu Arg Gln Leu Lys Ala Gly Glu Val
       50                  55                  60
   Ala Asp Asn Ala Ala Ala Lys Pro Leu Ile Thr Thr Leu Leu Pro Lys
   65 70 75 80
   Met Ile Ala Arg Ile Asn Asp Trp Phe Glu Glu Val Lys Ala Lys Arg
   85 90 95
   Gly Lys Arg Pro Thr Ala Phe Gln Phe Leu Gln Glu Ile Lys Pro Glu
   100 105 110
   Ala Val Ala Tyr Ile Thr Ile Lys Thr Thr Leu Ala Cys Leu Thr Ser
   115 120 125
   Ala Asp Asn Thr Thr Val Gln Ala Val Ala Ser Ala Ile Gly Arg Ala
   130 135 140
   Ile Glu Asp Glu Ala Arg Phe Gly Arg Ile Arg Asp Leu Glu Ala Lys
   145 150 155 160
   His Phe Lys Lys Asn Val Glu Glu Gln Leu Asn Lys Arg Val Gly His
   165 170 175
   Val Tyr Lys Lys Ala Phe Met Gln Val Val Glu Ala Asp Met Leu Ser
   180 185 190
   Lys Gly Leu Leu Gly Gly Glu Ala Trp Ser Ser Trp His Lys Glu Asp
   195 200 205
   Ser Ile His Val Gly Val Arg Cys Ile Glu Met Leu Ile Glu Ser Thr
   210 215 220
   Gly Met Val Ser Leu His Arg Gln Asn Ala Gly Val Val Gly Gln Asp
   225 230 235 240
   Ser Glu Thr Ile Glu Leu Ala Pro Glu Tyr Ala Glu Ala Ile Ala Thr
   245 250 255
   Arg Ala Gly Ala Leu Ala Gly Ile Ser Leu Met Phe Gln Pro Cys Val
   260 265 270
   Val Pro Pro Lys Pro Trp Thr Gly Ile Thr Gly Gly Gly Tyr Trp Ala
   275 280 285
   Asn Gly Arg Arg Pro Leu Ala Leu Val Arg Thr His Ser Lys Lys Ala
   290 295 300
   Leu Met Arg Tyr Glu Asp Val Tyr Met Pro Glu Val Tyr Lys Ala Ile
   305 310 315 320
   Asn Ile Ala Gln Asn Thr Ala Trp Lys Ile Asn Lys Lys Val Leu Ala
   325 330 335
   Val Ala Asn Val Ile Thr Lys Trp Lys His Cys Pro Val Glu Asp Ile
   340 345 350
   Pro Ala Ile Glu Arg Glu Glu Leu Pro Met Lys Pro Glu Asp Ile Asp
   355 360 365
   Met Asn Pro Glu Ala Leu Thr Ala Trp Lys Arg Ala Ala Ala Ala Val
   370 375 380
   Tyr Arg Lys Asp Lys Ala Arg Lys Ser Arg Arg Ile Ser Leu Glu Phe
   385 390 395 400
   Met Leu Glu Gln Ala Asn Lys Phe Ala Asn His Lys Ala Ile Trp Phe
   405 410 415
   Pro Tyr Asn Met Asp Trp Arg Gly Arg Val Tyr Ala Val Ser Met Phe
   420 425 430
   Asn Pro Gln Gly Asn Asp Met Thr Lys Gly Leu Leu Thr Leu Ala Lys
   435 440 445
   Gly Lys Pro Ile Gly Lys Glu Gly Tyr Tyr Trp Leu Lys Ile His Gly
   450 455 460
   Ala Asn Cys Ala Gly Val Asp Lys Val Pro Phe Pro Glu Arg Ile Lys
   465 470 475 480
   Phe Ile Glu Glu Asn His Glu Asn Ile Met Ala Cys Ala Lys Ser Pro
   485 490 495
   Leu Glu Asn Thr Trp Trp Ala Glu Gln Asp Ser Pro Phe Cys Phe Leu
   500 505 510
   Ala Phe Cys Phe Glu Tyr Ala Gly Val Gln His His Gly Leu Ser Tyr
   515 520 525
   Asn Cys Ser Leu Pro Leu Ala Phe Asp Gly Ser Cys Ser Gly Ile Gln
   530 535 540
   His Phe Ser Ala Met Leu Arg Asp Glu Val Gly Gly Arg Ala Val Asn
   545 550 555 560
   Leu Leu Pro Ser Glu Thr Val Gln Asp Ile Tyr Gly Ile Val Ala Lys
   565 570 575
   Lys Val Asn Glu Ile Leu Gln Ala Asp Ala Ile Asn Gly Thr Asp Asn
   580 585 590
   Glu Val Val Thr Val Thr Asp Glu Asn Thr Gly Glu Ile Ser Glu Lys
   595 600 605
   Val Lys Leu Gly Thr Lys Ala Leu Ala Gly Gln Trp Leu Ala Tyr Gly
   610 615 620
   Val Thr Arg Ser Val Thr Lys Arg Ser Val Met Thr Leu Ala Phe Gly
   625 630 635 640
   Ser Lys Glu Phe Gly Phe Arg Gln Gln Val Leu Glu Asp Thr Ile Gln
   645 650 655
   Pro Ala Ile Asp Ser Gly Lys Gly Leu Met Phe Thr Gln Pro Asn Gln
   660 665 670
   Ala Ala Gly Tyr Met Ala Lys Leu Ile Trp Glu Ser Val Ser Val Thr
   675 680 685
   Val Val Ala Ala Val Glu Ala Met Asn Trp Leu Lys Ser Ala Ala Lys
   690 695 700
   Leu Leu Ala Ala Glu Val Lys Asp Lys Lys Thr Gly Glu Ile Leu Arg
   705                 710                 715                 720
   Lys Arg Cys Ala Val His Trp Val Thr Pro Asp Gly Phe Pro Val Trp
                   725                 730                 735
   Gln Glu Tyr Lys Lys Pro Ile Gln Thr Arg Leu Asn Leu Met Phe Leu
               740                 745                 750
   Gly Gln Phe Arg Leu Gln Pro Thr Ile Asn Thr Asn Lys Asp Ser Glu
           755                 760                 765
   Ile Asp Ala His Lys Gln Glu Ser Gly Ile Ala Pro Asn Phe Val His
       770                 775                 780
   Ser Gln Asp Gly Ser His Leu Arg Lys Thr Val Val Trp Ala His Glu
   785                 790                 795                 800
   Lys Tyr Gly Ile Glu Ser Phe Ala Leu Ile His Asp Ser Phe Gly Thr
                   805                 810                 815
   Ile Pro Ala Asp Ala Ala Asn Leu Phe Lys Ala Val Arg Glu Thr Met
               820                 825                 830
   Val Asp Thr Tyr Glu Ser Cys Asp Val Leu Ala Asp Phe Tyr Asp Gln
           835                 840                 845
   Phe Ala Asp Gln Leu His Glu Ser Gln Leu Asp Lys Met Pro Ala Leu
       850                 855                 860
   Pro Ala Lys Gly Asn Leu Asn Leu Arg Asp Ile Leu Glu Ser Asp Phe
   865                 870                 875                 880
   Ala Phe Ala
<210> 11
   <211> 2652
   <212> ADN
   <213> Séquence artificielle
<220>
   <223> Description de la séquence artificielle: séquences mutées de l'ARN polymérase du bactériophage T7
<220>
   <221> CDS
   <222> (1)..(2652)
<400> 11
   atg aac acg att aac atc gct aag aac gac ttc tct gac atc gaa ctg   48
   Met Asn Thr Ile Asn Ile Ala Lys Asn Asp Phe Ser Asp Ile Glu Leu
    1               5                  10                  15
   gct gct atc ccg ttc aac act ctg gct gac cat tac ggt gag cgt tta   96
   Ala Ala Ile Pro Phe Asn Thr Leu Ala Asp His Tyr Gly Glu Arg Leu
               20                  25                  30
   gct cgc gaa cag ttg gcc ctt gag cat gag tct tac gag atg ggt gaa     144
   Ala Arg Glu Gln Leu Ala Leu Glu His Glu Ser Tyr Glu Met Gly Glu
               35                  40                  45
   gca cgc ttc cgc aag atg ttt gag cgt caa ctt aaa gct ggt gag gtt     192
   Ala Arg Phe Arg Lys Met Phe Glu Arg Gln Leu Lys Ala Gly Glu Val
           50                  55                  60
   gcg gat aac gct gcc gcc aag cct ctc atc act acc cta ctc cct aag     240
   Ala Asp Asn Ala Ala Ala Lys Pro Leu Ile Thr Thr Leu Leu Pro Lys
   65                  70                  75                  80
   atg att gca cgc atc aac gac tgg ttt gag gaa gtg aaa gct aag cgc     288
   Met Ile Ala Arg Ile Asn Asp Trp Phe Glu Glu Val Lys Ala Lys Arg
                   85                  90                  95
   ggc aag cgc ccg aca gcc ttc cag ttc ctg caa gaa atc aag ccg gaa     336
   Gly Lys Arg Pro Thr Ala Phe Gln Phe Leu Gln Glu Ile Lys Pro Glu
               100                 105                 110
   gcc gta gcg tac atc acc att aag acc act ctg gct tgc cta acc agt     384
   Ala Val Ala Tyr Ile Thr Ile Lys Thr Thr Leu Ala Cys Leu Thr Ser
           115                 120                 125
   gct gac aat aca acc gtt cag gct gta gca agc gca atc ggt cgg gcc     432
   Ala Asp Asn Thr Thr Val Gln Ala Val Ala Ser Ala Ile Gly Arg Ala
       130                 135                 140
   att gag gac gag gct cgc ttc ggt cgt atc cgt gac ctt gaa gct aag     480
   Ile Glu Asp Glu Ala Arg Phe Gly Arg Ile Arg Asp Leu Glu Ala Lys
   145                 150                 155                 160
   cac ttc aag aaa aac gtt gag gaa caa ctc aac aag cgc gta ggg cac     528
   His Phe Lys Lys Asn Val Glu Glu Gln Leu Asn Lys Arg Val Gly His
                   165                 170                 175
   gtc tac aag aaa gca ttt atg caa gtt gtc gag gct gac atg ctc tct     576
   Val Tyr Lys Lys Ala Phe Met Gln Val Val Glu Ala Asp Met Leu Ser
               180                 185                 190
   aag ggt cta ctc ggt ggc gag gcg tgg tct tcg tgg cat aag gaa gac     624
   Lys Gly Leu Leu Gly Gly Glu Ala Trp Ser Ser Trp His Lys Glu Asp
           195                 200                 205
   tct att cat gta gga gta cgc tgc atc gag atg ctc att gag tca acc     672
   Ser Ile His Val Gly Val Arg Cys Ile Glu Met Leu Ile Glu Ser Thr
       210                 215                 220
   gga atg gtt agc tta cac cgc caa aat gct ggc gta gta ggt caa gac     720
   Gly Met Val Ser Leu His Arg Gln Asn Ala Gly Val Val Gly Gln Asp
   225                 230                 235                 240
   tct gag act atc gaa ctc gca cct gaa tac gct gag gct atc gca acc     768
   Ser Glu Thr Ile Glu Leu Ala Pro Glu Tyr Ala Glu Ala Ile Ala Thr
                   245                 250                 255
   cgt gca ggt gcg ctg gct ggc atc tct ccg atg ttc caa cct tgc gta     816
   Arg Ala Gly Ala Leu Ala Gly Ile Ser Pro Met Phe Gln Pro Cys Val
               260                 265                 270
   gtt cct cct aag ccg tgg act ggc att act ggt ggt ggc tat tgg gct    864
   Val Pro Pro Lys Pro Trp Thr Gly Ile Thr Gly Gly Gly Tyr Trp Ala
           275                 280                 285
   aac ggt cgt cgt cct ctg gcg ctg gtg cgt act cac agt aag aaa gca    912
   Asn Gly Arg Arg Pro Leu Ala Leu Val Arg Thr His Ser Lys Lys Ala
       290                 295                 300
   ctg atg cgc tac gaa gac gtt tac atg cct gag gtg tac aaa gcg att    960
   Leu Met Arg Tyr Glu Asp Val Tyr Met Pro Glu Val Tyr Lys Ala Ile
   305                 310                 315                 320
   aac att gcg caa aac acc gca tgg aaa atc aac aag aaa gtc cta gcg    1008
   Asn Ile Ala Gln Asn Thr Ala Trp Lys Ile Asn Lys Lys Val Leu Ala
                   325                 330                 335
   gtc gcc aac gta atc acc aag tgg aag cat tgt ccg gtc gag gac atc    1056
   Val Ala Asn Val Ile Thr Lys Trp Lys His Cys Pro Val Glu Asp Ile
               340                 345                 350
   cct gcg att gag cgt gaa gaa ctc ccg atg aaa ccg gaa gac atc gac    1104
   Pro Ala Ile Glu Arg Glu Glu Leu Pro Met Lys Pro Glu Asp Ile Asp
           355                 360                 365
   atg aat cct gag gct ctc acc gcg tgg aaa cgt gct gcc gct gct gtg    1152
   Met Asn Pro Glu Ala Leu Thr Ala Trp Lys Arg Ala Ala Ala Ala Val
       370                 375                 380
   tac cgc aag gac aag gct cgc aag tct cgc cgt atc agc ctt gag ttc    1200
   Tyr Arg Lys Asp Lys Ala Arg Lys Ser Arg Arg Ile Ser Leu Glu Phe
   385                 390                 395                 400
   atg ctt gag caa gcc aat aag ttt gct aac cat aag gcc atc tgg ttc    1248
   Met Leu Glu Gln Ala Asn Lys Phe Ala Asn His Lys Ala Ile Trp Phe
                   405                 410                 415
   cct tac aac atg gac tgg cgc ggt cgt gtt tac gct gtg tca atg ttc    1296
   Pro Tyr Asn Met Asp Trp Arg Gly Arg Val Tyr Ala Val Ser Met Phe
               420                 425                 430
   aac ccg caa ggt aac gat atg acc aaa gga ctg ctt acg ctg gcg aaa    1344
   Asn Pro Gln Gly Asn Asp Met Thr Lys Gly Leu Leu Thr Leu Ala Lys
           435                 440                 445
   ggt aaa cca atc ggt aag gaa ggt tac tac tgg ctg aaa atc cac ggt    1392
   Gly Lys Pro Ile Gly Lys Glu Gly Tyr Tyr Trp Leu Lys Ile His Gly
       450                 455                 460
   gca aac tgt gcg ggt gtc gat aag gtt ccg ttc cct gag cgc atc aag    1440
   Ala Asn Cys Ala Gly Val Asp Lys Val Pro Phe Pro Glu Arg Ile Lys
   465                 470                 475                 480
   ttc att gag gaa aac cac gag aac atc atg gct tgc gct aag tct cca    1488
   Phe Ile Glu Glu Asn His Glu Asn Ile Met Ala Cys Ala Lys Ser Pro
                   485                 490                 495
   ctg gag aac act tgg tgg gct gag caa gat tct ccg ttc tgc ttc ctt    1536
   Leu Glu Asn Thr Trp Trp Ala Glu Gln Asp Ser Pro Phe Cys Phe Leu
               500                 505                 510
   gcg ttc tgc ttt gag tac gct ggg gta cag cac cac ggc ctg agc tat    1584
   Ala Phe Cys Phe Glu Tyr Ala Gly Val Gln His His Gly Leu Ser Tyr
           515                 520                 525
   aac tgc tcc ctt ccg ctg gcg ttt gac ggg tct tgc tct ggc atc cag    1632
   Asn Cys Ser Leu Pro Leu Ala Phe Asp Gly Ser Cys Ser Gly Ile Gln
       530                 535                 540
   cac ttc tcc gcg atg ctc cga gat gag gta ggt ggt cgc gcg gtt aac    1680
   His Phe Ser Ala Met Leu Arg Asp Glu Val Gly Gly Arg Ala Val Asn
   545                 550                 555                 560
   ttg ctt cct agt gaa acc gtt cag gac atc tac ggg att gtt gct aag    1728
   Leu Leu Pro Ser Glu Thr Val Gln Asp Ile Tyr Gly Ile Val Ala Lys
                   565                 570                 575
   aaa gtc aac gag att cta caa gca gac gca atc aat ggg acc gat aac    1776
   Lys Val Asn Glu Ile Leu Gln Ala Asp Ala Ile Asn Gly Thr Asp Asn
               580                 585                 590
   gaa gta gtt acc gtg acc gat gag aac act ggt gaa atc tct gag aaa    1824
   Glu Val Val Thr Val Thr Asp Glu Asn Thr Gly Glu Ile Ser Glu Lys
           595                 600                 605
   gtc aag ctg ggc act aag gca ctg gct ggt caa tgg ctg gct tac ggt    1872
   Val Lys Leu Gly Thr Lys Ala Leu Ala Gly Gln Trp Leu Ala Tyr Gly
       610                 615                 620
   gtt act cgc agt gtg act aag cgt tca gtc atg acg ctg gct tac ggg    1920
   Val Thr Arg Ser Val Thr Lys Arg Ser Val Met Thr Leu Ala Tyr Gly
   625                 630                 635                 640
   tcc aaa gag ttc ggc ttc cgt caa caa gtg ctg gaa gat acc att cag    1968
   Ser Lys Glu Phe Gly Phe Arg Gln Gln Val Leu Glu Asp Thr Ile Gln
                   645                 650                 655
   cca gct att gat tcc ggc aag ggt ctg atg ttc act cag ccg aat cag    2016
   Pro Ala Ile Asp Ser Gly Lys Gly Leu Met Phe Thr Gln Pro Asn Gln
               660                 665                 670
   gct gct gga tac atg gct aag ctg att tgg gaa tct gtg agc gtg acg    2064
   Ala Ala Gly Tyr Met Ala Lys Leu Ile Trp Glu Ser Val Ser Val Thr
           675                 680                 685
   gtg gta gct gcg gtt gaa gca atg aac tgg ctt aag tct gct gct aag    2112
   Val Val Ala Ala Val Glu Ala Met Asn Trp Leu Lys Ser Ala Ala Lys
       690                 695                 700
   ctg ctg gct gct gag gtc aaa gat aag aag act gga gag att ctt cgc    2160
   Leu Leu Ala Ala Glu Val Lys Asp Lys Lys Thr Gly Glu Ile Leu Arg
   705                 710                 715                 720
   aag cgt tgc gct gtg cat tgg gta act cct gat ggt ttc cct gtg tgg    2208
   Lys Arg Cys Ala Val His Trp Val Thr Pro Asp Gly Phe Pro Val Trp
                   725                 730                 735
   cag gaa tac aag aag cct att cag acg cgc ttg aac ctg atg ttc ctc    2256
   Gln Glu Tyr Lys Lys Pro Ile Gln Thr Arg Leu Asn Leu Met Phe Leu
               740                 745                 750
   ggt cag ttc cgc tta cag cct acc att aac acc aac aaa gat agc gag   2304
   Gly Gln Phe Arg Leu Gln Pro Thr Ile Asn Thr Asn Lys Asp Ser Glu
           755                 760                 765
   att gat gca cac aaa cag gag tct ggt atc gct cct aac ttt gta cac   2352
   Ile Asp Ala His Lys Gln Glu Ser Gly Ile Ala Pro Asn Phe Val His
       770                 775                 780
   agc caa gac ggt agc cac ctt cgt aag act gta gtg tgg gca cac gag   2400
   Ser Gln Asp Gly Ser His Leu Arg Lys Thr Val Val Trp Ala His Glu
   785                 790                 795                 800
   aag tac gga atc gaa tct ttt gca ctg aat cac gac tcc ttc ggt acc   2448
   Lys Tyr Gly Ile Glu Ser Phe Ala Leu Asn His Asp Ser Phe Gly Thr
                   805                 810                 815
   att ccg gct gac gct gcg aac ctg ttc aaa gca gtg cgc gaa act atg   2496
   Ile Pro Ala Asp Ala Ala Asn Leu Phe Lys Ala Val Arg Glu Thr Met
               820                 825                 830
   gtt gac aca tat gag tct tgt gat gta ctg gct gat ttc tac gac cag   2544
   Val Asp Thr Tyr Glu Ser Cys Asp Val Leu Ala Asp Phe Tyr Asp Gln
           835                 840                 845
   ttc gct gac cag ttg cac gag tct caa ttg gac aaa atg cca gca ctt   2592
   Phe Ala Asp Gln Leu His Glu Ser Gln Leu Asp Lys Met Pro Ala Leu
       850                 855                 860
   ccg gct aaa ggt aac ttg aac ctc cgt gac atc tta gag tcg gac ttc   2640
   Pro Ala Lys Gly Asn Leu Asn Leu Arg Asp Ile Leu Glu Ser Asp Phe
   865                 870                 875                 880
   gcg ttc gcg taa                                                   2652
   Ala Phe Ala
<210> 12
   <211> 883 .
   <212> PRT
   <213> Séquence artificielle
   <223> Description de la séquence artificielle: séquences mutées de l'ARN polymérase du bactériophage T7
<400> 12
   Met Asn Thr Ile Asn Ile Ala Lys Asn Asp Phe Ser Asp Ile Glu Leu
    1               5                  10                  15
   Ala Ala Ile Pro Phe Asn Thr Leu Ala Asp His Tyr Gly Glu Arg Leu
               20                  25                  30
   Ala Arg Glu Gln Leu Ala Leu Glu His Glu Ser Tyr Glu Met Gly Glu
           35                  40                  45
   Ala Arg Phe Arg Lys Met Phe Glu Arg Gln Leu Lys Ala Gly Glu Val
       50                  55                  60
   Ala Asp Asn Ala Ala Ala Lys Pro Leu Ile Thr Thr Leu Leu Pro Lys
   65                  70                  75                  80
   Met Ile Ala Arg Ile Asn Asp Trp Phe Glu Glu Val Lys Ala Lys Arg
                   85                  90                  95
   Gly Lys Arg Pro Thr Ala Phe Gln Phe Leu Gln Glu Ile Lys Pro Glu
               100                 105                 110
   Ala Val Ala Tyr Ile Thr Ile Lys Thr Thr Leu Ala Cys Leu Thr Ser
           115                 120                 125
   Ala Asp Asn Thr Thr Val Gln Ala Val Ala Ser Ala Ile Gly Arg Ala
       130                 135                 140
   Ile Glu Asp Glu Ala Arg Phe Gly Arg Ile Arg Asp Leu Glu Ala Lys
   145                 150                 155                 160
   His Phe Lys Lys Asn Val Glu Glu Gln Leu Asn Lys Arg Val Gly His
                   165                 170                 175
   Val Tyr Lys Lys Ala Phe Met Gln Val Val Glu Ala Asp Met Leu Ser
               180                 185                 190
   Lys Gly Leu Leu Gly Gly Glu Ala Trp Ser Ser Trp His Lys Glu Asp
           195                 200                 205
   Ser Ile His Val Gly Val Arg Cys Ile Glu Met Leu Ile Glu Ser Thr
       210                 215                 220
   Gly Met Val Ser Leu His Arg Gln Asn Ala Gly Val Val Gly Gln Asp
   225                 230                 235                 240
   Ser Glu Thr Ile Glu Leu Ala Pro Glu Tyr Ala Glu Ala Ile Ala Thr
                   245                 250                 255
   Arg Ala Gly Ala Leu Ala Gly Ile Ser Pro Met Phe Gln Pro Cys Val
               260                 265                 270
   Val Pro Pro Lys Pro Trp Thr Gly Ile Thr Gly Gly Gly Tyr Trp Ala
           275                 280                 285
   Asn Gly Arg Arg Pro Leu Ala Leu Val Arg Thr His Ser Lys Lys Ala
       290                 295                 300
   Leu Met Arg Tyr Glu Asp Val Tyr Met Pro Glu Val Tyr Lys Ala Ile
   305                 310                 315                 320
   Asn Ile Ala Gln Asn Thr Ala Trp Lys Ile Asn Lys Lys Val Leu Ala
                   325                 330                 335
   Val Ala Asn Val Ile Thr Lys Trp Lys His Cys Pro Val Glu Asp Ile
               340                 345                 350
   Pro Ala Ile Glu Arg Glu Glu Leu Pro Met Lys Pro Glu Asp Ile Asp
           355                 360                 365
   Met Asn Pro Glu Ala Leu Thr Ala Trp Lys Arg Ala Ala Ala Ala Val
       370                 375                 380
   Tyr Arg Lys Asp Lys Ala Arg Lys Ser Arg Arg Ile Ser Leu Glu Phe
   385                 390                 395                 400
   Met Leu Glu Gln Ala Asn Lys Phe Ala Asn His Lys Ala Ile Trp Phe
                   405                 410                 415
   Pro Tyr Asn Met Asp Trp Arg Gly Arg Val Tyr Ala Val Ser Met Phe
               420                 425                 430
   Asn Pro Gln Gly Asn Asp Met Thr Lys Gly Leu Leu Thr Leu Ala Lys
           435                 440                 445
   Gly Lys Pro Ile Gly Lys Glu Gly Tyr Tyr Trp Leu Lys Ile His Gly
       450                 455                 460
   Ala Asn Cys Ala Gly Val Asp Lys Val Pro Phe Pro Glu Arg Ile Lys
   465                 470                 475                 480
   Phe Ile Glu Glu Asn His Glu Asn Ile Met Ala Cys Ala Lys Ser Pro
                   485                 490                 495
   Leu Glu Asn Thr Trp Trp Ala Glu Gln Asp Ser Pro Phe Cys Phe Leu
               500                 505                 510
   Ala Phe Cys Phe Glu Tyr Ala Gly Val Gln His His Gly Leu Ser Tyr
           515                 520                 525
   Asn Cys Ser Leu Pro Leu Ala Phe Asp Gly Ser Cys Ser Gly Ile Gln
       530                 535                 540
   His Phe Ser Ala Met Leu Arg Asp Glu Val Gly Gly Arg Ala Val Asn
   545                 550                 555                 560
   Leu Leu Pro Ser Glu Thr Val Gln Asp Ile Tyr Gly Ile Val Ala Lys
                   565                 570                 575
   Lys Val Asn Glu Ile Leu Gln Ala Asp Ala Ile Asn Gly Thr Asp Asn
               580                 585                 590
   Glu Val Val Thr Val Thr Asp Glu Asn Thr Gly Glu Ile Ser Glu Lys
           595                 600                 605
   Val Lys Leu Gly Thr Lys Ala Leu Ala Gly Gln Trp Leu Ala Tyr Gly
       610                 615                 620
   Val Thr Arg Ser Val Thr Lys Arg Ser Val Met Thr Leu Ala Tyr Gly
   625                 630                 635                 640
   Ser Lys Glu Phe Gly Phe Arg Gln Gln Val Leu Glu Asp Thr Ile Gln
                   645                 650                 655
   Pro Ala Ile Asp Ser Gly Lys Gly Leu Met Phe Thr Gln Pro Asn Gln
               660                 665                 670
   Ala Ala Gly Tyr Met Ala Lys Leu Ile Trp Glu Ser Val Ser Val Thr
           675                 680                 685
   Val Val Ala Ala Val Glu Ala Met Asn Trp Leu Lys Ser Ala Ala Lys
       690                 695                 700
   Leu Leu Ala Ala Glu Val Lys Asp Lys Lys Thr Gly Glu Ile Leu Arg
   705                 710                 715                 720
   Lys Arg Cys Ala Val His Trp Val Thr Pro Asp Gly Phe Pro Val Trp
                   725                 730                 735
   Gln Glu Tyr Lys Lys Pro Ile Gln Thr Arg Leu Asn Leu Met Phe Leu
   740 745 750
   Gly Gln Phe Arg Leu Gln Pro Thr Ile Asn Thr Asn Lys Asp Ser Glu
   755 760 765
   Ile Asp Ala His Lys Gln Glu Ser Gly Ile Ala Pro Asn Phe Val His
   770 775 780
   Ser Gln Asp Gly Ser His Leu Arg Lys Thr Val Val Trp Ala His Glu
   785 790 795 800
   Lys Tyr Gly Ile Glu Ser Phe Ala Leu Asn His Asp Ser Phe Gly Thr
   805 810 815
   Ile Pro Ala Asp Ala Ala Asn Leu Phe Lys Ala Val Arg Glu Thr Met
   820 825 830
   Val Asp Thr Tyr Glu Ser Cys Asp Val Leu Ala Asp Phe Tyr Asp Gln
   835 840 845
   Phe Ala Asp Gln Leu His Glu Ser Gln Leu Asp Lys Met Pro Ala Leu
   850 855 860
   Pro Ala Lys Gly Asn Leu Asn Leu Arg Asp Ile Leu Glu Ser Asp Phe
   865 870 875 880
   Ala Phe Ala
<210> 13
   <211> 2652
   <212> ADN
   <213> Séquence artificielle
<220>
   <223> Description de la séquence artificielle: séquences mutées de l'ARN polymérase du bactériophage T7
<220>
   <221> CDS
   <222> (1)..(2652)
<400> 13
   atg aac acg att aac atc gct aag aac gac ttc tct gac atc gaa ctg     48
   Met Asn Thr Ile Asn Ile Ala Lys Asn Asp Phe Ser Asp Ile Glu Leu
     1               5                  10                  15
   gct gct atc ccg ttc aac act ctg gct gac cat tac ggt gag cgt tta     96
   Ala Ala Ile Pro Phe Asn Thr Leu Ala Asp His Tyr Gly Glu Arg Leu
                    20                  25                  30
   gct cgc gaa cag ttg gcc ctt gag cat gag tct tac gag atg ggt gaa    144
   Ala Arg Glu Gln Leu Ala Leu Glu His Glu Ser Tyr Glu Met Gly Glu
                35                  40                  45
   gca cgc ttc cgc aag atg ttt gag cgt caa ctt aaa gct ggt gag gtt    192
   Ala Arg Phe Arg Lys Met Phe Glu Arg Gln Leu Lys Ala Gly Glu Val
            50                  55                  60
   gcg gat aac gct gcc gcc aag cct ctc atc act acc cta ctc cct aag     240
   Ala Asp Asn Ala Ala Ala Lys Pro Leu Ile Thr Thr Leu Leu Pro Lys
    65                  70                  75                  80
   atg att gca cgc atc aac gac tgg ttt gag gaa gtg aaa gct aag cgc     288
   Met Ile Ala Arg Ile Asn Asp Trp Phe Glu Glu Val Lys Ala Lys Arg
                    85                  90                  95
   ggc aag cgc ccg aca gcc ttc cag ttc ctg caa gaa atc aag ccg gaa     336
   Gly Lys Arg Pro Thr Ala Phe Gln Phe Leu Gln Glu Ile Lys Pro Glu
               100                 105                 110
   gcc gta gcg tac atc acc att aag acc act ctg gct tgc cta acc agt     384
   Ala Val Ala Tyr Ile Thr Ile Lys Thr Thr Leu Ala Cys Leu Thr Ser
           115                 120                 125
   gct gac aat aca acc gtt cag gct gta gca agc gca atc ggt cgg gcc     432
   Ala Asp Asn Thr Thr Val Gln Ala Val Ala Ser Ala Ile Gly Arg Ala
       130                 135                 140
   att gag gac gag gct cgc ttc ggt cgt atc cgt gac ctt gaa gct aag     480
   Ile Glu Asp Glu Ala Arg Phe Gly Arg Ile Arg Asp Leu Glu Ala Lys
   145                 150                 155                 160
   cac ttc aag aaa aac gtt gag gaa caa ctc aac aag cgc gta ggg cac     528
   His Phe Lys Lys Asn Val Glu Glu Gln Leu Asn Lys Arg Val Gly His
                   165                 170                 175
   gtc tac aag aaa gca ttt atg caa gtt gtc gag gct gac atg ctc tct     576
   Val Tyr Lys Lys Ala Phe Met Gln Val Val Glu Ala Asp Met Leu Ser
               180                 185                 190
   aag ggt cta ctc ggt ggc gag gcg tgg tct tcg tgg cat aag gaa gac     624
   Lys Gly Leu Leu Gly Gly Glu Ala Trp Ser Ser Trp His Lys Glu Asp
           195                 200                 205
   tct att cat gta gga gta cgc tgc atc gag atg ctc att gag tca acc     672
   Ser Ile His Val Gly Val Arg Cys Ile Glu Met Leu Ile Glu Ser Thr
       210                 215                 220
   gga atg gtt agc tta cac cgc caa aat gct ggc gta gta ggt caa gac     720
   Gly Met Val Ser Leu His Arg Gln Asn Ala Gly Val Val Gly Gln Asp
   225                 230                 235                 240
   tct gag act atc gaa ctc gca cct gaa tac gct gag gct atc gca acc     768
   Ser Glu Thr Ile Glu Leu Ala Pro Glu Tyr Ala Glu Ala Ile Ala Thr
                   245                 250                 255
   cgt gca ggt gcg ctg gct ggc atc tct ctg atg ttc caa cct tgc gta     816
   Arg Ala Gly Ala Leu Ala Gly Ile Ser Leu Met Phe Gln Pro Cys Val
               260                 265                 270
   gtt cct cct aag ccg tgg act ggc att act ggt ggt ggc tat tgg gct     864
   Val Pro Pro Lys Pro Trp Thr Gly Ile Thr Gly Gly Gly Tyr Trp Ala
           275                 280                 285
   aac ggt cgt cgt cct ctg gcg ctg gtg cgt act cac agt aag aaa gca     912
   Asn Gly Arg Arg Pro Leu Ala Leu Val Arg Thr His Ser Lys Lys Ala
       290                 295                 300
   ctg atg cgc tac gaa gac gtt tac atg cct gag gtg tac aaa gcg att    960
   Leu Met Arg Tyr Glu Asp Val Tyr Met Pro Glu Val Tyr Lys Ala Ile
   305                 310                 315                 320
   aac att gcg caa aac acc gca tgg aaa atc aac aag aaa gtc cta gcg   1008
   Asn Ile Ala Gln Asn Thr Ala Trp Lys Ile Asn Lys Lys Val Leu Ala
                   325                 330                 335
   gtc gcc aac gta atc acc aag tgg aag cat tgt ccg gtc gag gac atc   1056
   Val Ala Asn Val Ile Thr Lys Trp Lys His Cys Pro Val Glu Asp Ile
               340                 345                 350
   cct gcg att gag cgt gaa gaa ctc ccg atg aaa ccg gaa gac atc gac   1104
   Pro Ala Ile Glu Arg Glu Glu Leu Pro Met Lys Pro Glu Asp Ile Asp
           355                 360                 365
   atg aat cct gag gct ctc acc gcg tgg aaa cgt gct gcc gct gct gtg   1152
   Met Asn Pro Glu Ala Leu Thr Ala Trp Lys Arg Ala Ala Ala Ala Val
       370                 375                 380
   tac cgc aag gac aag gct cgc aag tct cgc cgt atc agc ctt gag ttc   1200
   Tyr Arg Lys Asp Lys Ala Arg Lys Ser Arg Arg Ile Ser Leu Glu Phe
   385                 390                 395                 400
   atg ctt gag caa gcc aat aag ttt gct aac cat aag gcc atc tgg ttc   1248
   Met Leu Glu Gln Ala Asn Lys Phe Ala Asn His Lys Ala Ile Trp Phe
                   405                 410                 415
   cct tac aac atg gac tgg cgc ggt cgt gtt tac gct gtg tca atg ttc   1296
   Pro Tyr Asn Met Asp Trp Arg Gly Arg Val Tyr Ala Val Ser Met Phe
               420                 425                 430
   aac ccg caa ggt aac gat atg acc aaa gga ctg ctt acg ctg gcg aaa   1344
   Asn Pro Gln Gly Asn Asp Met Thr Lys Gly Leu Leu Thr Leu Ala Lys
           435                 440                 445
   ggt aaa cca atc ggt aag gaa ggt tac tac tgg ctg aaa atc cac ggt   1392
   Gly Lys Pro Ile Gly Lys Glu Gly Tyr Tyr Trp Leu Lys Ile His Gly
       450                 455                 460
   gca aac tgt gcg ggt gtc gat aag gtt ccg ttc cct gag cgc atc aag   1440
   Ala Asn Cys Ala Gly Val Asp Lys Val Pro Phe Pro Glu Arg Ile Lys
   465                 470                 475                 480
   ttc att gag gaa aac cac gag aac atc atg gct tgc gct aag tct cca   1488
   Phe Ile Glu Glu Asn His Glu Asn Ile Met Ala Cys Ala Lys Ser Pro
                   485                 490                 495
   ctg gag aac act tgg tgg gct gag caa gat tct ccg ttc tgc ttc ctt   1536
   Leu Glu Asn Thr Trp Trp Ala Glu Gln Asp Ser Pro Phe Cys Phe Leu
               500                 505                 510
   gcg ttc tgc ttt gag tac gct ggg gta cag cac cac ggc ctg agc tat   1584
   Ala Phe Cys Phe Glu Tyr Ala Gly Val Gln His His Gly Leu Ser Tyr
           515                 520                 525
   aac tgc tcc ctt ccg ctg gcg ttt gac ggg tct tgc tct ggc atc cag   1632
   Asn Cys Ser Leu Pro Leu Ala Phe Asp Gly Ser Cys Ser Gly Ile Gln
       530                 535                 540
   cac ttc tcc gcg atg ctc cga gat gag gta ggt ggt cgc gcg gtt aac    1680
   His Phe Ser Ala Met Leu Arg Asp Glu Val Gly Gly Arg Ala Val Asn
   545                 550                 555                 560
   ttg ctt cct agt gaa acc gtt cag gac atc tac ggg att gtt gct aag    1728
   Leu Leu Pro Ser Glu Thr Val Gln Asp Ile Tyr Gly Ile Val Ala Lys
                   565                 570                 575
   aaa gtc aac gag att cta caa gca gac gca atc aat ggg acc gat aac    1776
   Lys Val Asn Glu Ile Leu Gln Ala Asp Ala Ile Asn Gly Thr Asp Asn
               580                 585                 590
   gaa gta gtt acc gtg acc gat gag aac act ggt gaa atc tct gag aaa    1824
   Glu Val Val Thr Val Thr Asp Glu Asn Thr Gly Glu Ile Ser Glu Lys
           595                 600                 605
   gtc aag ctg ggc act aag gca ctg gct ggt caa tgg ctg gct tac ggt    1872
   Val Lys Leu Gly Thr Lys Ala Leu Ala Gly Gln Trp Leu Ala Tyr Gly
       610                 615                 620
   gtt act cgc agt gtg act aag cgt tca gtc atg acg ctg gct tac ggg    1920
   Val Thr Arg Ser Val Thr Lys Arg Ser Val Met Thr Leu Ala Tyr Gly
   625                 630                 635                 640
   tcc aaa gag ttc ggc ttc cgt caa caa gtg ctg gaa gat acc att cag    1968
   Ser Lys Glu Phe Gly Phe Arg Gln Gln Val Leu Glu Asp Thr Ile Gln
                   645                 650                 655
   cca gct att gat tcc ggc aag ggt ctg atg ttc act cag ccg aat cag    2016
   Pro Ala Ile Asp Ser Gly Lys Gly Leu Met Phe Thr Gln Pro Asn Gln
               660                 665                 670
   gct gct gga tac atg gct aag ctg att tgg gaa tct gtg agc gtg acg    2064
   Ala Ala Gly Tyr Met Ala Lys Leu Ile Trp Glu Ser Val Ser Val Thr
           675                 680                 685
   gtg gta gct gcg gtt gaa gca atg aac tgg ctt aag tct gct gct aag    2112
   Val Val Ala Ala Val Glu Ala Met Asn Trp Leu Lys Ser Ala Ala Lys
       690                 695                 700
   ctg ctg gct gct gag gtc aaa gat aag aag act gga gag att ctt cgc    2160
   Leu Leu Ala Ala Glu Val Lys Asp Lys Lys Thr Gly Glu Ile Leu Arg
   705                 710                 715                 720
   aag cgt tgc gct gtg cat tgg gta act cct gat ggt ttc cct gtg tgg    2208
   Lys Arg Cys Ala Val His Trp Val Thr Pro Asp Gly Phe Pro Val Trp
                   725                 730                 735
   cag gaa tac aag aag cct att cag acg cgc ttg aac ctg atg ttc ctc    2256
   Gln Glu Tyr Lys Lys Pro Ile Gln Thr Arg Leu Asn Leu Met Phe Leu
               740                 745                 750
   ggt cag ttc cgc tta cag cct acc att aac acc aac aaa gat agc gag    2304
   Gly Gln Phe Arg Leu Gln Pro Thr Ile Asn Thr Asn Lys Asp Ser Glu
           755                 760                 765
   att gat gca cac aaa cag gag tct ggt atc gct cct aac ttt gta cac    2352
   Ile Asp Ala His Lys Gln Glu Ser Gly Ile Ala Pro Asn Phe Val His
       770                 775                 780
   agc caa gac ggt agc cac ctt cgt aag act gta gtg tgg gca cac gag   2400
   Ser Gln Asp Gly Ser His Leu Arg Lys Thr Val Val Trp Ala His Glu
   785                 790                 795                 800
   aag tac gga atc gaa tct ttt gca ctg aat cac gac tcc ttc ggt acc   2448
   Lys Tyr Gly Ile Glu Ser Phe Ala Leu Asn His Asp Ser Phe Gly Thr
                   805                 810                 815
   att ccg gct gac gct gcg aac ctg ttc aaa gca gtg cgc gaa act atg   2496
   Ile Pro Ala Asp Ala Ala Asn Leu Phe Lys Ala Val Arg Glu Thr Met
               820                 825                 830
   gtt gac aca tat gag tct tgt gat gta ctg gct gat ttc tac gac cag   2544
   Val Asp Thr Tyr Glu Ser Cys Asp Val Leu Ala Asp Phe Tyr Asp Gln
           835                 840                 845
   ttc gct gac cag ttg cac gag tct caa ttg gac aaa atg cca gca ctt   2592
   Phe Ala Asp Gln Leu His Glu Ser Gln Leu Asp Lys Met Pro Ala Leu
       850                 855                 860
   ccg gct aaa ggt aac ttg aac ctc cgt gac atc tta gag tcg gac ttc   2640
   Pro Ala Lys Gly Asn Leu Asn Leu Arg Asp Ile Leu Glu Ser Asp Phe
   865                 870                 875                 880
   gcg ttc gcg taa                                                   2652
   Ala Phe Ala
<210> 14
   <211> 883
   <212> PRT
   <213> Séquence artificielle
   <223> Description de la séquence artificielle: séquences mutées de l'ARN polymérase du bactériophage T7
<400> 14
   Met Asn Thr Ile Asn Ile Ala Lys Asn Asp Phe Ser Asp Ile Glu Leu
   1               5                   10                  15
   Ala Ala Ile Pro Phe Asn Thr Leu Ala Asp His Tyr Gly Glu Arg Leu
               20                  25                  30
   Ala Arg Glu Gln Leu Ala Leu Glu His Glu Ser Tyr Glu Met Gly Glu
           35                  40                  45
   Ala Arg Phe Arg Lys Met Phe Glu Arg Gln Leu Lys Ala Gly Glu Val
       50                  55                  60
   Ala Asp Asn Ala Ala Ala Lys Pro Leu Ile Thr Thr Leu Leu Pro Lys
   65                  70                  75                  80
   Met Ile Ala Arg Ile Asn Asp Trp Phe Glu Glu Val Lys Ala Lys Arg
                   85                  90                  95
   Gly Lys Arg Pro Thr Ala Phe Gln Phe Leu Gln Glu Ile Lys Pro Glu
               100                 105                 110
   Ala Val Ala Tyr Ile Thr Ile Lys Thr Thr Leu Ala Cys Leu Thr Ser
           115                 120                 125
   Ala Asp Asn Thr Thr Val Gln Ala Val Ala Ser Ala Ile Gly Arg Ala
       130                 135                 140
   Ile Glu Asp Glu Ala Arg Phe Gly Arg Ile Arg Asp Leu Glu Ala Lys
   145                 150                 155                 160
   His Phe Lys Lys Asn Val Glu Glu Gln Leu Asn Lys Arg Val Gly His
                   165                 170                 175
   Val Tyr Lys Lys Ala Phe Met Gln Val Val Glu Ala Asp Met Leu Ser
               180                 185                 190
   Lys Gly Leu Leu Gly Gly Glu Ala Trp Ser Ser Trp His Lys Glu Asp
           195                 200                 205
   Ser Ile His Val Gly Val Arg Cys Ile Glu Met Leu Ile Glu Ser Thr
       210                 215                 220
   Gly Met Val Ser Leu His Arg Gln Asn Ala Gly Val Val Gly Gln Asp
   225                 230                 235                 240
   Ser Glu Thr Ile Glu Leu Ala Pro Glu Tyr Ala Glu Ala Ile Ala Thr
                   245                 250                 255
   Arg Ala Gly Ala Leu Ala Gly Ile Ser Leu Met Phe Gln Pro Cys Val
               260                 265                 270
   Val Pro Pro Lys Pro Trp Thr Gly Ile Thr Gly Gly Gly Tyr Trp Ala
           275                 280                 285
   Asn Gly Arg Arg Pro Leu Ala Leu Val Arg Thr His Ser Lys Lys Ala
       290                 295                 300
   Leu Met Arg Tyr Glu Asp Val Tyr Met Pro Glu Val Tyr Lys Ala Ile
   305                 310                 315                 320
   Asn Ile Ala Gln Asn Thr Ala Trp Lys Ile Asn Lys Lys Val Leu Ala
                   325                 330                 335
   Val Ala Asn Val Ile Thr Lys Trp Lys His Cys Pro Val Glu Asp Ile
               340                 345                 350
   Pro Ala Ile Glu Arg Glu Glu Leu Pro Met Lys Pro Glu Asp Ile Asp
           355                 360                 365
   Met Asn Pro Glu Ala Leu Thr Ala Trp Lys Arg Ala Ala Ala Ala Val
       370                 375                 380
   Tyr Arg Lys Asp Lys Ala Arg Lys Ser Arg Arg Ile Ser Leu Glu Phe
   385                 390                 395                 400
   Met Leu Glu Gln Ala Asn Lys Phe Ala Asn His Lys Ala Ile Trp Phe
                   405                 410                 415
   Pro Tyr Asn Met Asp Trp Arg Gly Arg Val Tyr Ala Val Ser Met Phe
               420                 425                 430
   Asn Pro Gln Gly Asn Asp Met Thr Lys Gly Leu Leu Thr Leu Ala Lys
           435                 440                 445
   Gly Lys Pro Ile Gly Lys Glu Gly Tyr Tyr Trp Leu Lys Ile His Gly
       450                 455                 460
   Ala Asn Cys Ala Gly Val Asp Lys Val Pro Phe Pro Glu Arg Ile Lys
   465                 470                 475                 480
   Phe Ile Glu Glu Asn His Glu Asn Ile Met Ala Cys Ala Lys Ser Pro
                   485                 490                 495
   Leu Glu Asn Thr Trp Trp Ala Glu Gln Asp Ser Pro Phe Cys Phe Leu
               500                 505                 510
   Ala Phe Cys Phe Glu Tyr Ala Gly Val Gln His His Gly Leu Ser Tyr
           515                 520                 525
   Asn Cys Ser Leu Pro Leu Ala Phe Asp Gly Ser Cys Ser Gly Ile Gln
       530                 535                 540
   His Phe Ser Ala Met Leu Arg Asp Glu Val Gly Gly Arg Ala Val Asn
   545                 550                 555                 560
   Leu Leu Pro Ser Glu Thr Val Gln Asp Ile Tyr Gly Ile Val Ala Lys
                   565                 570                 575
   Lys Val Asn Glu Ile Leu Gln Ala Asp Ala Ile Asn Gly Thr Asp Asn
               580                 585                 590
   Glu Val Val Thr Val Thr Asp Glu Asn Thr Gly Glu Ile Ser Glu Lys
           595                 600                 605
   Val Lys Leu Gly Thr Lys Ala Leu Ala Gly Gln Trp Leu Ala Tyr Gly
       610                 615                 620
   Val Thr Arg Ser Val Thr Lys Arg Ser Val Met Thr Leu Ala Tyr Gly
   625                 630                 635                 640
   Ser Lys Glu Phe Gly Phe Arg Gln Gln Val Leu Glu Asp Thr Ile Gln
                   645                 650                 655
   Pro Ala Ile Asp Ser Gly Lys Gly Leu Met Phe Thr Gln Pro Asn Gln
               660                 665                 670
   Ala Ala Gly Tyr Met Ala Lys Leu Ile Trp Glu Ser Val Ser Val Thr
           675                 680                 685
   Val Val Ala Ala Val Glu Ala Met Asn Trp Leu Lys Ser Ala Ala Lys
       690                 695                 700
   Leu Leu Ala Ala Glu Val Lys Asp Lys Lys Thr Gly Glu Ile Leu Arg
   705                 710                 715                 720
   Lys Arg Cys Ala Val His Trp Val Thr Pro Asp Gly Phe Pro Val Trp
                   725                 730                 735
   Gln Glu Tyr Lys Lys Pro Ile Gln Thr Arg Leu Asn Leu Met Phe Leu
               740                 745                 750
   Gly Gln Phe Arg Leu Gln Pro Thr Ile Asn Thr Asn Lys Asp Ser Glu
           755                 760                 765
   Ile Asp Ala His Lys Gln Glu Ser Gly Ile Ala Pro Asn Phe Val His
       770                 775                 780
   Ser Gln Asp Gly Ser His Leu Arg Lys Thr Val Val Trp Ala His Glu
   785                 790                 795                 800
   Lys Tyr Gly Ile Glu Ser Phe Ala Leu Asn His Asp Ser Phe Gly Thr
                   805                 810                 815
   Ile Pro Ala Asp Ala Ala Asn Leu Phe Lys Ala Val Arg Glu Thr Met
               820                 825                 830
   Val Asp Thr Tyr Glu Ser Cys Asp Val Leu Ala Asp Phe Tyr Asp Gln
           835                 840                 845
   Phe Ala Asp Gln Leu His Glu Ser Gln Leu Asp Lys Met Pro Ala Leu
       850                 855                 860
   Pro Ala Lys Gly Asn Leu Asn Leu Arg Asp Ile Leu Glu Ser Asp Phe
   865                 870                 875                 880
   Ala Phe Ala
<210> 15
   <211> 2652
   <212> ADN
   <213> Séquence artificielle
<220>
   <223> Description de la séquence artificielle: séquences mutées de l'ARN polymérase du bactériophage T7
<220>
   <221> CDS
   <222> (1)..(2652)
<400> 15
   atg aac acg att aac atc gct aag aac gac ttc tct gac atc gaa ctg      48
   Met Asn Thr Ile Asn Ile Ala Lys Asn Asp Phe Ser Asp Ile Glu Leu
     1               5                  10                  15
   gct gct atc ccg ttc aac act ctg gct gac cat tac ggt gag cgt tta      96
   Ala Ala Ile Pro Phe Asn Thr Leu Ala Asp His Tyr Gly Glu Arg Leu
               20                  25                  30
   gct cgc gaa cag ttg gcc ctt gag cat gag tct tac gag atg ggt gaa     144
   Ala Arg Glu Gln Leu Ala Leu Glu His Glu Ser Tyr Glu Met Gly Glu
           35                  40                  45
   gca cgc ttc cgc aag atg ttt gag cgt caa ctt aaa gct ggt gag gtt     192
   Ala Arg Phe Arg Lys Met Phe Glu Arg Gln Leu Lys Ala Gly Glu Val
       50                  55                  60
   gcg gat aac gct gcc gcc aag cct ctc atc act acc cta ctc cct aag     240
   Ala Asp Asn Ala Ala Ala Lys Pro Leu Ile Thr Thr Leu Leu Pro Lys
    65                  70                  75                  80
   atg att gca cgc atc aac gac tgg ttt gag gaa gtg aaa gct aag cgc     288
   Met Ile Ala Arg Ile Asn Asp Trp Phe Glu Glu Val Lys Ala Lys Arg
                    85                  90                  95
   ggc aag cgc ccg aca gcc ttc cag ttc ctg caa gaa atc aag ccg gaa     336
   Gly Lys Arg Pro Thr Ala Phe Gln Phe Leu Gln Glu Ile Lys Pro Glu
               100                 105                 110
   gcc gta gcg tac atc acc gtt aag acc act ctg gct tgc cta acc agt     384
   Ala Val Ala Tyr Ile Thr Val Lys Thr Thr Leu Ala Cys Leu Thr Ser
           115                 120                 125
   gct gac aat aca acc gtt cag gct gta gca agc gca atc ggt cgg gcc     432
   Ala Asp Asn Thr Thr Val Gln Ala Val Ala Ser Ala Ile Gly Arg Ala
       130                 135                 140
   att gag aac gag gct cgc ttc ggt cgt atc cgt gac ctt gaa gct aag     480
   Ile Glu Asn Glu Ala Arg Phe Gly Arg Ile Arg Asp Leu Glu Ala Lys
   145                 150                 155                 160
   cac ttc aag aaa aac gtt gag gaa caa ctc aac aag cgc gta ggg cac     528
   His Phe Lys Lys Asn Val Glu Glu Gln Leu Asn Lys Arg Val Gly His
                   165                 170                 175
   gtc tac aag aaa gca ttt atg caa gtt gtc gag gct gac atg ctc tct     576
   Val Tyr Lys Lys Ala Phe Met Gln Val Val Glu Ala Asp Met Leu Ser
               180                 185                 190
   aag ggt cta ctc ggt ggc gag gcg tgg tct tcg tgg cat aag gaa gac     624
   Lys Gly Leu Leu Gly Gly Glu Ala Trp Ser Ser Trp His Lys Glu Asp
           195                 200                 205
   tct att cat gta gga gta cgc tgc atc gag atg ctc att gag tca acc     672
   Ser Ile His Val Gly Val Arg Cys Ile Glu Met Leu Ile Glu Ser Thr
       210                 215                 220
   gga atg gtt agc tta cac cgc caa aat gct ggc gta gta ggt caa gac     720
   Gly Met Val Ser Leu His Arg Gln Asn Ala Gly Val Val Gly Gln Asp
   225                 230                 235                 240
   tct gag act atc gaa ctc gca cct gaa tac gct gag gct atc gca acc     768
   Ser Glu Thr Ile Glu Leu Ala Pro Glu Tyr Ala Glu Ala Ile Ala Thr
                   245                 250                 255
   cgt gca ggt gcg ctg gct ggc atc tct ccg atg ttc caa cct tgc gta     816
   Arg Ala Gly Ala Leu Ala Gly Ile Ser Pro Met Phe Gln Pro Cys Val
               260                 265                 270
   gtt cct cct aag ccg tgg act ggc att act ggt ggt ggc tat tgg gct     864
   Val Pro Pro Lys Pro Trp Thr Gly Ile Thr Gly Gly Gly Tyr Trp Ala
           275                 280                 285
   aac ggt cgt cgt cct ctg gcg ctg gtg cgt act cac agt aag aaa gca     912
   Asn Gly Arg Arg Pro Leu Ala Leu Val Arg Thr His Ser Lys Lys Ala
       290                 295                 300
   ctg atg cgc tac gaa gac gtt tac atg cct gag gtg tac aaa gcg att     960
   Leu Met Arg Tyr Glu Asp Val Tyr Met Pro Glu Val Tyr Lys Ala Ile
   305                 310                 315                 320
   aac att gcg caa aac acc gca tgg aaa atc aac aag aaa gtc cta gcg     1008
   Asn Ile Ala Gln Asn Thr Ala Trp Lys Ile Asn Lys Lys Val Leu Ala
                   325                 330                 335
   gtc gcc aac gta atc acc aag tgg aag cat tgt ccg gtc gag gac atc     1056
   Val Ala Asn Val Ile Thr Lys Trp Lys His Cys Pro Val Glu Asp Ile
               340                 345                 350
   cct gcg att gag cgt gaa gaa ctc ccg atg aaa ccg gaa gac atc gac     1104
   Pro Ala Ile Glu Arg Glu Glu Leu Pro Met Lys Pro Glu Asp Ile Asp
           355                 360                 365
   atg aat cct gag gct ctc acc gcg tgg aaa cgt gct gcc gct gct gtg     1152
   Met Asn Pro Glu Ala Leu Thr Ala Trp Lys Arg Ala Ala Ala Ala Val
       370                 375                 380
   tac cgc aag gac aag gct cgc aag tct cgc cgt atc agc ctt gag ttc     1200
   Tyr Arg Lys Asp Lys Ala Arg Lys Ser Arg Arg Ile Ser Leu Glu Phe
   385                 390                 395                 400
   atg ctt gag caa gcc aat aag ttt gct aac cat aag gcc atc tgg ttc     1248
   Met Leu Glu Gln Ala Asn Lys Phe Ala Asn His Lys Ala Ile Trp Phe
                   405                 410                 415
   cct tac aac atg gac tgg cgc ggt cgt gtt tac gct gtg tca atg ttc     1296
   Pro Tyr Asn Met Asp Trp Arg Gly Arg Val Tyr Ala Val Ser Met Phe
               420                 425                 430
   aac ccg caa ggt aac gat atg acc aaa gga ctg ctt acg ctg gcg aaa     1344
   Asn Pro Gln Gly Asn Asp Met Thr Lys Gly Leu Leu Thr Leu Ala Lys
           435                 440                 445
   ggt aaa cca atc ggt aag gaa ggt tac tac tgg ctg aaa atc cac ggt     1392
   Gly Lys Pro Ile Gly Lys Glu Gly Tyr Tyr Trp Leu Lys Ile His Gly
       450                 455                 460
   gca aac tgt gcg ggt gtc gat aag gtt ccg ttc cct gag cgc atc aag     1440
   Ala Asn Cys Ala Gly Val Asp Lys Val Pro Phe Pro Glu Arg Ile Lys
   465                 470                 475                 480
   ttc att gag gaa aac cac gag aac atc atg gct tgc gct aag tct cca     1488
   Phe Ile Glu Glu Asn His Glu Asn Ile Met Ala Cys Ala Lys Ser Pro
                   485                 490                 495
   ctg gag aac act tgg tgg gct gag caa gat tct ccg ttc tgc ttc ctt     1536
   Leu Glu Asn Thr Trp Trp Ala Glu Gln Asp Ser Pro Phe Cys Phe Leu
               500                 505                 510
   gcg ttc tgc ttt gag tac gct ggg gta cag cac cac ggc ctg agc tat     1584
   Ala Phe Cys Phe Glu Tyr Ala Gly Val Gln His His Gly Leu Ser Tyr
           515                 520                 525
   aac tgc tcc ctt ccg ctg gcg ttt gac ggg tct tgc tct ggc atc cag     1632
   Asn Cys Ser Leu Pro Leu Ala Phe Asp Gly Ser Cys Ser Gly Ile Gln
       530                 535                 540
   cac ttc tcc gcg atg ctc cga gat gag gta ggt ggt cgc gcg gtt aac     1680
   His Phe Ser Ala Met Leu Arg Asp Glu Val Gly Gly Arg Ala Val Asn
   545                 550                 555                 560
   ttg ctt cct agt gaa acc gtt cag gac atc tac ggg att gtt gct aag    1728
   Leu Leu Pro Ser Glu Thr Val Gln Asp Ile Tyr Gly Ile Val Ala Lys
                   565                 570                 575
   aaa gtc aac gag att cta caa gca gac gca atc aat ggg acc gat aac    1776
   Lys Val Asn Glu Ile Leu Gln Ala Asp Ala Ile Asn Gly Thr Asp Asn
               580                 585                 590
   gaa gta gtt acc gtg acc gat gag aac act ggt gaa atc tct gag aaa    1824
   Glu Val Val Thr Val Thr Asp Glu Asn Thr Gly Glu Ile Ser Glu Lys
           595                 600                 605
   gtc aag ctg ggc act aag gca ctg gct ggt caa tgg ctg gct tac ggt    1872
   Val Lys Leu Gly Thr Lys Ala Leu Ala Gly Gln Trp Leu Ala Tyr Gly
       610                 615                 620
   gtt act cgc agt gtg act aag cgt tca gtc atg acg ctg gct tac ggg    1920
   Val Thr Arg Ser Val Thr Lys Arg Ser Val Met Thr Leu Ala Tyr Gly
   625                 630                 635                 640
   tcc aaa gag ttc ggc ttc cgt caa caa gtg ctg gaa gat acc att cag    1968
   Ser Lys Glu Phe Gly Phe Arg Gln Gln Val Leu Glu Asp Thr Ile Gln
                   645                 650                 655
   cca gct att gat tcc ggc aag ggt ctg atg ttc act cag ccg aat cag    2016
   Pro Ala Ile Asp Ser Gly Lys Gly Leu Met Phe Thr Gln Pro Asn Gln
               660                 665                 670
   gct gct gga tac atg gct aag ctg att tgg gaa tct gtg agc gtg acg    2064
   Ala Ala Gly Tyr Met Ala Lys Leu Ile Trp Glu Ser Val Ser Val Thr
           675                 680                 685
   gtg gta gct gcg gtt gaa gca atg aac tgg ctt aag tct gct gct aag    2112
   Val Val Ala Ala Val Glu Ala Met Asn Trp Leu Lys Ser Ala Ala Lys
       690                 695                 700
   ctg ctg gct gct gag gtc aaa gat aag aag act gga gag att ctt cgc    2160
   Leu Leu Ala Ala Glu Val Lys Asp Lys Lys Thr Gly Glu Ile Leu Arg
   705                 710                 715                 720
   aag cgt tgc gct gtg cat tgg gta act cct gat ggt ttc cct gtg tgg    2208
   Lys Arg Cys Ala Val His Trp Val Thr Pro Asp Gly Phe Pro Val Trp
                   725                 730                 735
   cag gaa tac aag aag cct att cag acg cgc ttg aac ctg atg ttc ctc    2256
   Gln Glu Tyr Lys Lys Pro Ile Gln Thr Arg Leu Asn Leu Met Phe Leu
               740                 745                 750
   ggt cag ttc cgc tta cag cct acc att aac acc aac aaa gat agc gag    2304
   Gly Gln Phe Arg Leu Gln Pro Thr Ile Asn Thr Asn Lys Asp Ser Glu
           755                 760                 765
   att gat gca cac aaa cag gag tct ggt atc gct cct aac ttt gta cac    2352
   Ile Asp Ala His Lys Gln Glu Ser Gly Ile Ala Pro Asn Phe Val His
       770                 775                 780
   agc caa gac ggt agc cac ctt cgt aag act gta gtg tgg gca cac gag    2400
   Ser Gln Asp Gly Ser His Leu Arg Lys Thr Val Val Trp Ala His Glu
   785                 790                 795                 800
   aag tac gga atc gaa tct ttt gca ctg aat cac gac tcc ttc ggt acc     2448
   Lys Tyr Gly Ile Glu Ser Phe Ala Leu Asn His Asp Ser Phe Gly Thr
                   805                 810                 815
   att ccg gct gac gct gcg aac ctg ttc aaa gca gtg cgc gaa act atg     2496
   Ile Pro Ala Asp Ala Ala Asn Leu Phe Lys Ala Val Arg Glu Thr Met
               820                 825                 830
   gtt gac aca tat gag tct tgt gat gta ctg gct gat ttc tac gac cag     2544
   Val Asp Thr Tyr Glu Ser Cys Asp Val Leu Ala Asp Phe Tyr Asp Gln
           835                 840                 845
   ttc gct gac cag ttg cac gag tct caa ttg gac aaa atg cca gca ctt     2592
   Phe Ala Asp Gln Leu His Glu Ser Gln Leu Asp Lys Met Pro Ala Leu
       850                 855                 860
   ccg gct aaa ggt aac ttg aac ctc cgt gac atc tta gag tcg gac ttc     2640
   Pro Ala Lys Gly Asn Leu Asn Leu Arg Asp Ile Leu Glu Ser Asp Phe
   865                 870                 875                 880
   gcg ttc gcg taa                                                     2652
   Ala Phe Ala
<210> 16
   <211> 883
   <212> PRT
   <213> Séquence artificielle
   <223> Description de la séquence artificielle: séquences mutées de l'ARN polymérase du bactériophage T7
<400> 16
   Met Asn Thr Ile Asn Ile Ala Lys Asn Asp Phe Ser Asp Ile Glu Leu
    1               5                  10                  15
   Ala Ala Ile Pro Phe Asn Thr Leu Ala Asp His Tyr Gly Glu Arg Leu
               20                  25                  30
   Ala Arg Glu Gln Leu Ala Leu Glu His Glu Ser Tyr Glu Met Gly Glu
           35                  40                  45
   Ala Arg Phe Arg Lys Met Phe Glu Arg Gln Leu Lys Ala Gly Glu Val
       50                  55                  60
   Ala Asp Asn Ala Ala Ala Lys Pro Leu Ile Thr Thr Leu Leu Pro Lys
   65                  70                  75                  80
   Met Ile Ala Arg Ile Asn Asp Trp Phe Glu Glu Val Lys Ala Lys Arg
                   85                  90                  95
   Gly Lys Arg Pro Thr Ala Phe Gln Phe Leu Gln Glu Ile Lys Pro Glu
               100                 105                 110
   Ala Val Ala Tyr Ile Thr Val Lys Thr Thr Leu Ala Cys Leu Thr Ser
           115                 120                 125
   Ala Asp Asn Thr Thr Val Gln Ala Val Ala Ser Ala Ile Gly Arg Ala
       130                 135                 140
   Ile Glu Asn Glu Ala Arg Phe Gly Arg Ile Arg Asp Leu Glu Ala Lys
   145                 150                 155                 160
   His Phe Lys Lys Asn Val Glu Glu Gln Leu Asn Lys Arg Val Gly His
   165 170 175
   Val Tyr Lys Lys Ala Phe Met Gln Val Val Glu Ala Asp Met Leu Ser
   180 185 190
   Lys Gly Leu Leu Gly Gly Glu Ala Trp Ser Ser Trp His Lys Glu Asp
   195 200 205
   Ser Ile His Val Gly Val Arg Cys Ile Glu Met Leu Ile Glu Ser Thr
   210 215 220
   Gly Met Val Ser Leu His Arg Gln Asn Ala Gly Val Val Gly Gln Asp
   225 230 235 240
   Ser Glu Thr Ile Glu Leu Ala Pro Glu Tyr Ala Glu Ala Ile Ala Thr
   245 250 255
   Arg Ala Gly Ala Leu Ala Gly Ile Ser Pro Met Phe Gln Pro Cys Val
   260 265 270
   Val Pro Pro Lys Pro Trp Thr Gly Ile Thr Gly Gly Gly Tyr Trp Ala
   275 280 285
   Asn Gly Arg Arg Pro Leu Ala Leu Val Arg Thr His Ser Lys Lys Ala
   290 295 300
   Leu Met Arg Tyr Glu Asp Val Tyr Met Pro Glu Val Tyr Lys Ala Ile
   305 310 315 320
   Asn Ile Ala Gln Asn Thr Ala Trp Lys Ile Asn Lys Lys Val Leu Ala
   325 330 335
   Val Ala Asn Val Ile Thr Lys Trp Lys His Cys Pro Val Glu Asp Ile
   340 345 350
   Pro Ala Ile Glu Arg Glu Glu Leu Pro Met Lys Pro Glu Asp Ile Asp
   355 360 365
   Met Asn Pro Glu Ala Leu Thr Ala Trp Lys Arg Ala Ala Ala Ala Val
   370 375 380
   Tyr Arg Lys Asp Lys Ala Arg Lys Ser Arg Arg Ile Ser Leu Glu Phe
   385 390 395 400
   Met Leu Glu Gln Ala Asn Lys Phe Ala Asn His Lys Ala Ile Trp Phe
   405 410 415
   Pro Tyr Asn Met Asp Trp Arg Gly Arg Val Tyr Ala Val Ser Met Phe
   420 425 430
   Asn Pro Gln Gly Asn Asp Met Thr Lys Gly Leu Leu Thr Leu Ala Lys
   435 440 445
   Gly Lys Pro Ile Gly Lys Glu Gly Tyr Tyr Trp Leu Lys Ile His Gly
   450 455 460
   Ala Asn Cys Ala Gly Val Asp Lys Val Pro Phe Pro Glu Arg Ile Lys
   465 470 475 480
   Phe Ile Glu Glu Asn His Glu Asn Ile Met Ala Cys Ala Lys Ser Pro
   485 490 495
   Leu Glu Asn Thr Trp Trp Ala Glu Gln Asp Ser Pro Phe Cys Phe Leu
   500 505 510
   Ala Phe Cys Phe Glu Tyr Ala Gly Val Gln His His Gly Leu Ser Tyr
   515 520 525
   Asn Cys Ser Leu Pro Leu Ala Phe Asp Gly Ser Cys Ser Gly Ile Gln
   530 535 540
   His Phe Ser Ala Met Leu Arg Asp Glu Val Gly Gly Arg Ala Val Asn
   545 550 555 560
   Leu Leu Pro Ser Glu Thr Val Gln Asp Ile Tyr Gly Ile Val Ala Lys
   565 570 575
   Lys Val Asn Glu Ile Leu Gln Ala Asp Ala Ile Asn Gly Thr Asp Asn
   580 585 590
   Glu Val Val Thr Val Thr Asp Glu Asn Thr Gly Glu Ile Ser Glu Lys
   595 600 605
   Val Lys Leu Gly Thr Lys Ala Leu Ala Gly Gln Trp Leu Ala Tyr Gly
   610 615 620
   Val Thr Arg Ser Val Thr Lys Arg Ser Val Met Thr Leu Ala Tyr Gly
   625 630 635 640
   Ser Lys Glu Phe Gly Phe Arg Gln Gln Val Leu Glu Asp Thr Ile Gln
   645 650 655
   Pro Ala Ile Asp Ser Gly Lys Gly Leu Met Phe Thr Gln Pro Asn Gln
   660 665 670
   Ala Ala Gly Tyr Met Ala Lys Leu Ile Trp Glu Ser Val Ser Val Thr
   675 680 685
   Val Val Ala Ala Val Glu Ala Met Asn Trp Leu Lys Ser Ala Ala Lys
   690 695 700
   Leu Leu Ala Ala Glu Val Lys Asp Lys Lys Thr Gly Glu Ile Leu Arg
   705 710 715 720
   Lys Arg Cys Ala Val His Trp Val Thr Pro Asp Gly Phe Pro Val Trp
   725 730 735
   Gln Glu Tyr Lys Lys Pro Ile Gln Thr Arg Leu Asn Leu Met Phe Leu
   740 745 750
   Gly Gln Phe Arg Leu Gln Pro Thr Ile Asn Thr Asn Lys Asp Ser Glu
   755 760 765
   Ile Asp Ala His Lys Gln Glu Ser Gly Ile Ala Pro Asn Phe Val His
   770 775 780
   Ser Gln Asp Gly Ser His Leu Arg Lys Thr Val Val Trp Ala His Glu
   785 790 795 800
   Lys Tyr Gly Ile Glu Ser Phe Ala Leu Asn His Asp Ser Phe Gly Thr
                   805                 810                 815
   Ile Pro Ala Asp Ala Ala Asn Leu Phe Lys Ala Val Arg Glu Thr Met
               820                 825                 830
   Val Asp Thr Tyr Glu Ser Cys Asp Val Leu Ala Asp Phe Tyr Asp Gln
           835                 840                 845
   Phe Ala Asp Gln Leu His Glu Ser Gln Leu Asp Lys Met Pro Ala Leu
       850                 855                 860
   Pro Ala Lys Gly Asn Leu Asn Leu Arg Asp Ile Leu Glu Ser Asp Phe
   865                 870                 875                     880
   Ala Phe Ala